# EUROPEAN PATENT APPLICATION

(11) **EP 4 349 858 A1**
(43) Date of publication of application: **10.04.2024**
(21) Application number: 22811626.5
(22) Date of filing: 24.05.2022
(51) Int. Cl.: C07K 14/705, C07K 16/28, C12N 15/85, A61K 35/17, A61P 35/00

(54) **CHIMERIC ANTIGEN RECEPTOR SPECIFICALLY BINDING TO CD300C ANTIGEN OR RECEPTOR THEREOF**

(30) Priority: 24.05.2021 KR 20210066547
(71) Applicant: CentricsBio, Inc., Seoul 05836 (KR)
(72) Inventor: JEON, Jae-Won, Suwon-si, Gyeonggi-do 16512 (KR); LEE, Suin, Gunpo-si, Gyeonggi-do 15801 (KR); KIM, Haneul, Namyangju-si, Gyeonggi-do 12221 (KR); LIM, Chang Ki, Hwaseong-si, Gyeonggi-do 18496 (KR)
(74) Representative: Regimbeau
(86) International application number: PCT/KR2022/007384
(87) International publication number: WO 2022/250433

(57) **Abstract**

Disclosed are a chimeric antigen receptor specifically binding to a CD300c antigen or a receptor thereof, an immune cell expressing the same, and the like, wherein the chimeric antigen receptor specifically binding to a CD300c antigen or a receptor thereof according to the present disclosure can specifically recognize cancer cells expressing the CD300c antigen or the CD300c receptor and thus can inhibit the growth, metastasis, development, and the like of cancer in a direct and effective manner and can be effectively used as an immunotherapeutic agent for various types of cancer.

## Description

### [Technical Field]

The present disclosure relates to a chimeric antigen receptor specifically binding to a CD300c antigen or a receptor thereof, immune cells expressing the same, uses thereof, and the like.

### [Background Art]

Cancer is one of the diseases that account for the largest share of the causes of death in modern people. This disease is caused by changes in normal cells due to genetic mutations that result from various causes and refers to a malignant tumor that does not follow differentiation, proliferation, growth pattern, or the like of normal cells. Cancer is characterized by "uncontrolled cell growth", and this abnormal cell growth causes the formation of a cell mass called a tumor, which infiltrates the surrounding tissues and, in severe cases, may metastasize to other organs of the body. Cancer is an intractable chronic disease that is not fundamentally cured in many cases even if it is treated with surgery, radiotherapy, chemotherapy, and the like, causes pain to patients, and ultimately leads to death. In particular, in recent years, the global cancer incidence rate is increasing by 5% or higher every year due to increased elderly population, environmental deterioration, or the like. According to the WHO report, it is estimated that within the next 25 years, the number of cancer patients will increase to 30 million, of which 20 million will die from cancer.

Cancer drug treatments, that is, anticancer agents are generally cytotoxic compounds and treat cancer by attacking and killing cancer cells. However, these anticancer agents exhibit high adverse effects since they damage not only cancer cells but also normal cells. Thus, targeted therapeutic agents have been developed to decrease adverse effects. However, these targeted therapeutic agents could exhibit decreased adverse effects, but had a limitation in that resistance occurs with a high probability. Therefore, in recent years, interest in immunotherapeutic agents, which use the body's immune system to decrease problems due to toxicity and resistance, is rapidly increasing. As an example of such immunotherapeutic agents, immune checkpoint inhibitors have been developed that specifically bind to PD-L1 on the surface of cancer cells and inhibit the binding of T cells to PD-1 so that T cells are activated to attack cancer cells. However, even these immune checkpoint inhibitors are not effective in various types of cancer. Therefore, there is an urgent need to develop novel immunotherapeutic agents that exhibit an equivalent therapeutic effect in various cancers.

Meanwhile, chimeric antigen receptors (CARs) are artificial receptors designed to deliver antigen specificity to T cells, and are composed of an antigen-specific domain that activates T cells and provides specific immunity, a transmembrane domain, an intracellular domain, and the like. Recently, studies are actively conducted on cancer immunotherapy using cells into which a gene encoding such a chimeric antigen receptor has been introduced, that is, a method for treating cancer through a therapy in which T cells are collected from a patient, a gene encoding a chimeric antigen receptor is introduced into these T cells and amplified, and transferred back into the patient.

### [Prior Art Document]

### [Patent Document]

(Patent Document 1) Korean Patent Publication No. 10-2016-0016725

### [Disclosure]

### [Technical Problem]

The present disclosure has been made to solve the problems of the prior art as described above.

An aspect of the present disclosure is to provide a chimeric antigen receptor for preventing or treating cancer, including a binding domain specifically binding to a CD300c antigen or a receptor thereof.

Another aspect of the present disclosure is to provide an immune cell expressing the chimeric antigen receptor.

Still another aspect of the present disclosure is to provide an isolated nucleic acid molecule encoding the chimeric antigen receptor.

Still another aspect of the present disclosure is to provide a vector including the nucleic acid molecule encoding the chimeric antigen receptor.

Still another aspect of the present disclosure is to provide an anticancer therapy using the chimeric antigen receptor or the immune cell including the same.

Still another aspect of the present disclosure is to provide a method for preventing or treating cancer, the method using the chimeric antigen receptor or the immune cells including the same.

Still another aspect of the present disclosure is to provide use of the chimeric antigen receptor or the immune cells including the same for the prevention or treatment of cancer.

Still another aspect of the present disclosure is to provide use of the chimeric antigen receptor or the immune cells including the same for the manufacture of a medicament for preventing or treating cancer.

An aspect of the present disclosure is not limited to the aspects as mentioned above. The aspect of the present disclosure will become clearer from the following description, and will be realized by the means as described in the claims and combinations thereof.

### [Technical Solution]

Representative features of the present disclosure for achieving the above-mentioned aspects are as follows.

In accordance with an aspect of the present disclosure, there is provided a chimeric antigen receptor including a binding domain specifically binding to a CD300c antigen or a receptor thereof.

In accordance with another aspect of the present disclosure, there is provided an immune cell including the chimeric antigen receptor.

In accordance with still another aspect of the present disclosure, there is provided a nucleic acid encoding the chimeric antigen receptor.

In accordance with still another aspect of the present disclosure, there is provided a vector expressing the chimeric antigen receptor.

In accordance with still another aspect of the present disclosure, there is provided a pharmaceutical composition containing the chimeric antigen receptor or the immune cell including the same.

In accordance with still another aspect of the present disclosure, there is provided a method for preventing or treating cancer, the method including administering to a subject the chimeric antigen receptor or the immune cell including the same.

In accordance with still another aspect of the present disclosure, there is provided an anticancer therapy using the chimeric antigen receptor or the immune cells including the same.

In accordance with still another aspect of the present disclosure, there is provided use of the chimeric antigen receptor or the immune cell including the same for the prevention or treatment of cancer.

In accordance with still another aspect of the present disclosure, there is provided use of the chimeric antigen receptor or the immune cell including the same for the manufacture of a medicament for preventing or treating cancer.

### [Advantageous Effects]

The chimeric antigen receptor specifically binding to a CD300c antigen or a receptor thereof, according to the present disclosure, can specifically recognize cancer cells expressing the CD300c antigen or the CD300c receptor, and thus can inhibit the growth, metastasis, development, and the like of cancer in a direct and effective manner and can be effectively used for the treatment of various cancers expressing the CD300c antigen or the CD300c receptor on the surface thereof.

### [Brief Description of Drawings]

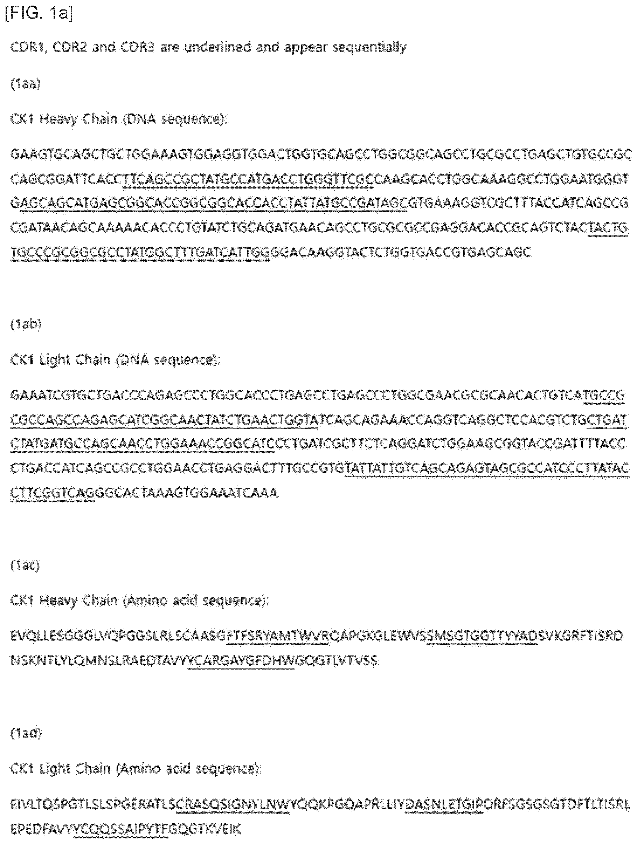

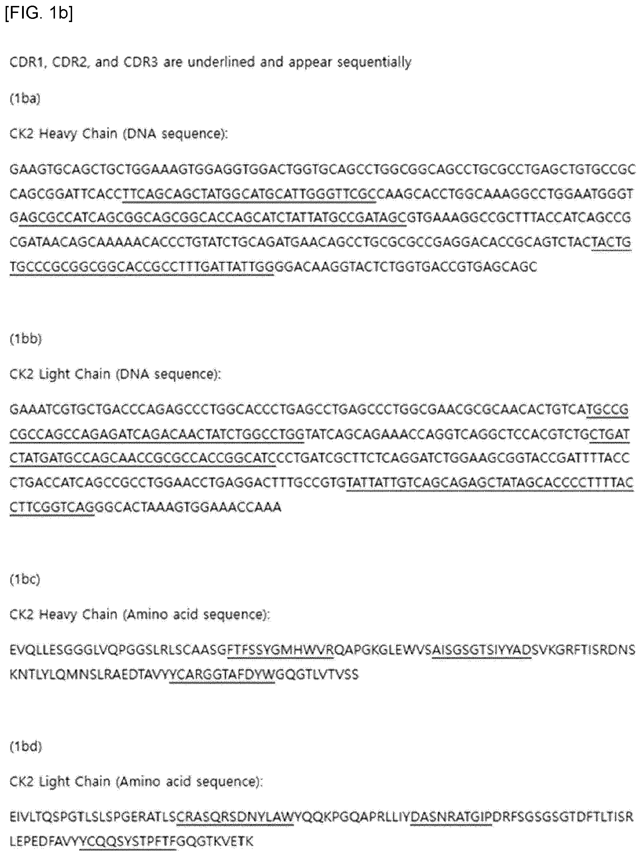

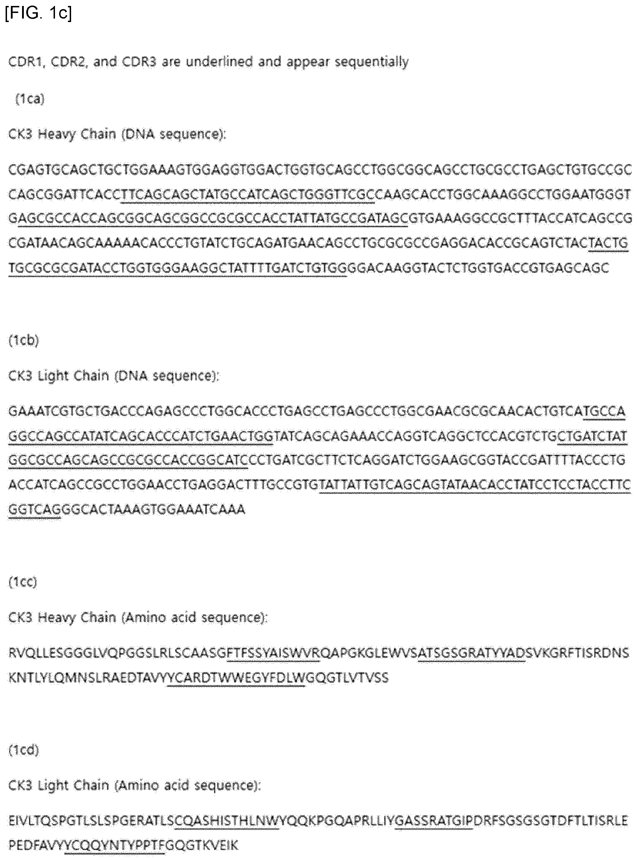

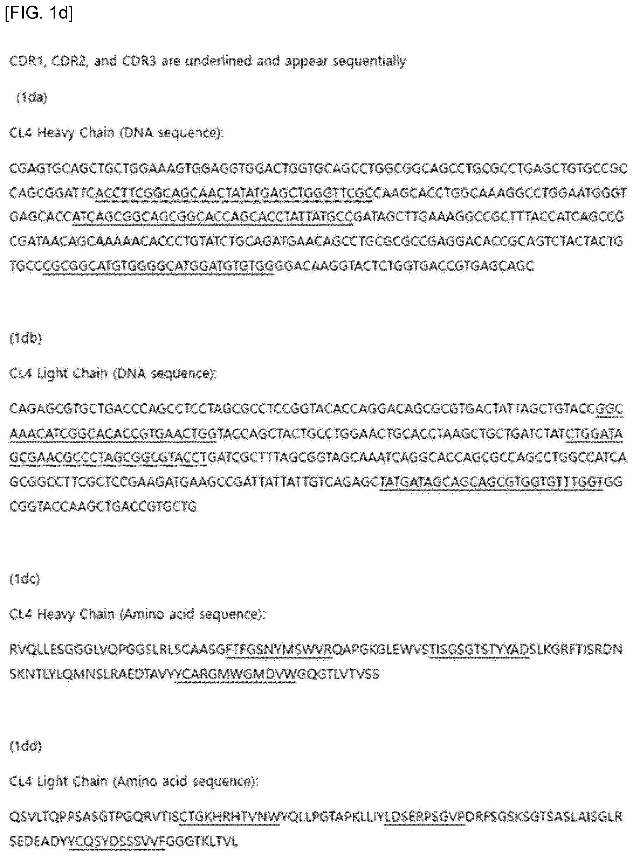

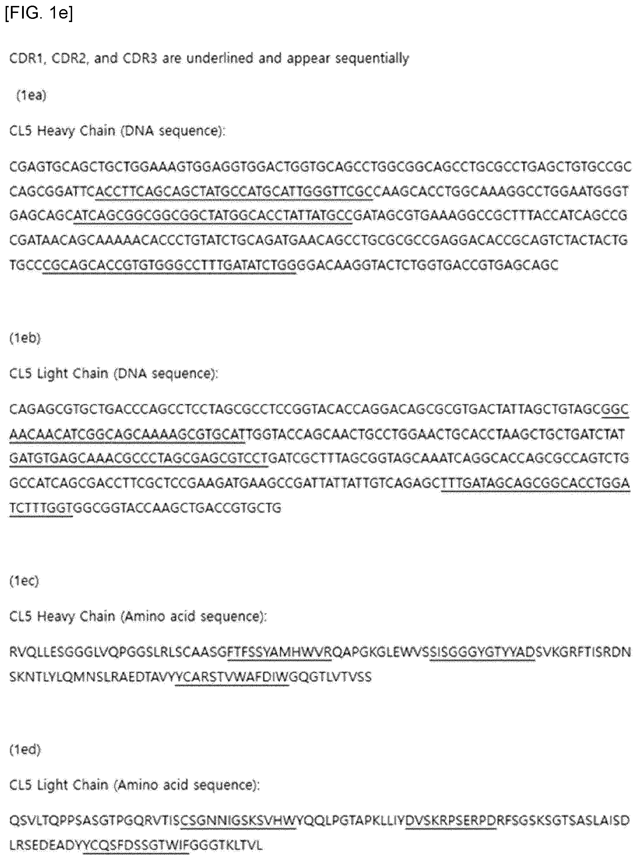

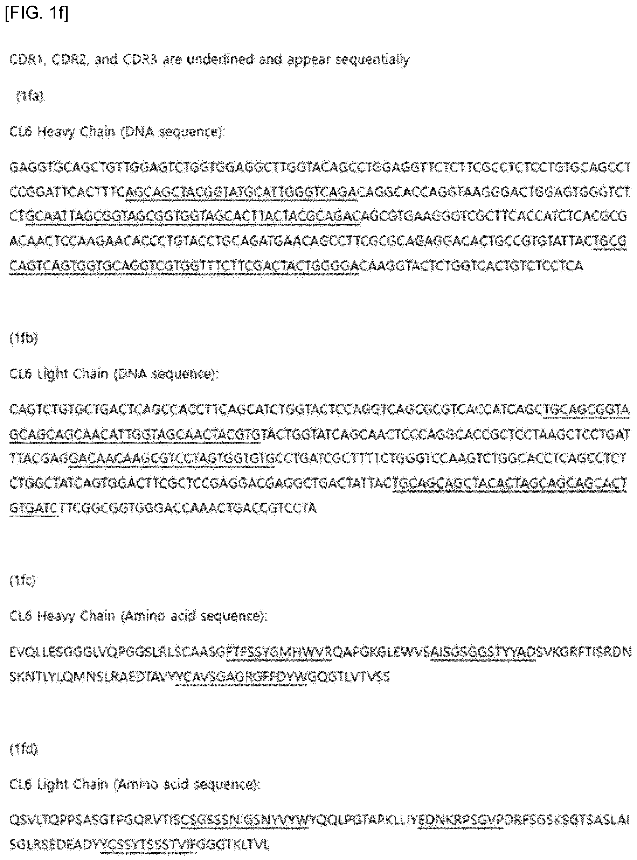

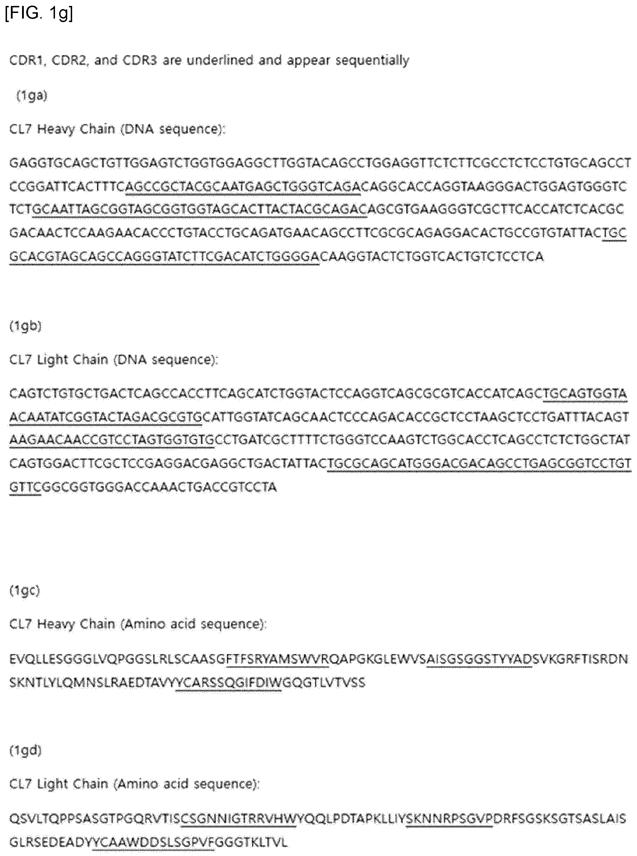

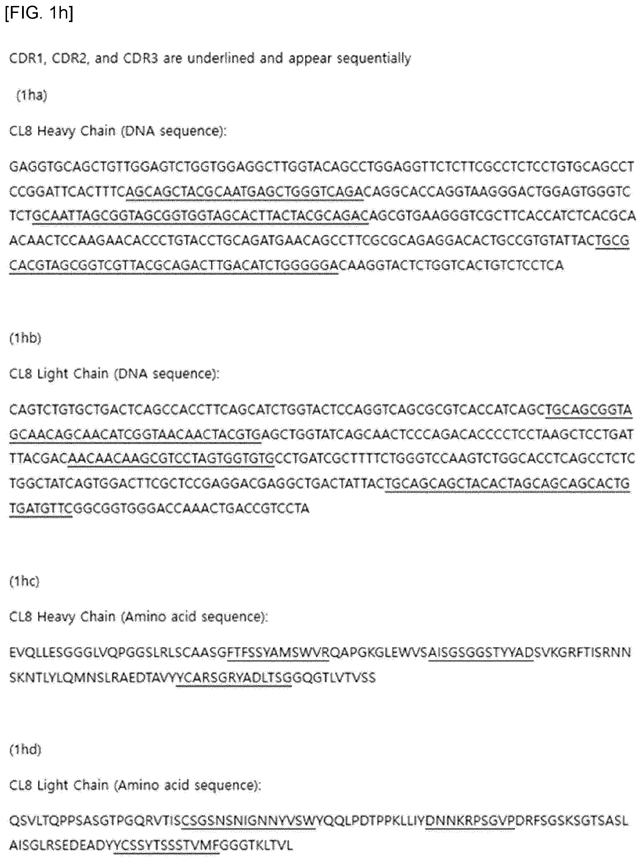

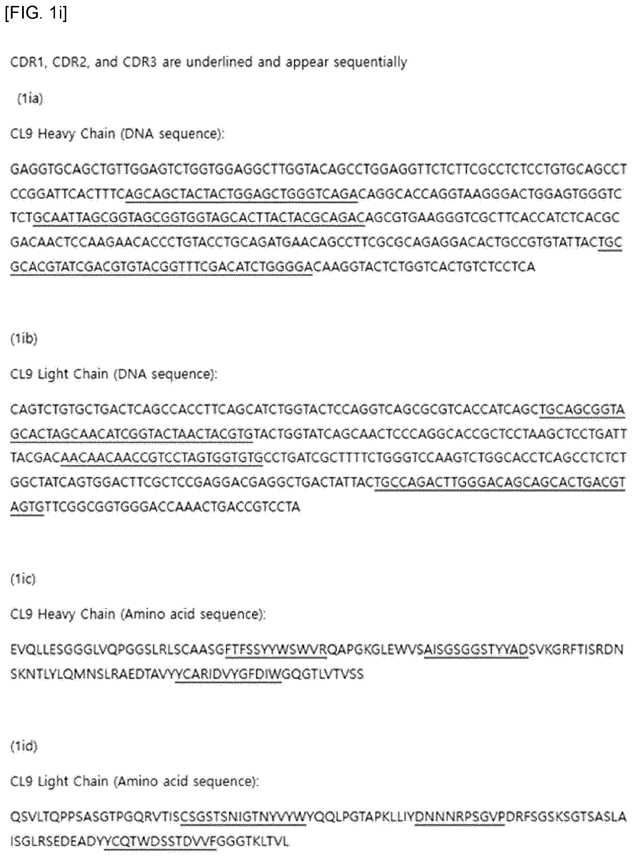

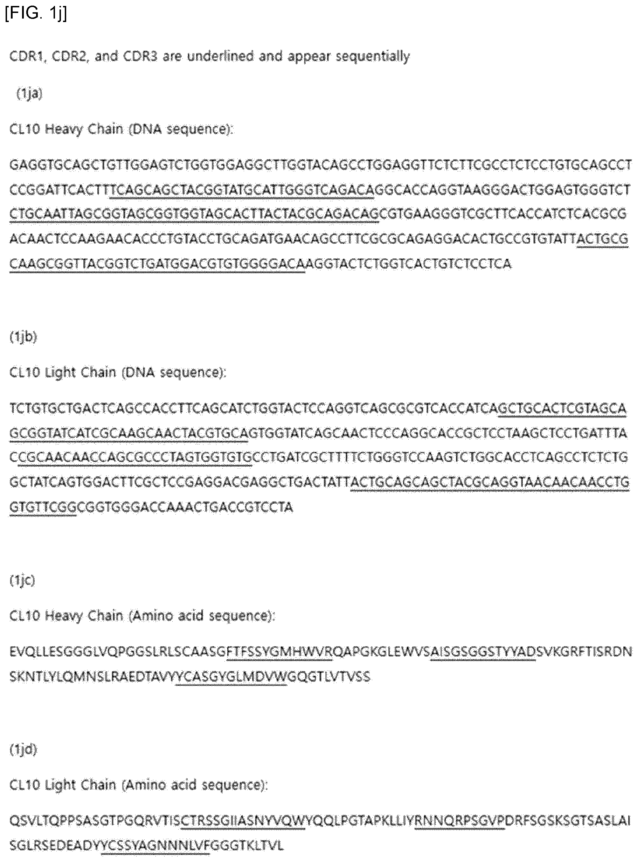

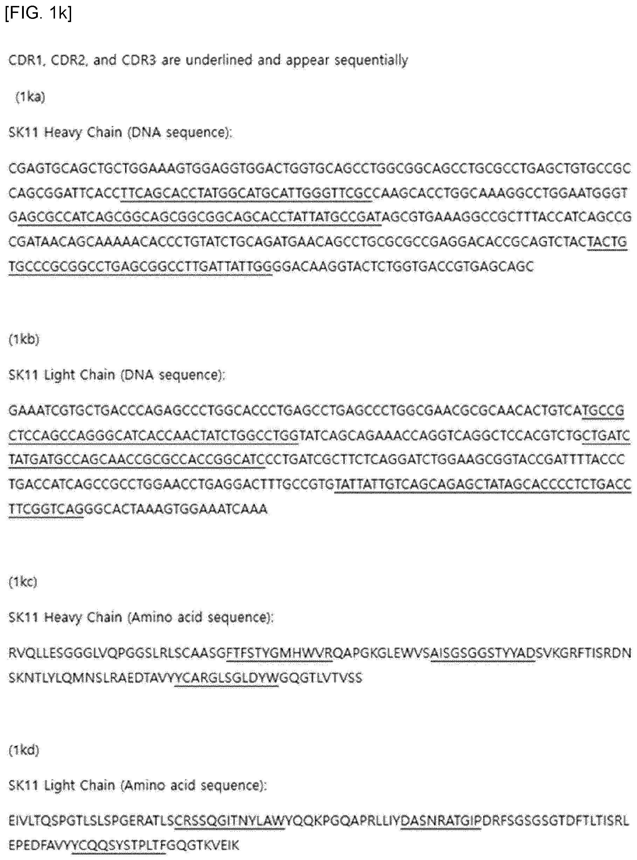

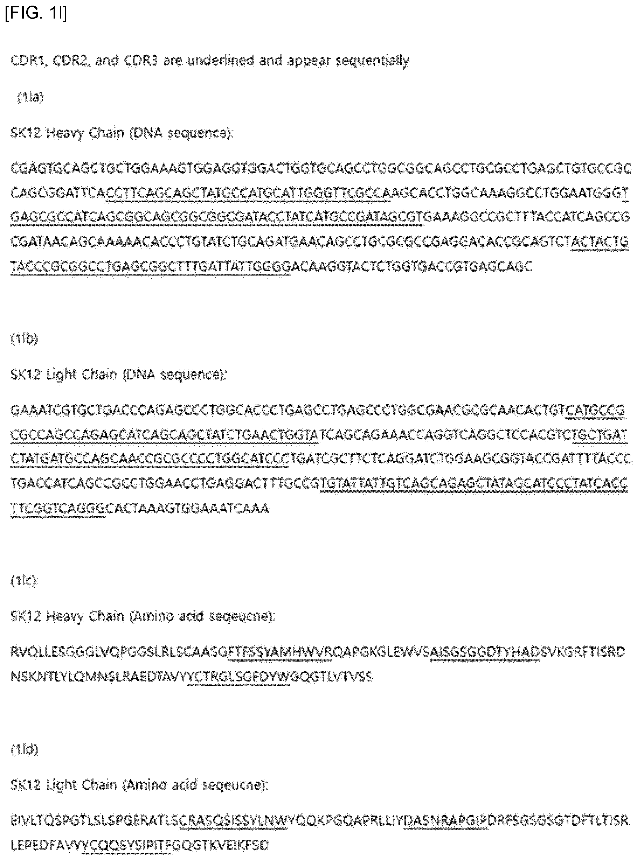

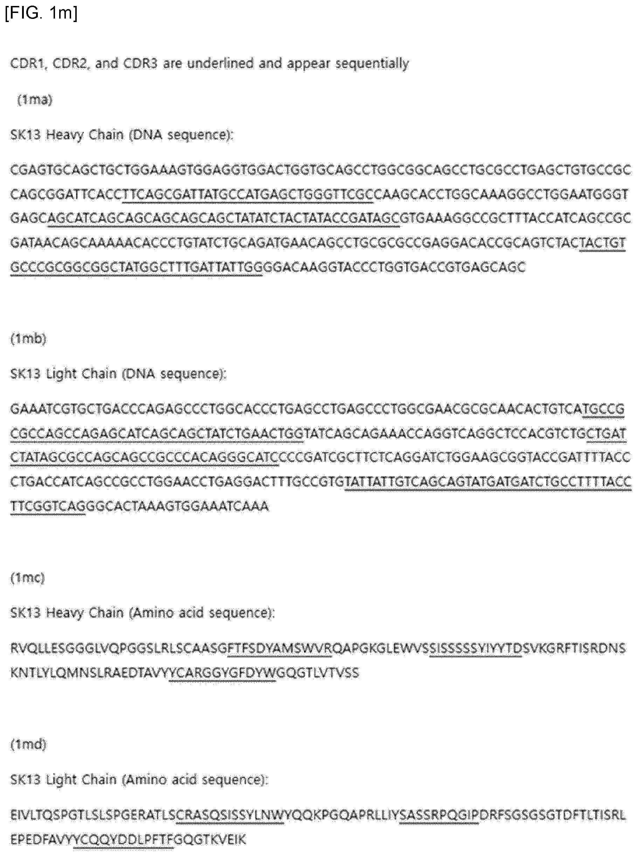

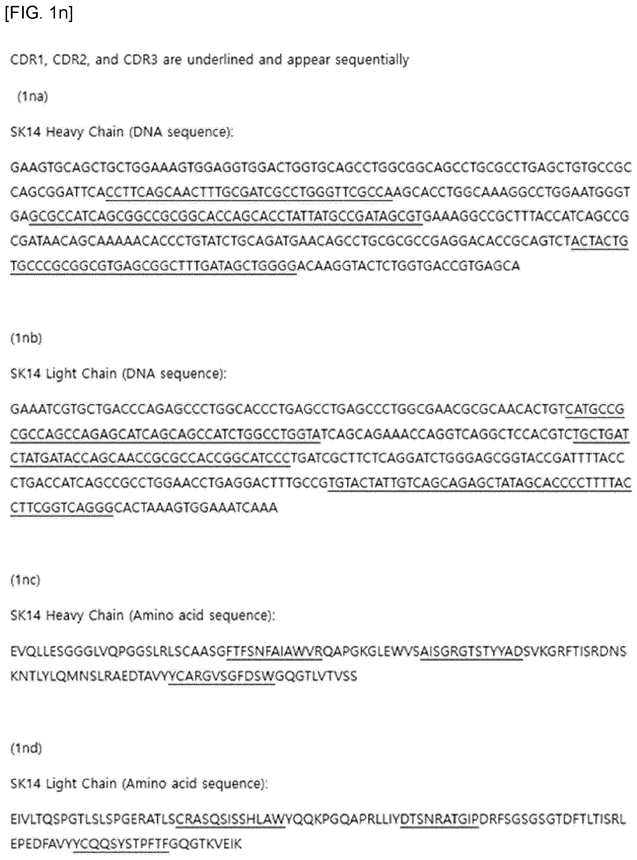

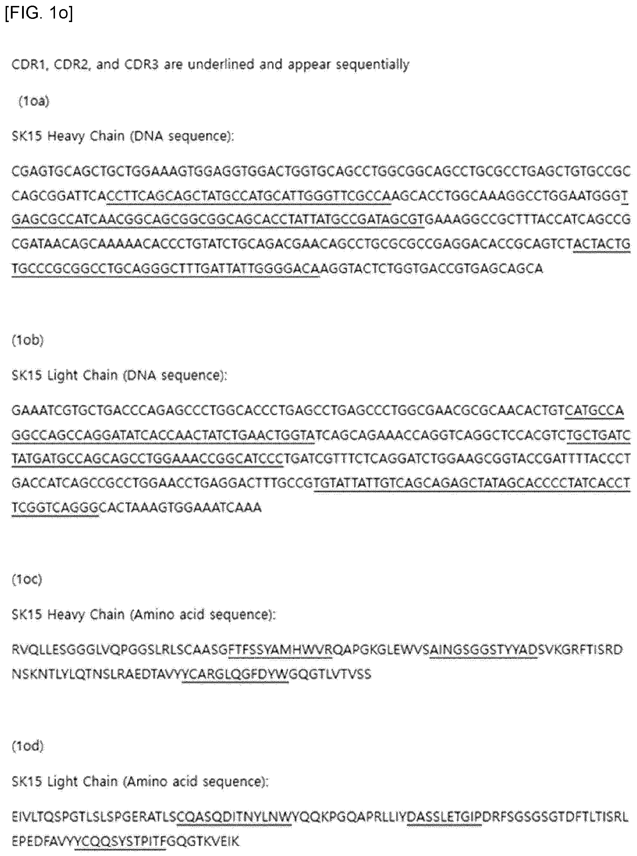

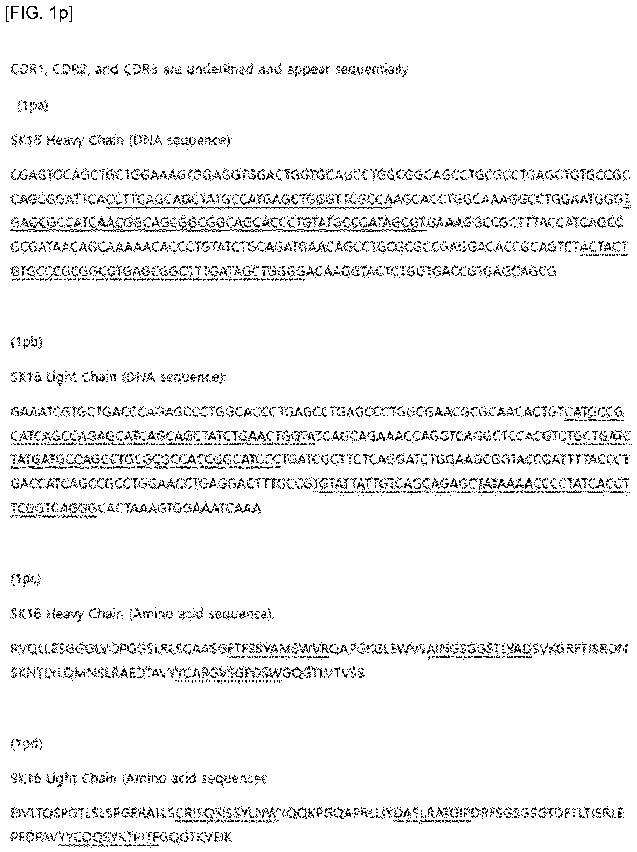

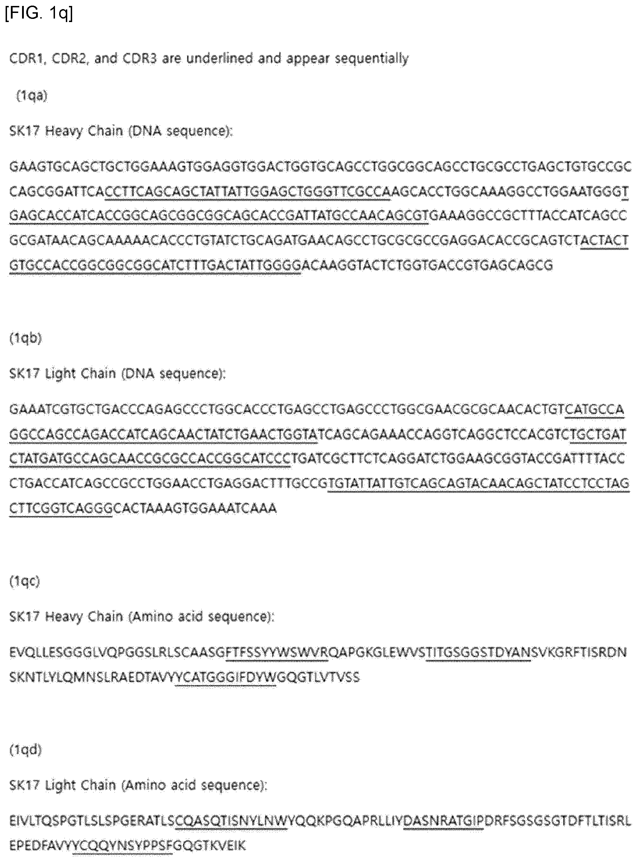

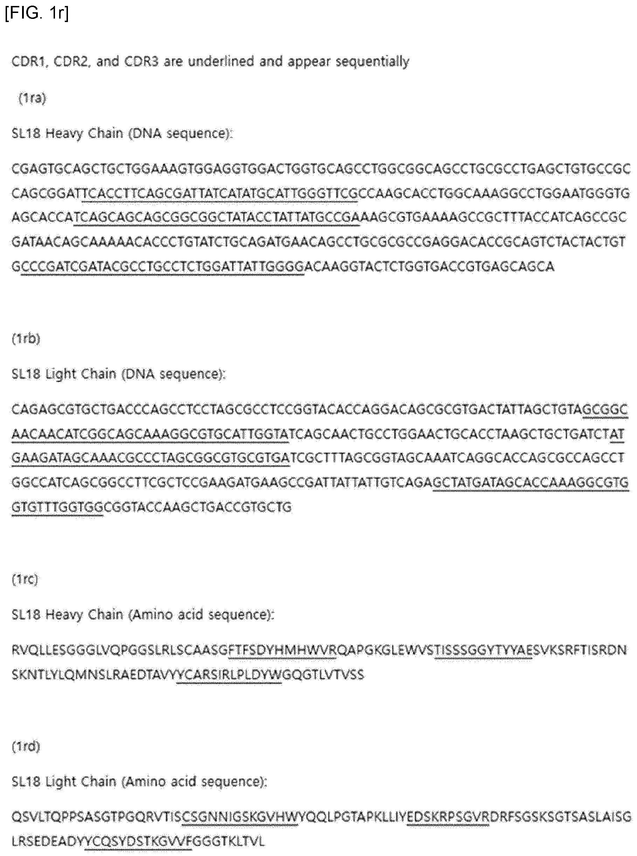

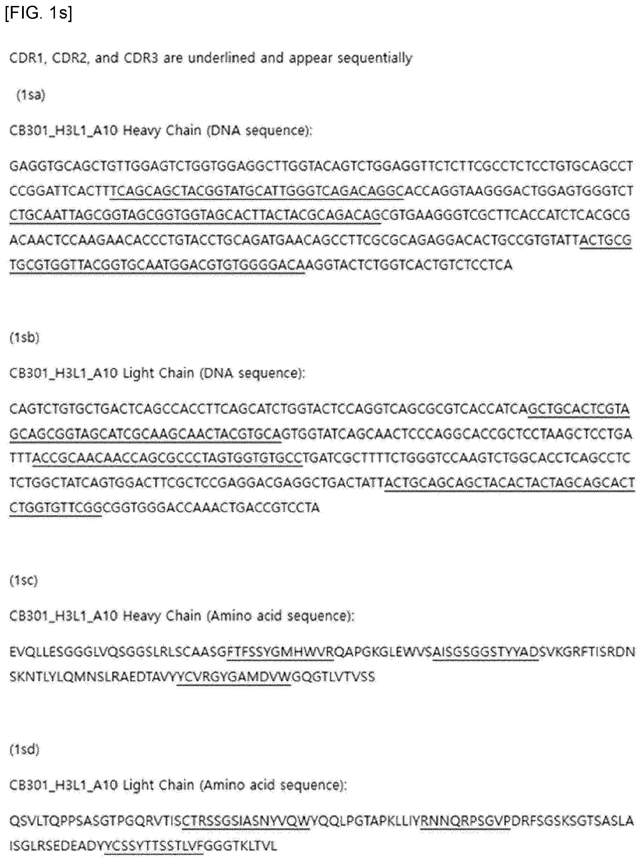

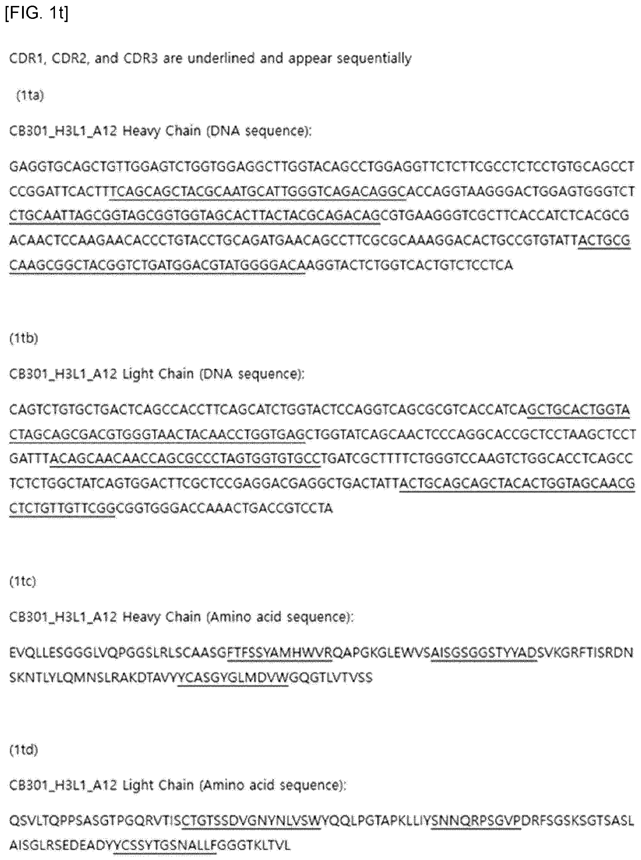

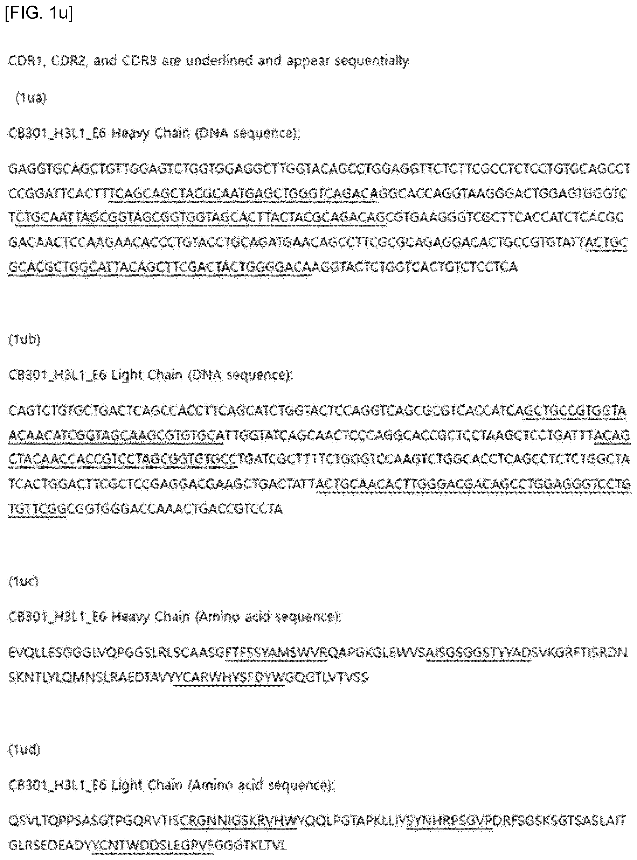

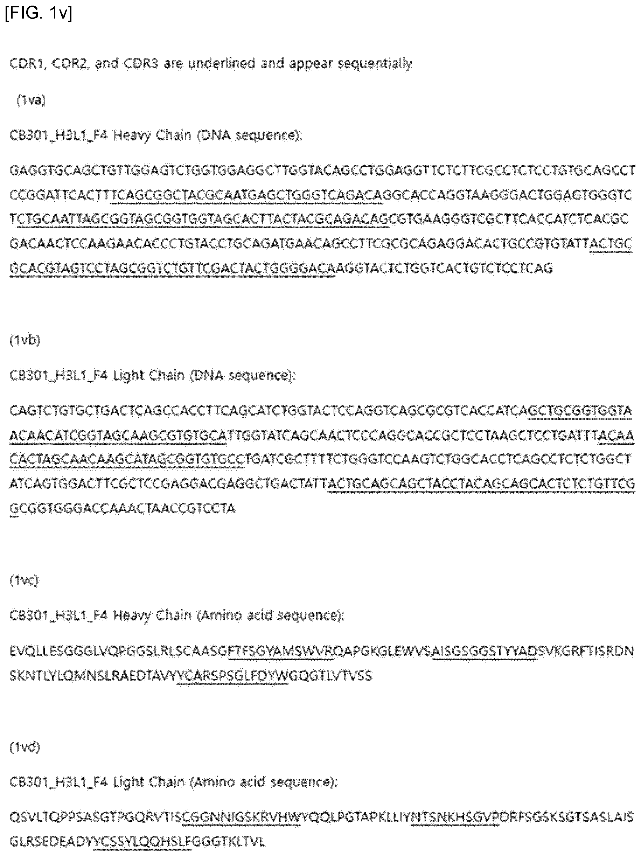

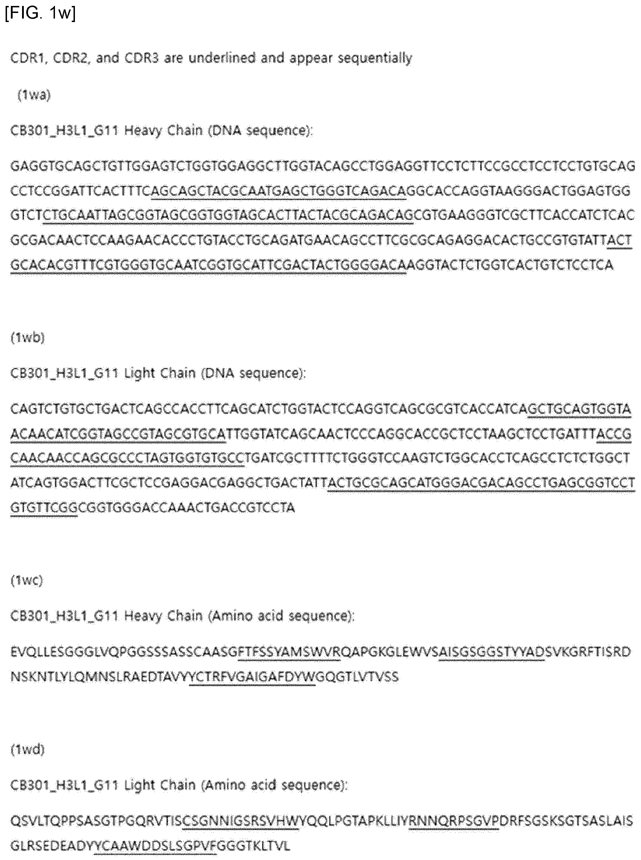

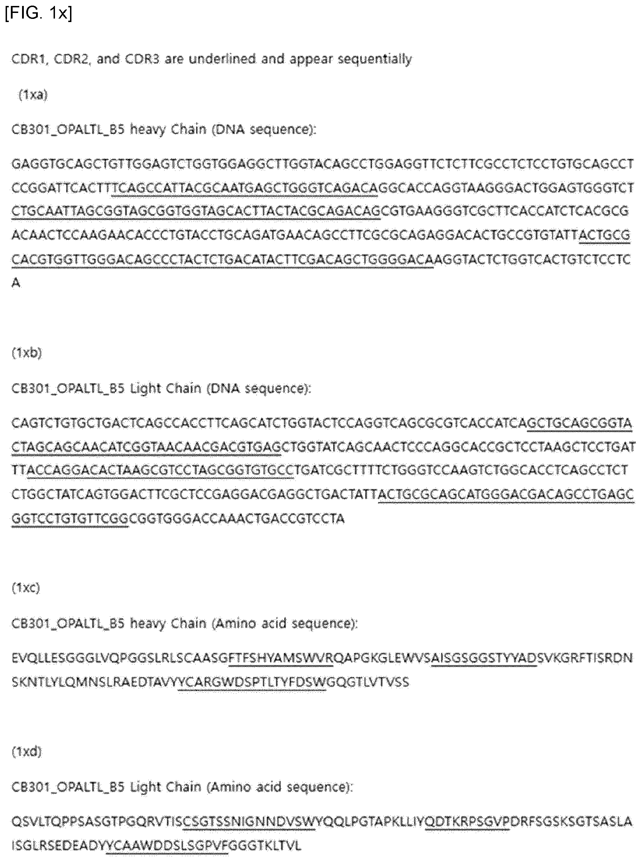

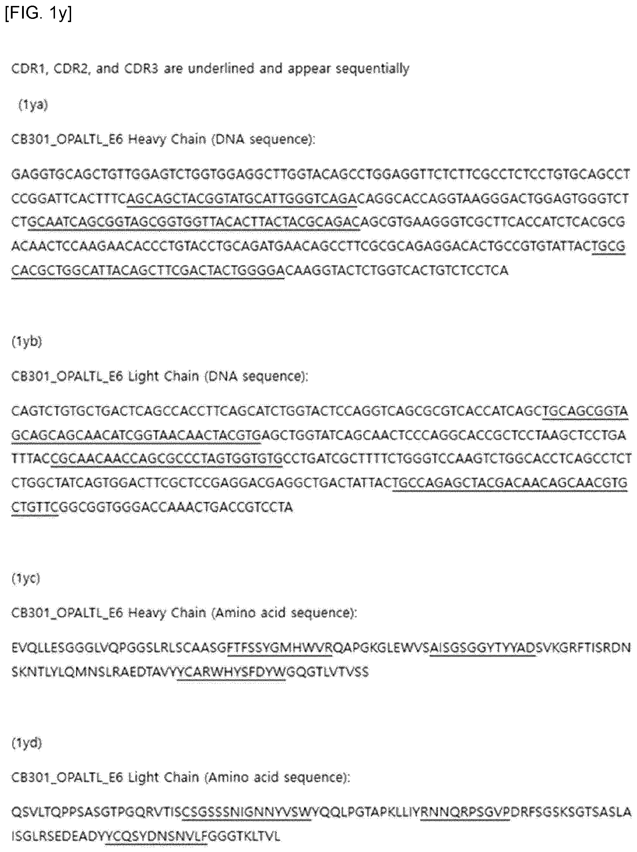

FIGS. 1a to 1y illustrate heavy chain and light chain variable region sequences (nucleic acid and amino acid sequences) of 25 types of anti-CD300c monoclonal antibodies according to the present disclosure, respectively. In each drawing, CDR regions (CDR1, CDR2, and CDR3) appear sequentially.

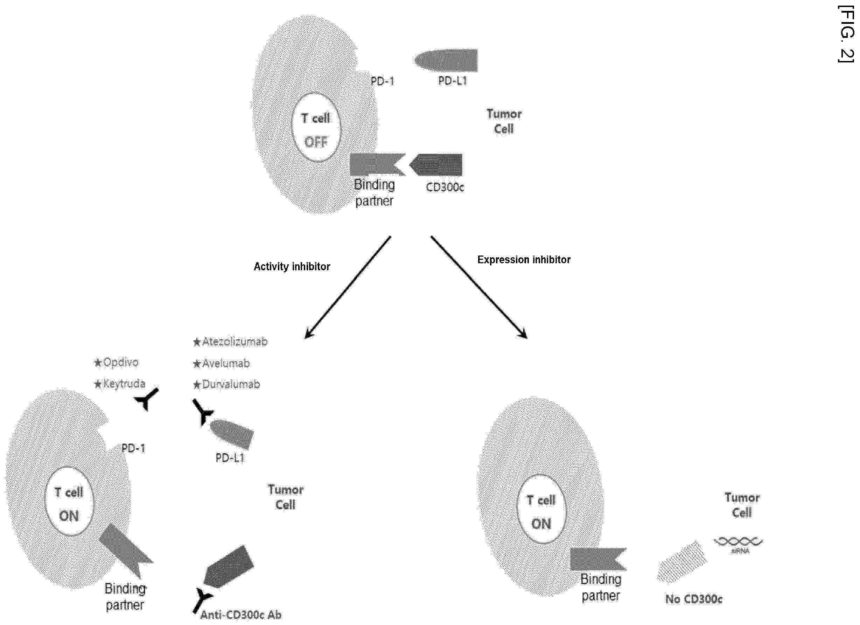

FIG. 2 is a schematic diagram briefly illustrating a mechanism whereby an anti-CD300c monoclonal antibody and/or CD300c siRNA of the present disclosure exhibits an anticancer effect.

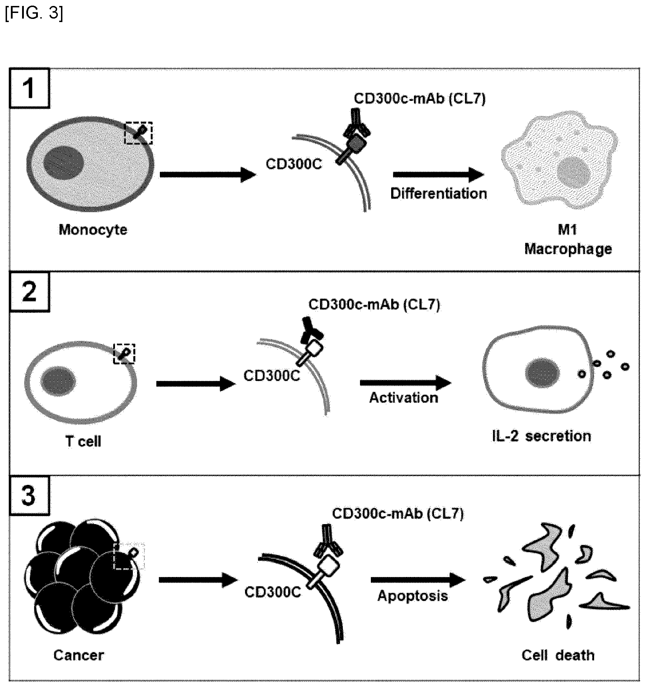

FIG. 3 is a schematic diagram briefly illustrating mechanisms whereby an anti-CD300c monoclonal antibody of the present disclosure acts on a monocyte, a T cell, and a cancer cell, separately.
FIG. 4 illustrates the SDS-PAGE results of anti-CD300c monoclonal antibodies under non-reducing conditions according to an embodiment of the present disclosure.
FIG. 5 illustrates the SDS-PAGE results of the anti-CD300c monoclonal antibodies under reducing conditions according to an embodiment of the present disclosure.
FIG. 6 illustrates the results obtained by comparing the expression of CD300c in normal cells, immune cells, and a cancer cell line, according to an embodiment of the present disclosure.
FIG. 7 illustrates the results obtained by examining the binding affinity, to a CD300c antigen, of the anti-CD300c monoclonal antibodies according to an embodiment of the present disclosure.
FIG. 8 illustrates the results obtained by examining the anticancer effect of an anti-CD300c monoclonal antibody through T cell activation according to an embodiment of the present disclosure.
FIGS. 9 and 10 illustrate the results obtained by examining the effect of anti-CD300c monoclonal antibodies on differentiation into M1 macrophages according to an embodiment of the present disclosure.
FIGS. 11 and 12 illustrate the results obtained by examining the concentration-dependent effect of anti-CD300c monoclonal antibodies on the differentiation into M1 macrophages according to an embodiment of the present disclosure.
FIG. 13 illustrates the results obtained by examining the effect of an anti-CD300c monoclonal antibody on the differentiation into M1 macrophages according to an embodiment of the present disclosure.
FIG. 14 illustrates the results obtained by re-examining whether an anti-CD300c monoclonal antibody promotes the differentiation of human monocytes into M1 macrophages according to an embodiment of the present disclosure.
FIGS. 15 to 18 illustrate the results obtained by comparing, using ELISA, the differentiation ability into M1 macrophages between anti-CD300c monoclonal antibodies and conventional immunotherapeutic agents according to an embodiment of the present disclosure.
FIG. 19 illustrates the results obtained by comparing, using ELISA, the differentiation ability from M0 macrophages into M1 macrophages between an anti-CD300c monoclonal antibody and an immunotherapeutic agent according to an embodiment of the present disclosure.
FIG. 20 illustrates the results obtained by comparing, using ELISA, the differentiation ability into M1 macrophages between an anti-CD300c monoclonal antibody and an immunotherapeutic agent according to an embodiment of the present disclosure.
FIGS. 21 to 23 illustrate the results obtained by examining, using ELISA, the redifferentiation(repolarization) ability of an anti-CD300c monoclonal antibody from M2 macrophages into M1 macrophages according to an embodiment of the present disclosure.
FIG. 24 illustrates the results obtained by examining the differentiation ability and redifferentiation(repolarization) ability, into M1 macrophages, of an anti-CD300c monoclonal antibody according to an embodiment of the present disclosure.
FIGS. 25 to 27 illustrate the results obtained by examining the signaling of MAPK (FIG. 25), NF-kB (FIG. 26), and IkB (FIG. 27), which are signals of M1 macrophage differentiation in the co-treatment with an anti-CD300c monoclonal antibody and an immunotherapeutic agent according to an embodiment of the present disclosure.
FIG. 28 illustrates the results obtained by examining the cancer cell growth inhibitory effect of anti-CD300c monoclonal antibodies under 0% FBS conditions according to an embodiment of the present disclosure.
FIG. 29 illustrates the results obtained by examining the cancer cell growth inhibitory effect of anti-CD300c monoclonal antibodies under 0.1% FBS conditions according to an embodiment of the present disclosure.
FIG. 30 illustrates the results obtained by examining the cancer (lung cancer) cell growth inhibitory effect of anti-CD300c monoclonal antibodies and an immunotherapeutic agent according to an embodiment of the present disclosure.
FIG. 31 illustrates the results obtained by examining the cancer (breast cancer) cell growth inhibitory effect of anti-CD300c monoclonal antibodies and an immunotherapeutic agent according to an embodiment of the present disclosure.
FIG. 32 illustrates the results obtained by examining the cancer cell growth inhibitory effect of an anti-CD300c monoclonal antibody depending on its concentration according to an embodiment of the present disclosure.
FIG. 33 illustrates the results obtained by examining the change in the apoptosis signal in the co-treatment with an anti-CD300c monoclonal antibody and an immunotherapeutic agent according to an embodiment of the present disclosure.
FIGS. 34 and 35 illustrate the results obtained by examining the cancer cell growth inhibitory effects in the co-treatment with an anti-CD300c monoclonal antibody and immunotherapeutic agents according to an embodiment of the present disclosure.
FIG. 36 illustrates binding ELISA results according to an embodiment of the present disclosure.
FIG. 37 illustrates the results obtained by examining whether anti-CD300c monoclonal antibodies can promote the differentiation from mouse macrophages into M1 macrophages according to an embodiment of the present disclosure.
FIG. 38 illustrates the results obtained by examining whether anti-CD300c monoclonal antibodies exhibit an anticancer effect in a mouse cancer cell line according to an embodiment of the present disclosure.
FIG. 39 schematically illustrates an experimental method used in an embodiment of the present disclosure.
FIG. 40 illustrates the cancer growth inhibitory effects *in vivo* observed when mice implanted with a colorectal cancer cell line were administered with an anti-CD300c monoclonal antibody and an anti-PD-1 antibody alone or in combination according to an embodiment of the present disclosure.
FIG. 41 illustrates the results obtained by examining whether an anti-CD300c monoclonal antibody stimulates the CD8+ T cell immunity in mouse tumor models according to an embodiment of the present disclosure.
FIG. 42 illustrates the results obtained by examining whether an anti-CD300c monoclonal antibody increases M1 macrophages in cancer tissues in mouse models according to an embodiment of the present disclosure.
FIG. 43 briefly illustrates a gene arrangement for constructing a chimeric antigen receptor specifically binding to a CD300c antigen or a receptor thereof according to an embodiment of the present disclosure.
FIG. 44 briefly illustrates a vector map for constructing a chimeric antigen receptor specifically binding to a CD300c antigen or a receptor thereof according to an embodiment of the present disclosure.
FIG. 45 illustrates the results obtained by examining, using Western blotting, Jurkat cell line expressing a chimeric antigen receptor specifically binding to a CD300c antigen or a receptor thereof according to an embodiment of the present disclosure.
FIG. 46 illustrates the results obtained by examining the anticancer effects of Jurkat cell line expressing a chimeric antigen receptor specifically binding to a CD300c antigen or a receptor thereof according to an embodiment of the present disclosure.

### [Best Mode for Carrying Out the Invention]

The following detailed description of the present disclosure will be described, with reference to specific drawings, for specific embodiments in which the present disclosure may be practiced. However, the present disclosure is not limited thereto, and the scope of the present disclosure is defined only by the appended claims, appropriately interpreted, along with the full range of equivalents to which the claims are entitled. It is to be understood that various embodiments of the present disclosure, although different from each other, are not necessarily mutually exclusive. For example, a particular feature, structure, or characteristic described herein may vary from one embodiment to another or be implemented as a combination of embodiments without departing from the spirit and scope of the present disclosure. Technical and scientific terms used herein have the same meanings as commonly used in the art to which the present disclosure belongs, unless otherwise defined. For the purpose of interpreting this specification, the following definitions will apply, and the singular forms include plural referents and vice versa unless the context clearly dictates otherwise.

### Definitions

As used herein, the term "about" means within an acceptable error range for the particular value which is known to one of ordinary skill in the art.

The term "(antigen-) binding domain" refers to a portion of a protein which binds to an antigen. The antigen-binding domain may be a synthetic polypeptide, an enzymatically obtainable polypeptide, or a genetically engineered polypeptide, and may be an immunoglobulin (for example, an antibody) or a portion thereof (for example, an antigen-binding fragment) which binds to an antigen.

The term "antibody" is used broadly and includes monoclonal antibodies (including full length antibodies) of any isotype such as IgG, IgM, IgA, IgD, and IgE, polyclonal antibodies, multispecific antibodies (for example, bispecific antibodies), antibody fusions (for example, a fusion of an antibody with a (poly)peptide or a fusion of an antibody with a compound), and antibody fragments (including antigen-binding fragments). As used herein, the prefix "anti-", when in conjunction with an antigen, indicates that the given antibody is reactive with the given antigen. An antibody reactive with a specific antigen can be generated, without limitation, by synthetic and/or recombinant methods such as selection of libraries of recombinant antibodies in phage or similar vectors, or by immunizing an animal with the antigen or an antigen-encoding nucleic acid. A typical IgG antibody is composed of two identical heavy chains and two identical light chains that are joined by disulfide bonds. Each of the heavy and light chains contains a constant region and a variable region. Heavy chain variable regions (HVRs) and light chain variable regions (LVRs) contain three segments, referred to as "complementarity determining regions" ("CDRs") or "hypervariable regions", respectively, which are primarily responsible for binding an epitope of an antigen. These are usually referred to as CDR1, CDR2, and CDR3, numbered sequentially from the N-terminus. The more highly conserved portions of the variable regions outside of the CDRs are called the "framework regions" ("FRs"). An antibody herein may be, for example, an animal antibody, a chimeric antibody, a humanized antibody, or a human antibody.

The term "single domain antibody" is an antibody specific for the CD300c antigen, in which a CDR is a portion of a single domain polypeptide, and may be generally produced using only two heavy chains and an antigen-binding site. However, the single domain antibody may include all of antibodies naturally devoid of light chains, single-domain antibodies derived from conventional 4-chain antibodies, engineered antibodies, and single domain scaffolds other than those derived from antibodies.

The term "single-chain variable fragment (scFv)" refers to a protein in which light chain and heavy chain variable regions of an antibody are linked to each other via a linker consisting of a peptide sequence having about 15 amino acid residues. The scFv may be in an order of light chain variable domain-linker-heavy chain variable region, or an order of heavy chain variable region-linker-light chain variable region, and has the same or similar antigen specificity as its original antibody. The linking site is a hydrophilic flexible peptide chain (SG linker) mainly composed of glycine and serine. The 15-amino acid sequence of "(Gly-Gly-Gly-Gly-Ser)3" or a sequence similar thereto is mainly used. The antibody refers to an immunoglobulin molecule that is immunologically reactive with a specific antigen, and includes all of polyclonal antibodies, monoclonal antibodies, and functional fragments thereof. In addition, the term may include forms produced by genetic engineering, such as chimeric antibodies (for example, humanized murine antibodies) and heterologous antibodies (for example, bispecific antibodies). Among these, the monoclonal antibodies are antibodies that exhibit single binding specificity and affinity for a single antigenic site (epitope). Unlike polyclonal antibodies including antibodies that exhibit specificity for different epitopes, the monoclonal antibodies exhibit binding specificity and affinity for a single epitope on an antigen, which allows for easy quality control as a therapeutic. In particular, the anti-CD300c monoclonal antibody of the present disclosure not only exhibits anticancer activity by itself by specifically binding to CD300c-expressing cancer cells, but also exhibits maximized cancer cell-dependent anticancer activity by stimulating immune cells. The antibody includes variable region(s) of a heavy chain and/or a light chain in terms of the constitution, wherein the variable region includes, as a primary structure thereof, a portion that forms an antigen-binding site of the antibody molecule. The antibody of the present disclosure may be composed of a partial fragment containing the variable region.

The term "humanization" (also called reshaping or CDR-grafting) includes a well-established technique for reducing the immunogenicity of monoclonal antibodies from xenogeneic sources (commonly rodent) and for improving their affinity or effector function (ADCC, complement activation, Clq binding).

The term "monoclonal antibody" as used herein refers to an antibody obtained from a population of substantially homogeneous antibodies, that is, the individual antibodies constituting the population are identical except for possible naturally occurring mutations and/or post-translation modifications (for example, isomerization or amidation) that may be present in minor amounts. Monoclonal antibodies are highly specific and are directed for a single antigenic site. The monoclonal antibody is obtained from a substantially homogeneous population of antibodies, displays the nature of an antibody, and is not to be construed as requiring production of the antibody by any particular method. For example, monoclonal antibodies to be used according to the present disclosure may be constructed by a variety of techniques, including but not limited to, hybridoma methods, recombinant DNA methods, phage-display methods, and methods utilizing transgenic animals containing all or part of the human immunoglobulin loci.

The term "antigen-binding fragment" refers to a portion of an antibody having specific binding ability to an antigen or a polypeptide including the same. The terms "antibody" and "antigen-binding fragment" may be used interchangeably except for a case where it is understood in the context that the "antibody" specifically excludes the "antigen-binding fragment," and the "antibody" may be construed as including the "antigen-binding fragment". Examples of the antigen-binding fragment include, but are not limited to, Fv, Fab, Fab', Fab'-SH, F(ab')2, diabodies, triabodies, tetrabodies, cross-Fab fragments, linear antibodies, single chain antibody molecules (for example, scFv), and multispecific antibodies formed of antibody fragments and single domain antibodies.

The term "chimeric antigen receptor" or "CAR" is defined as a cell surface receptor that includes an extracellular target-binding domain, a transmembrane domain, and an intracellular signaling domain. The chimeric antigen receptor of the present disclosure is intended primarily for use with lymphocytes, such as T cells and natural killer (NK) cells.

The term "anticancer agent" collectively refers to known medications used in conventional cancer treatment which act on various metabolic pathways of cells and exhibit cytotoxic or cytostatic effects on cancer cells. The anticancer agent encompasses chemotherapeutic agents, targeted therapeutic agents, and immunotherapeutic agents.

The term "immunotherapeutic agent" refers to a medication which activates immune cells to kill cancer cells.

The term "subject" is used interchangeably with "patient" and may be a mammal, which is in need of prevention or treatment of cancer, such as a primate (for example, a human), a companion animal (for example, a dog, a cat, etc.), livestock (for example, a cow, a pig, a horse, sheep, a goat, etc.), and a laboratory animal (for example, a rat, a mouse, a guinea pig, etc.). In an embodiment of the present disclosure, the subject is a human.

The term "treatment" generally means obtaining a desired pharmacological and/or physiological effect. The effect may be therapeutic in terms of partially or completely curing a disease and/or an adverse effect attributed to the disease. Desirable therapeutic effects include, but are not limited to, prevention of onset or recurrence of disease, alleviation of symptoms, diminishment of any direct or indirect pathological consequences of the disease, prevention of metastasis, decreasing the rate of disease progression, amelioration or slowing of the disease state, and remission or improved prognosis. Preferably, the "treatment" may refer to medical intervention of a disease or disorder that has already developed.

The term "prevention" is directed to a prophylactic treatment, that is, to a measure or procedure, the purpose of which is to prevent, rather than to cure a disease. The term "prevention" means that a desired pharmacological and/or physiological effect is obtained which is prophylactic in terms of completely or partially preventing a disease or symptom thereof.

The term "administration" means providing a substance (for example, an anti-CD300c antibody or an antigen-binding fragment thereof and other anticancer agents) to a subject to achieve a prophylactic or therapeutic purpose (for example, prevention or treatment of cancer).

The term "biological sample" encompasses a variety of sample types obtained from a subject and may be used in diagnostic or monitoring assays. The biological sample includes, but is not limited to, blood and other liquid samples of biological origin, and solid tissue samples such as a biopsy specimen or tissue cultures or cells derived therefrom and the progeny thereof. Therefore, the biological sample encompasses a clinical sample, and also includes cells in culture, cell supernatants, cell lysates, serum, plasma, biological fluid, and tissue samples, particularly, tumor samples.

### Chimeric Antigen Receptor

According to an aspect of the present disclosure, there is provided a chimeric antigen receptor including a binding domain specifically binding to a CD300c antigen or a receptor thereof. The chimeric antigen receptor (CAR) is an artificially constructed hybrid protein or polypeptide which contains an antigen-binding domain of an antibody (for example, scFv) linked to a T-cell signaling domain. The chimeric antigen receptor can induce T-cell specificity and reactivity towards a selected target in a non-MHC-restricted manner by exploiting the antigen-binding ability of a monoclonal antibody. The chimeric antigen receptor may include an (extracellular) antigen-binding domain, a transmembrane domain, and an intracellular signaling domain. In addition, the chimeric antigen receptor may further include a GS linker. In addition, the chimeric antigen receptor may further include a signal peptide. In an embodiment, the chimeric antigen receptor may include an (extracellular) antigen-binding domain, a GS linker, a transmembrane domain, and an intracellular signaling domain. In another embodiment, the chimeric antigen receptor may include an (extracellular) antigen-binding domain, a signal peptide, a GS linker, a transmembrane domain, and an intracellular signaling domain. In addition to the components listed above, the chimeric antigen receptor of the present disclosure may include any component of chimeric antigen receptors commonly known in the art.

In an embodiment, the binding domain may include any one or more selected from the group consisting of an antibody, a single domain antibody, and a single chain variable fragment, each of which specifically binds to the CD300c antigen or a receptor thereof, and an antigen.

Specifically, the binding domain may be a CD300c antigen. The CD300c antigen may include the entire CD300c antigen sequence or an extracellular domain (ECD) of the CD300c antigen sequence, for binding to a receptor thereof. The extracellular domain sequence of the CD300c antigen may include or consist of the amino acid sequence represented by SEQ ID NO: 402. In addition, the extracellular domain sequence may include an amino acid sequence that has 70% or more, 75% or more, 80% or more, 85% or more, 90% or more, 95% or more, or 98% or more sequence identity to SEQ ID NO: 402.

In addition, the binding domain may be an anti-CD300c antibody (preferably an anti-CD300c monoclonal antibody) or an antigen-binding fragment thereof. However, the binding domain may include any substance as long as it can specifically bind to the CD300c antigen or a receptor thereof. In this regard, in a case where the binding domain is an antibody or an antigen-binding fragment thereof, such binding domain may be prepared by any antibody production technique known in the art.

In an embodiment, the binding domain may include:
(i) a heavy chain variable region including: CDR1 including or consisting of an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
   CDR2 including or consisting of an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
   CDR3 including or consisting of an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region including: CDR1 including or consisting of an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
   CDR2 including or consisting of an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
   CDR3 including or consisting of an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

In another embodiment, (i) the heavy chain variable region may include:
CDR1 including or consisting of the amino acid sequence represented by SEQ ID NO: 7, SEQ ID NO: 67, SEQ ID NO: 79, SEQ ID NO: 115, or SEQ ID NO: 211;
CDR2 including or consisting of the amino acid sequence represented by SEQ ID NO: 8, SEQ ID NO: 68, SEQ ID NO: 80, SEQ ID NO: 116, or SEQ ID NO: 212; and
CDR3 including or consisting of the amino acid sequence represented by SEQ ID NO: 9, SEQ ID NO: 69, SEQ ID NO: 81, SEQ ID NO: 117, or SEQ ID NO: 213, and
(ii) the light chain variable region may include:
CDR1 including or consisting of the amino acid sequence represented by SEQ ID NO: 10, SEQ ID NO: 70, SEQ ID NO: 82, SEQ ID NO: 118, or SEQ ID NO: 214;
CDR2 including or consisting of the amino acid sequence represented by SEQ ID NO: 11, SEQ ID NO: 71, SEQ ID NO: 83, SEQ ID NO: 119, or SEQ ID NO: 215; and
CDR3 including or consisting of the amino acid sequence represented by SEQ ID NO: 12, SEQ ID NO: 72, SEQ ID NO: 84, SEQ ID NO: 120, or SEQ ID NO: 216.

In still another embodiment, the heavy chain variable region may include the amino acid sequence represented by SEQ ID NO: 303, SEQ ID NO: 323, SEQ ID NO: 327, SEQ ID NO: 339 or SEQ ID NO: 371, and the light chain variable region may include the amino acid sequence represented by SEQ ID NO: 304, SEQ ID NO: 324, SEQ ID NO: 328, SEQ ID NO: 340, or SEQ ID NO: 372. Preferably, the heavy chain variable region may include the amino acid sequence represented by SEQ ID NO: 303 and the light chain variable region may include the amino acid sequence represented by SEQ ID NO: 304; the heavy chain variable region may include the amino acid sequence represented by SEQ ID NO: 323 and the light chain variable region may include the amino acid sequence represented by SEQ ID NO: 324; the heavy chain variable region may include the amino acid sequence represented by SEQ ID NO: 327 and the light chain variable region may include the amino acid sequence represented by SEQ ID NO: 328; the heavy chain variable region may include the amino acid sequence represented by SEQ ID NO: 339 and the light chain variable region may include the amino acid sequence represented by SEQ ID NO: 340; the heavy chain variable region may include the amino acid sequence represented by SEQ ID NO: 371 and the heavy chain variable region may include the amino acid sequence represented by SEQ ID NO: 372.

In still another embodiment, the binding domain of the chimeric antigen receptor may include a heavy chain variable region including CDR1 to CDR3 including or consisting of amino acid sequences represented by Formulas (1) to (3), respectively, and a light chain variable region including CDR1 to CDR3 including or consisting of amino acid sequences represented by Formulas (4) to (6), respectively (each amino acid sequence is shown in N→C direction):
FTFSX1YX2MX3WVR (1) (SEQ ID NO: 403)
   wherein,
   X1= R, S, or D
   X2= A, G, or H
   X3= T, H, or S
X1X2SX3X4GGX5TYYAX6 (2) (SEQ ID NO: 404)
   wherein,
   X1= S, A, or T
   X2= M or I
   X3= G or S
   X4= T or S
   X5= T, S, or Y
   X6= D or E
YCAX1X2X3X4X5X6X7X8X9X10X11W (3) (SEQ ID NO: 405)
   wherein,
   X1= R, V, or S
   X2= G or S
   X3= A, G, S, Y, or I
   X4= Y, A, Q, G, or R
   X5= G or L
   X6= F, R, I, M, or P
   X7= D, G, F, or L
   X8= H, F, D, or V
   X9= F, I, Y or not present
   X10= D or not present
   X11= Y or not present
CX1X2X3X4X5X6X7X8X9X10X11X12X13W (4) (SEQ ID NO: 406)
   wherein,
   X1= R, S, or T
   X2= A, G, or R
   X3= S or N
   X4= Q, S, or N
   X5= S, I, or G
   X6= I, N, or G
   X7= G, I, T, or S
   X8= N, G, R, A, or K
   X9= Y, S, R, or G
   X10= N or not present
   X11= Y or not present
   X12= L or V
   X13= N, Y, H, or Q
X1X2X3X4X5X6X7GX8X9 (5) (SEQ ID NO: 407)
   wherein,
   X1= D, E, S, or R
   X2= A, D, K, or N
   X3= S or N
   X4= N, K, or Q
   X5= L or R
   X6= E or P
   X7= T or S
   X8= I or V
   X9= P or R
YCX1X2X3X4X5X6X7X8X9X10X11F (6) (SEQ ID NO: 408)
   wherein,
   X1= Q, S, or A
   X2= Q, S, or A
   X3= S, Y, or W
   X4= S, T, D, or A
   X5= A, S, D, or G
   X6= I, S, N, or T
   X7= P, S, L, N, or K
   X8= Y, T, S, N, or G
   X9= V, G, L or not present
   X10 = P or not present
   X11= T, I, or V.

In a specific embodiment, the binding domain may be a single chain variable segment (scFv) and may include or consist of an amino acid sequence selected from the group consisting of SEQ ID NOs: 412, 414, 416, 418, 420, 422, 424, 426, 428, 430, 432, 434, 436, 438, and 440. Preferably, the binding domain may include or consist of SEQ ID NO: 412, 414, 416, 418, or 440.

The binding domain may include a sequence having 80% or more, preferably 90% or more, more preferably 95% or more, and most preferably 98% or more sequence identity to any of the above-described amino acid sequences.

In a specific embodiment, amino acid sequence variants of the antibodies of the present disclosure are contemplated. For example, it may be desirable to improve the binding affinity and/or other biological properties of the antibody. Amino acid sequence variants of the antibody may be prepared by introducing appropriate modifications into the nucleotide sequences encoding molecules, or by peptide synthesis. Such modifications include, for example, deletions of residues from the amino acid sequence of the antibody, and/or insertions of residues into such amino acid sequences, and/or substitutions of residues within such amino acid sequences. Any combination of various modifications including deletion, insertion, and substitution can be made to arrive at final constructs, provided that the final constructs possess desired characteristics, for example, antigen-binding characteristics. Sites of interest for substitutional mutagenesis include heavy chain variable regions (HVRs) and framework regions (FRs). Conservative substitutions are provided in Table 1 under the heading "Preferred substitution" and further described below in reference to amino acid side chain classes (1) to (6). Amino acid substitutions may be introduced into the molecule of interest and the products screened for desired activity, for example, retained/improved antigen binding, decreased immunogenicity, or improved ADCC or CDC.

**[TABLE 1]**

| Original residue | Exemplary substitution | Preferred substitution |
|---|---|---|
| Ala(A) | Val; Leu; Ile | Val |
| Arg(R) | Lys; Gin; Asn | Lys |
| Asn(N) | Gin; His; Asp; Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gin; Lys; Arg | Arg |
| Ile(I) | Leu; Val; Met; Ala; Phe; Norleucine | Leu |
| Leu(L) | Norleucine; Ile; Val; Met; Ala; Phe | Ile |
| Lys(K) | Arg; Gin; Asn | Arg |
| Met(M) | Leu; Phe; Ile | Ile |
| Phe(F) | Trp; Leu; Val; Ile; Ala; Tyr | Tyr |
| Pro(P) | Ala | Ala |
| Ser(S) | Thr | Thr |
| Thr(T) | Val; Ser | Ser |
| Trp(W) | Tyr; Phe | Tyr |
| Tyr(Y) | Trp; Phe; Thr; Ser | Phe |
| Val(V) | Ile; Leu; Met; Phe; Ala; Norleucine | Leu |

Amino acids may be grouped according to common side-chain properties:
(1) hydrophobic: norleucine, Met, Ala, Val, Leu, Ile;
(2) neutral hydrophilic: Cys, Ser, Thr, Asn, Gin;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

As used herein, the term "amino acid sequence variant" includes substantial variants wherein there are amino acid substitution(s) in one or more hypervariable region residues of a parent antigen binding molecule (for example, a humanized or human antibody). In general, the resultant variant(s) selected for further study will have modifications, for example, improvements (for example, increased affinity or reduced immunogenicity) in certain biological properties relative to the parent antigen binding molecule and/or will have substantially retained certain biological properties of the parent antigen binding molecule. An exemplary substitutional variant is an affinity matured antibody, which may be conveniently generated, for example, using phage display-based affinity maturation techniques known in the art. Briefly, one or more HVR residues are mutated and the variant antigen binding molecules are displayed on phage and screened for a particular biological activity (for example, binding affinity). In a specific embodiment, substitution(s), insertion(s), or deletion(s) may occur within one or more HVRs so long as such alterations do not substantially reduce the ability of the antigen binding molecule to bind antigen. For example, conservative alterations (for example, conservative substitutions as provided herein) that do not substantially reduce binding affinity may be made in HVRs.

In addition, there are provided variants of the antibody or an antigen-binding fragment thereof of the present disclosure, which have improved affinity for the CD300c antigen or a receptor thereof. Such variants may be obtained by a number of affinity maturation protocols including CDR mutation (Yang et al., J. Mol. Biol., 254, 392-403, 1995), chain shuffling (Marks et al., Bio/Technology, 10, 779-783, 1992), use of mutator strains of *E. coli* (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996), and sexual PCR (Crameri et al., Nature, 391, 288-291, 1998). These methods of affinity maturation are discussed in Vaughan et al. (Science, 239, 1534-1536, 1988).

The anti-CD300c monoclonal antibody or an antigen-binding fragment thereof may have inter-species cross-reactivity. Specifically, the anti-CD300c monoclonal antibody or an antigen-binding fragment thereof may exhibit cross-reactivity between human and mouse CD300c antigens. Such cross-reactivity is identified in Experimental Examples 6.3 and 6.4.

In a specific embodiment, the signal peptide may be or include a CD8α signal peptide.

In a specific embodiment, the GS linker may be a 5 to 15-peptide consisting of glycine and serine. Specifically, the GS linker may include or consist of the amino acid sequence represented by SEQ ID NO: 422.

In a specific embodiment, the transmembrane domain may be or include a CD8 hinge (hinge of cluster of differentiation 8) and/or a CD28 transmembrane domain. The CD8 hinge may include or consist of the amino acid sequence represented by SEQ ID NO: 424. The CD28 transmembrane domain may include or consist of the amino acid sequence represented by SEQ ID NO: 426.

In a specific embodiment, the intracellular signaling domain may be or include a CD28 intracellular domain and/or a CD3ζ intracellular domain. The CD28 intracellular domain may include or consist of the amino acid sequence represented by SEQ ID NO: 428. The CD3ζ intracellular domain may include or consist of the amino acid sequence represented by SEQ ID NO: 430.

### Polynucleotide, Vector, and Immune Cell

According to another aspect of the present disclosure, there are provided a polynucleotide including a nucleic acid sequence encoding the chimeric antigen receptor, a vector (for example, expression vector) including the polynucleotide, and an immune cell expressing the chimeric antigen receptor.

The polynucleotide of the present disclosure may include any nucleic acid sequence encoding an amino acid sequence that constitutes or is included in the chimeric antigen receptor, and may also include a nucleic acid sequence having 80% or more, preferably 90% or more, more preferably 95% or more, and most preferably 98% or more identity thereto.

The "vector" refers to a nucleic acid molecule capable of transporting another nucleic acid linked thereto. One type of vector is a "plasmid," which refers to a circular double stranded DNA loop into which an additional DNA segment can be inserted. Another type of vector is a viral vector wherein virally-derived DNA or RNA sequences are present in the vector for packaging into a virus. Specific vectors are capable of autonomous replication in a host cell into which they are introduced (for example, bacterial vectors having a bacterial origin of replication and episomal mammalian vectors). Other vectors (for example, non-episomal mammalian vectors) are integrated into the genome of a host cell upon the introduction into the host cell, and thereby are replicated along with the host genome. Moreover, specific vectors are capable of inducing the expression of genes to which they are operatively-linked. Such vectors are referred to herein as "expression vectors". Common expression vectors of utility in recombinant DNA techniques are often in the form of a plasmid. As used herein, the terms "plasmid" and "vector" may be used interchangeably as the plasmid is the most commonly used form of vector. However, the present disclosure includes such other forms of expression vectors, such as viral vectors (for example, lentiviruses, replication defective retroviruses, adenoviruses, and adeno-associated viruses), which serve equivalent functions.

The immune cells expressing the chimeric antigen receptor of the present disclosure may be produced by transforming immune cells with the vector. For example, the immune cells may be produced by introducing into immune cells a lentiviral vector including a nucleic acid sequence that encodes a desired chimeric antigen receptor.

In an embodiment, the immune cell(s) may be any one or more selected from the group consisting of monocyte(s), macrophage(s), T cell(s), natural killer cell(s) (NK cell(s)), and dendritic cell(s). In addition, any immune cells may be included therein as long as they can be used for the prevention or treatment of cancer. Preferably, the immune cells of the present disclosure may be T cells. For purposes of the present disclosure, the T cell may be any T cell, such as a cultured T cell, for example, a primary T cell, or a T cell from a cultured T cell line, for example, Jurkat, SupT1 or the like, or a T cell obtained from a mammal. The T cell, when obtained from a mammal, can be obtained from a number of sources including, but not limited to, bone marrow, blood, lymph nodes, thymus, or other tissues or body fluids. The T cell may also be enriched or purified. The T cell may be a human T cell. The T cell may be a T cell isolated from a human. The T cell may be any type of T cell and may be of any developmental stage, including but not limited to, CD4+/CD8+ double positive T cells, CD8+ T cells (for example, cytotoxic T cells), CD4+ helper T cells, for example, Th1 and Th2 cells, peripheral blood mononuclear cells (PBMCs), peripheral blood leukocytes (PBLs), tumor infiltrating lymphocytes (TILs), memory T cells, naive T cells, and the like. The T cell may be a CD8+ T cell or a CD4+ T cell.

### Method for Prevention or Treatment of Cancer

According to still another aspect of the present disclosure, there is provided a method for preventing or treating cancer, alleviating or decreasing the severity of at least one symptom or sign of cancer, inhibiting metastasis, or inhibiting growth of cancer, in a subject, by using the immune cells of the present disclosure. As used herein, "preventing or treating cancer" may include inhibiting proliferation, survival, metastasis, recurrence, or anticancer agent resistance of cancer. Such a method may include administering the immune cells of the present disclosure to a subject in need of prevention or treatment of cancer. Accordingly, there is provided use of a composition including the immune cell(s) as an active ingredient, for preventing or treating cancer.

As used herein, the term "cancer" refers to a physiological condition that is typically characterized by unregulated cell growth in mammals. The cancer to be prevented or treated in the present disclosure may include, depending on the site of occurrence, colorectal cancer, small intestine cancer, rectal cancer, colon cancer, thyroid cancer, endocrine adenocarcinoma, oral cancer, tongue cancer, pharyngeal cancer, laryngeal cancer, esophageal cancer, cervical cancer, uterine cancer, fallopian tube cancer, ovarian cancer, brain cancer, head and neck cancer, lung cancer, lymph gland cancer, gallbladder cancer, bladder cancer, kidney cancer, liver cancer, pancreatic cancer, prostate cancer, skin cancer (or melanoma), breast cancer, stomach cancer, bone cancer, blood cancer, and the like. However, any cancer can be included therein as long as it expresses a CD300c protein on the surface of cancer cells. In an embodiment, the cancer may include at least any one selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer. In another embodiment, the cancer may be a solid cancer.

In an embodiment, the method may further include administering one or more anticancer agents (for example, immunotherapeutic agents). In a case where (i) the immune cells of the present disclosure are used in combination with (ii) one or more immunotherapeutic agents, (i) and (ii) may be administered simultaneously or sequentially.

The wording "administered sequentially" means that one ingredient is first administered and another ingredient is administered immediately or at a predetermined interval after the first administration, wherein the ingredients may be administered in any order. That is, one or more immunotherapeutic agents may be administered immediately or at a predetermined interval after the immune cells are administered, or vice versa. In addition, any of the one or more immunotherapeutic agents may be first administered first, followed by the immune cells, and then another of the one or more immunotherapeutic agents.

Immunotherapeutic agents have a novel mechanism by which immune cells in the body are activated to kill cancer cells, and thus are advantageous in that they can be widely used for most cancers without specific genetic mutations. In addition, the immunotherapeutic agents have fewer adverse effects in that they treat cancer by strengthening the patient's own immune system, and have effects of improving the patient's quality of life and significantly extending the survival. These immunotherapeutic agents include immune checkpoint inhibitors, and may be manufactured by known methods or commercially available products. Examples of the immunotherapeutic agents include, but are not limited to, anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies. In addition, examples of the immunotherapeutic agents include, but are not limited to, durvalumab (Imfinzi^{®}), atezolizumab (Tecentriq), avelumab (Bavencio^{®}), pembrolizumab (Keytruda^{®}), nivolumab (Opdivo^{®}), αCD47, cemiplimab (Libtayo^{®}), magrolimab (Hu5F9-G4), and ipilimumab (Yervoy^{®}).

In an embodiment, the immunotherapeutic agent may include at least any one selected from the group consisting of anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies. In one example, the immunotherapeutic agent may include at least any one selected from the group consisting of anti-PD-1, anti-PD-L1, anti-CTLA-4, and anti-CD47 antibodies.

In another embodiment, the immunotherapeutic agent may include at least any one selected from the group consisting of durvalumab (Imfinzi), atezolizumab (Tecentriq), pembrolizumab (Keytruda), nivolumab (Opdivo), αCD47, and ipilimumab (Yervoy).

The immune cells according to the present disclosure and optionally one or more additional anticancer agents in each case may be administered in several ways depending on whether local or systemic treatment is desired and the area to be treated. Methods of administering these ingredients to a subject may vary depending on the purpose of administration, the site of the disease, the subject's condition, and the like. The route of administration may be oral, parenteral, inhalation, local or topical (for example, intralesional administration). Examples of parenteral administration may include, but are not limited to, intravenous, subcutaneous, intraperitoneal, intrapulmonary, intraarterial, intramuscular, rectal, vaginal, intraarticular, intraprostatic, intranasal, intraocular, intravesical, intrathecal, or intraventricular administration (for example, intracerebroventricular administration). In addition, when used in combination, the immune cells and the additional anticancer agent may be administered by the same route or may be administered by different routes.

In the method, the number of the immune cells according to the present disclosure may vary depending on the age, sex, and body weight of an individual (patient). The immune cells may be included at about 1 to about 10 times the number of tumor cells in the individual. In addition, the effective amount of at least one additional anticancer agent may vary depending on the age, sex, and body weight of an individual (patient). In general, administration may be performed in an amount of about 0.01 mg to 100 mg, or 5 mg to about 50 mg, per kg of body. The amount may be administrated once a day or several times a day in divided doses. However, the effective amount may be increased or decreased depending on route and period of administration, severity of disease, sex, body weight, age, and the like. Thus, the range of the present disclosure is not limited thereto.

### Pharmaceutical Composition

According to still another aspect of the present disclosure, there is provided a pharmaceutical composition for preventing or treating cancer, including the immune cells according to the present disclosure as an active ingredient. In addition, there is provided use of the immune cell(s) according to the present disclosure for the manufacture of a medicament for preventing or treating cancer.

The immune cells may be included in the composition in a prophylactically or therapeutically effective amount. The pharmaceutical composition may be administered to a subject to inhibit proliferation, survival, metastasis, recurrence, or anticancer agent resistance of cancer.

In an embodiment, the pharmaceutical composition may further include at least one additional anticancer agent (for example, immunotherapeutic agent). Specifically, the immune cells and optionally the additional immunotherapeutic agent may be included in the same composition or may be included in separate compositions. When included in separate compositions, the immune cells and the additional immunotherapeutic agent may be formulated separately, and may be administered simultaneously or sequentially.

To prepare the pharmaceutical composition of the present disclosure, the immune cells and optionally the additional immunotherapeutic agent may be mixed with a pharmaceutically acceptable carrier and/or excipient. The pharmaceutical composition may be prepared in the form of a lyophilized preparation or an aqueous solution. For example, see Remington's Pharmaceutical Sciences and U.S. Pharmacopeia: National Formulary, Mack Publishing Company, Easton, PA (1984).

Acceptable carriers and/or excipients (including stabilizers) are nontoxic to recipients at the dosages and concentrations employed, and include, but are not limited to, buffers (for example, phosphate, citrate, or other organic acids); antioxidants (for example, ascorbic acid or methionine); preservatives (for example, octadecyldimethylbenzyl ammonium chloride; hexamethonium chloride; benzalkonium chloride or benzethonium chloride; phenol, butyl, or benzyl alcohol; alkyl parabens, such as methyl or propyl paraben; catechol; resorcinol; cyclohexanol; 3-pentanol; and m-cresol); low-molecular weight (less than about 10 residues) polypeptides; proteins (for example, serum albumin, gelatin, or immunoglobulin); hydrophilic polymers (for example, polyvinylpyrrolidone); amino acids (for example, glycine, glutamine, asparagine, histidine, arginine, or lysine); monosaccharides, disaccharides, and other carbohydrates, such as glucose, mannose, or dextrins; chelating agents (for example, EDTA); sugars (for example, sucrose, mannitol, trehalose or sorbitol); salt-forming counter-ions (for example, sodium); metal complexes (for example, Zn-protein complexes); and (or) non-ionic surfactants (for example, TWEEN^{™}, PLURONICS^{™} or polyethylene glycol (PEG)).

The pharmaceutical composition of the present disclosure may be formulated in a suitable form known in the art depending on the route of administration.

As used herein, the term "prophylactically or therapeutically effective amount" or "effective amount" refers to an amount of an active ingredient in a composition, which is effective for preventing or treating cancer in a subject. Also, this amount is sufficient for preventing or treating cancer at a reasonable benefit/risk ratio applicable to medical treatment and does not cause adverse effects. The level of the effective amount may be determined depending on the patient's health status, type of disease, severity of disease, activity of the drug, sensitivity to the drug, method of administration, frequency of administration, route of administration and rate of excretion, duration of treatment, drugs used in combination or coincidentally therewith, and other factors well known in the medical field. It is important to administer a minimum amount that allows the maximum effect to be achieved with minimal or no adverse effects in consideration of all of the above factors, which can be easily determined by those skilled in the art.

For the effective amount of each active ingredient in the pharmaceutical composition of the present disclosure, refer to the description in the section on the method for preventing or treating cancer.

In another embodiment, the pharmaceutical composition can inhibit proliferation, survival, metastasis, recurrence, or anticancer agent resistance of cancer.

### [Mode for Carrying Out the Invention]

Hereinafter, the present disclosure will be described in more detail by way of examples. However, the following examples are only for illustrating the present disclosure, and the scope of the present disclosure is not limited thereto.

### EXAMPLES

### I. Production of Anti-CD300c Monoclonal Antibodies

### Example 1. Production of Anti-CD300c Monoclonal Antibodies

### Example 1.1. Construction of Anti-CD300c Monoclonal Antibody Library

In order to select anti-CD300c monoclonal antibodies, biopanning was performed using a lambda phage library, a kappa phage library, a VH3VL1 phage library, and an OPALTL phage library. Specifically, a CD300c antigen was added at a concentration of 5 µg/mL to an immunotube and reacted for 1 hour to allow the antigen to be adsorbed on the surface of the immunotube. Thereafter, 3% skim milk was added to inhibit non-specific reactions, and then, 10¹² PFU of each antibody phage library dispersed in 3% skim milk was added to each immunotube for antigen binding. After washing was performed three times using Tris buffered saline-Tween 20 (TBST) solution to remove non-specifically bound phages, single-chain variable fragment (scFv) phage antibodies, which are specifically bound to the CD300c antigen, were eluted using 100 mM triethylamine solution. The eluted phages were neutralized using 1.0 M Tris-HCl buffer (pH 7.8), and then infected at 37°C for 1 hour by treatment to *E. coli* ER2537. The infected *E. coli* was applied onto LB agar medium containing carbenicillin, and then cultured at 37°C for 16 hours. Then, the formed *E. coli* colonies were suspended using 3 mL of super broth (SB)-carbenicillin culture medium. Some of the suspension was stored at -80°C until use with the addition of 15% glycerol, and the remaining portion was re-inoculated into SB-carbenicillin-2% glucose solution and cultured at 37°C. The obtained culture was centrifuged, and biopanning was repeated three times again using the supernatant containing phage particles to obtain and concentrate antigen-specific antibodies.

After repeating the biopanning three times, *E. coli* containing the antibody gene was applied onto LB agar medium containing carbenicillin and cultured at 37°C for 16 hours. The formed *E. coli* colonies were inoculated again into SB-carbenicillin-2% glucose solution and cultured at 37°C until the absorbance (at OD₆₀₀ₙₘ) reached 0.5. Then, IPTG was added and further cultured at 30°C for 16 hours. Thereafter, periplasmic extraction was performed. From the results, a library pool of antibodies, which specifically bind to the CD300c antigen, was primarily obtained.

### Example 1.2. Selection of Anti-CD300c Monoclonal Antibodies

In order to select anti-CD300c monoclonal antibodies specifically binding, with high binding affinity, to the CD300c antigen, ELISA was performed using the library pool obtained in the same manner as in Example 1.1. More specifically, CD300c and CD300a antigens in a coating buffer (0.1 M sodium carbonate, pH 9.0) were separately dispensed into an ELISA plate at a concentration of 5 µg/mL per well and then incubated at room temperature for 3 hours to allow the antigen to be bound to the plate. Thereafter, the plate was washed three times with phosphate buffered saline-Tween 20 (PBST) to remove unbound antigen, and then 350 µL of PBST supplemented with 2% bovine serum albumin (BSA) was added to each well, followed by incubation at room temperature for 1 hour, and the plate was again washed with PBST. Then, 25 µg of a periplasmic extract containing scFv obtained in the same manner as in Example 1.1 was added thereto and incubated at room temperature for 1 hour for antigen binding. After 1 hour, washing was performed three times using PBST to remove unbound scFv, and then 4 µg/mL of an antibody for detection was added, followed by again incubation at room temperature for 1 hour. Subsequently, the unbound antibody for detection was removed using PBST. Then, anti-rabbit IgG to which HRP was bound was added, followed by incubation at room temperature for 1 hour, and the unbound antibody was removed again using PBST. Subsequently, TMB solution was added, followed by incubation for 10 minutes for development. Then, 2 N sulfuric acid solution was added to terminate the development reaction, and the absorbance was measured at 450 nm to identify the antibodies specifically binding to the CD300c antigen.

### Example 1.3. Identification of Anti-CD300c Monoclonal Antibody Sequences

The nucleotide sequences of the anti-CD300c monoclonal antibodies, which were selected using the same method as in Example 1.2, were identified. More specifically, for each of the selected antibody clones, plasmid DNA was extracted therefrom using a plasmid miniprep kit, and then DNA sequencing was performed to sequence complementarity-determining regions (CDRs). As a result, 25 types of anti-CD300c monoclonal antibodies having different amino acid sequences were obtained. The heavy chain and light chain variable regions of these 25 types of anti-CD300c monoclonal antibodies are shown in Tables 2 and 3.

**[TABLE 2]**

| Antibody name | Origin (phage library) | Heavy chain variable region (nucleic acid) | Light chain variable region (nucleic acid) | Heavy chain variable region (amino acid) | Light chain variable region (amino acid) |
|---|---|---|---|---|---|
| CK1 | Kappa | FIG. 1aa (SEQ ID NO: 301) | FIG. 1ab (SEQ ID NO: 302) | FIG. 1ac (SEQ ID NO: 303) | FIG. 1ad (SEQ ID NO: 304) |
| CK2 | Kappa | FIG. 1ba (SEQ ID NO: 305) | FIG. 1bb (SEQ ID NO: 306) | FIG. 1bc (SEQ ID NO: 307) | FIG. 1bd (SEQ ID NO: 308) |
| CK3 | Kappa | FIG. 1ca (SEQ ID NO: 309) | FIG. 1cb (SEQ ID NO: 310) | FIG. 1cc (SEQ ID NO: 311) | FIG. 1cd (SEQ ID NO: 312) |
| CL4 | Lambda | FIG. 1da (SEQ ID NO: 313) | FIG. 1db (SEQ ID NO: 314) | FIG. 1dc (SEQ ID NO: 315) | FIG. 1dd (SEQ ID NO: 316) |
| CL5 | Lambda | FIG. 1ea (SEQ ID NO: 317) | FIG. 1eb (SEQ ID NO: 318) | FIG. 1ec (SEQ ID NO: 319) | FIG. 1ed (SEQ ID NO: 320) |
| CL6 | VH3VL1 | FIG. 1fa (SEQ ID NO: 321) | FIG. 1fb (SEQ ID NO: 322) | FIG. 1fc (SEQ ID NO: 323) | FIG. 1fd (SEQ ID NO: 324) |
| CL7 | VH3VL1 | FIG. 1ga (SEQ ID NO: 325) | FIG. 1gb (SEQ ID NO: 326) | FIG. 1gc (SEQ ID NO: 327) | FIG. 1gd (SEQ ID NO: 328) |
| CL8 | VH3VL1 | FIG. 1ha (SEQ ID NO: 329) | FIG. 1hb (SEQ ID NO: 330) | FIG. 1hc (SEQ ID NO: 331) | FIG. 1hd (SEQ ID NO: 332) |
| CL9 | VH3VL1 | FIG. 1ia (SEQ ID NO: 333) | FIG. 1ib (SEQ ID NO: 334) | FIG. 1ic (SEQ ID NO: 335) | FIG. 1id (SEQ ID NO: 336) |
| CL10 | VH3VL1 | FIG. 1ja (SEQ ID NO: 337) | FIG. 1jb (SEQ ID NO: 338) | FIG. 1jc (SEQ ID NO: 339) | FIG. 1jd (SEQ ID NO: 340) |
| SK11 | Kappa | FIG. 1ka (SEQ ID NO: 341) | FIG. 1kb (SEQ ID NO: 342) | FIG. 1kc (SEQ ID NO: 343) | FIG. 1kd (SEQ ID NO: 344) |
| SK12 | Kappa | FIG. 11a (SEQ ID NO: 345) | FIG. 11b (SEQ ID NO: 346) | FIG. 11c (SEQ ID NO: 347) | FIG. 11d (SEQ ID NO: 348) |
| SK13 | Kappa | FIG. 1ma (SEQ ID NO: 349) | FIG. 1mb (SEQ ID NO: 350) | FIG. 1mc (SEQ ID NO: 351) | FIG. 1md (SEQ ID NO: 352) |
| SK14 | Kappa | FIG. 1na (SEQ ID NO: 353) | FIG. 1nb (SEQ ID NO: 354) | FIG. 1nc (SEQ ID NO: 355) | FIG. 1nd (SEQ ID NO: 356) |
| SK15 | Kappa | FIG. 1oa (SEQ ID NO: 357) | FIG. 1ob (SEQ ID NO: 358) | FIG. 1oc (SEQ ID NO: 359) | FIG. 1od (SEQ ID NO: 360) |
| SK16 | Kappa | FIG. 1pa (SEQ ID NO: 361) | FIG. 1pb (SEQ ID NO: 362) | FIG. 1pc (SEQ ID NO: 363) | FIG. 1pd (SEQ ID NO: 364) |
| SK17 | Kappa | FIG. 1qa (SEQ ID NO: 365) | FIG. 1qb (SEQ ID NO: 366) | FIG. 1qc (SEQ ID NO: 367) | FIG. 1qd (SEQ ID NO: 368) |

**[TABLE 3]**

| Antibody name | Origin (phage library) | Heavy chain variable region (nucleic acid) | Light chain variable region (nucleic acid) | Heavy chain variable region (amino acid) | Light chain variable region (amino acid) |
|---|---|---|---|---|---|
| SL18 | Lambda | FIG. 1ra (SEQ ID NO: 369) | FIG. 1rb (SEQ ID NO: 370) | FIG. 1rc (SEQ ID NO: 371) | FIG. 1rd (SEQ ID NO: 372) |
| CB301_H3L1_A10 | VH3VL1 | FIG. 1sa (SEQ ID NO: 373) | FIG. 1sb (SEQ ID NO: 374) | FIG. 1sc (SEQ ID NO: 375) | FIG. 1sd (SEQ ID NO: 376) |
| CB301_H3L1_A12 | VH3VL1 | FIG. 1ta (SEQ ID NO: 377) | FIG. 1tb (SEQ ID NO: 378) | FIG. 1tc (SEQ ID NO: 379) | FIG. 1td (SEQ ID NO: 380) |
| CB301_H3L1_E6 | VH3VL1 | FIG. 1ua (SEQ ID NO: 381) | FIG. 1ub (SEQ ID NO: 382) | FIG. 1uc (SEQ ID NO: 383) | FIG. 1ud (SEQ ID NO: 384) |
| CB301_H3L1_F4 | VH3VL1 | FIG. 1va (SEQ ID NO: 385) | FIG. 1vb (SEQ ID NO: 386) | FIG. 1vc (SEQ ID NO: 387) | FIG. 1vd (SEQ ID NO: 388) |
| CB301_H3L1_G11 | VH3VL1 | FIG. 1wa (SEQ ID NO: 389) | FIG. 1wb (SEQ ID NO: 390) | FIG. 1wc (SEQ ID NO: 391) | FIG. 1wd (SEQ ID NO: 392) |
| CB301_OPALTL_B5 | OPALTL | FIG. 1xa (SEQ ID NO: 393) | FIG. 1xb (SEQ ID NO: 394) | FIG. 1xc (SEQ ID NO: 395) | FIG. 1xd (SEQ ID NO: 396) |
| CB301_OPALTL_E6 | OPALTL | FIG. 1ya (SEQ ID NO: 397) | FIG. 1yb (SEQ ID NO: 398) | FIG. 1yc (SEQ ID NO: 399) | FIG. 1yd (SEQ ID NO: 400) |

In each of the drawings stated in Tables 2 and 3, the CDR regions (CDR1, CDR2, and CDR3) are underlined and appear sequentially (that is, CDR1 appears, followed by CDR2, and then CDR3). In addition, the CDR regions included in each drawing are represented by the sequence numbers as shown in Table 4:

**[TABLE 4]**

| | Antibody | Heavy chain /Light chain | Amino acid /DNA | CDR1 | CDR2 | CDR3 |
|---|---|---|---|---|---|---|
| FIG. 1aa | CK1 | Heavy chain | DNA | SEQ ID NO: 1 | SEQ ID NO: 2 | SEQ ID NO: 3 |
| FIG. 1ab | | Light chain | DNA | SEQ ID NO: 4 | SEQ ID NO: 5 | SEQ ID NO: 6 |
| FIG. 1ac | | Heavy chain | Amino acid | SEQ ID NO: 7 | SEQ ID NO: 8 | SEQ ID NO: 9 |
| FIG. 1ad | | Light chain | Amino acid | SEQ ID NO: 10 | SEQ ID NO: 11 | SEQ ID NO: 12 |
| FIG. 1ba | CK2 | Heavy chain | DNA | SEQ ID NO: 13 | SEQ ID NO: 14 | SEQ ID NO: 15 |
| FIG. 1bb | | Light chain | DNA | SEQ ID NO: 16 | SEQ ID NO: 17 | SEQ ID NO: 18 |
| FIG. 1bc | | Heavy chain | Amino acid | SEQ ID NO: 19 | SEQ ID NO: 20 | SEQ ID NO: 21 |
| FIG. 1bd | | Light chain | Amino acid | SEQ ID NO: 22 | SEQ ID NO: 23 | SEQ ID NO: 24 |
| FIG. 1ca | CK3 | Heavy chain | DNA | SEQ ID NO: 25 | SEQ ID NO: 26 | SEQ ID NO: 27 |
| FIG. 1cb | | Light chain | DNA | SEQ ID NO: 28 | SEQ ID NO: 29 | SEQ ID NO: 30 |
| FIG. 1cc | | Heavy chain | Amino acid | SEQ ID NO: 31 | SEQ ID NO: 32 | SEQ ID NO: 33 |
| FIG. 1cd | | Light chain | Amino acid | SEQ ID NO: 34 | SEQ ID NO: 35 | SEQ ID NO: 36 |
| FIG. 1da | CL4 | Heavy chain | DNA | SEQ ID NO: 37 | SEQ ID NO: 38 | SEQ ID NO: 39 |
| FIG. 1db | | Light chain | DNA | SEQ ID NO: 40 | SEQ ID NO: 41 | SEQ ID NO: 42 |
| FIG. 1dc | | Heavy chain | Amino acid | SEQ ID NO: 43 | SEQ ID NO: 44 | SEQ ID NO: 45 |
| FIG. 1dd | | Light chain | Amino acid | SEQ ID NO: 46 | SEQ ID NO: 47 | SEQ ID NO: 48 |
| FIG. 1ea | CL5 | Heavy chain | DNA | SEQ ID NO: 49 | SEQ ID NO: 50 | SEQ ID NO: 51 |
| FIG. 1eb | | Light chain | DNA | SEQ ID NO: 52 | SEQ ID NO: 53 | SEQ ID NO: 54 |
| FIG. 1ec | | Heavy chain | Amino acid | SEQ ID NO: 55 | SEQ ID NO: 56 | SEQ ID NO: 57 |
| FIG. led | | Light chain | Amino acid | SEQ ID NO: 58 | SEQ ID NO: 59 | SEQ ID NO: 60 |
| FIG. 1fa | CL6 | Heavy chain | DNA | SEQ ID NO: 61 | SEQ ID NO: 62 | SEQ ID NO: 63 |
| FIG. 1fb | | Light chain | DNA | SEQ ID NO: 64 | SEQ ID NO: 65 | SEQ ID NO: 66 |
| FIG. 1fc | | Heavy chain | Amino acid | SEQ ID NO: 67 | SEQ ID NO: 68 | SEQ ID NO: 69 |
| FIG. 1fd | | Light chain | Amino acid | SEQ ID NO: 70 | SEQ ID NO: 71 | SEQ ID NO: 72 |
| FIG. 1ga | CL7 | Heavy chain | DNA | SEQ ID NO: 73 | SEQ ID NO: 74 | SEQ ID NO: 75 |
| FIG. 1gb | | Light chain | DNA | SEQ ID NO: 76 | SEQ ID NO: 77 | SEQ ID NO: 78 |
| FIG. 1gc | | Heavy chain | Amino acid | SEQ ID NO: 79 | SEQ ID NO: 80 | SEQ ID NO: 81 |
| FIG. 1gd | | Light chain | Amino acid | SEQ ID NO: 82 | SEQ ID NO: 83 | SEQ ID NO: 84 |
| FIG. 1ha | CL8 | Heavy chain | DNA | SEQ ID NO: 85 | SEQ ID NO: 86 | SEQ ID NO: 87 |
| FIG. 1hb | | Light chain | DNA | SEQ ID NO: 88 | SEQ ID NO: 89 | SEQ ID NO: 90 |
| FIG. 1hc | | Heavy chain | Amino acid | SEQ ID NO: 91 | SEQ ID NO: 92 | SEQ ID NO: 93 |
| FIG. 1hd | | Light chain | Amino acid | SEQ ID NO: 94 | SEQ ID NO: 95 | SEQ ID NO: 96 |
| FIG. 1ia | CL9 | Heavy chain | DNA | SEQ ID NO: 97 | SEQ ID NO: 98 | SEQ ID NO: 99 |
| FIG. 1ib | | Light chain | DNA | SEQ ID NO: 100 | SEQ ID NO: 101 | SEQ ID NO: 102 |
| FIG. 1ic | | Heavy chain | Amino acid | SEQ ID NO: 103 | SEQ ID NO: 104 | SEQ ID NO: 105 |
| FIG. 1id | | Light chain | Amino acid | SEQ ID NO: 106 | SEQ ID NO: 107 | SEQ ID NO: 108 |
| FIG. 1ja | CL10 | Heavy chain | DNA | SEQ ID NO: 109 | SEQ ID NO: 110 | SEQ ID NO: 111 |
| FIG. 1jb | | Light chain | DNA | SEQ ID NO: 112 | SEQ ID NO: 113 | SEQ ID NO: 114 |
| FIG. 1jc | | Heavy chain | Amino acid | SEQ ID NO: 115 | SEQ ID NO: 116 | SEQ ID NO: 117 |
| FIG. 1jd | | Light chain | Amino acid | SEQ ID NO: 118 | SEQ ID NO: 119 | SEQ ID NO: 120 |
| FIG. 1ka | SK11 | Heavy chain | DNA | SEQ ID NO: 121 | SEQ ID NO: 122 | SEQ ID NO: 123 |
| FIG. 1kb | | Light chain | DNA | SEQ ID NO: 124 | SEQ ID NO: 125 | SEQ ID NO: 126 |
| FIG. 1kc | | Heavy chain | Amino acid | SEQ ID NO: 127 | SEQ ID NO: 128 | SEQ ID NO: 129 |
| FIG. 1kd | | Light chain | Amino acid | SEQ ID NO: 130 | SEQ ID NO: 131 | SEQ ID NO: 132 |
| FIG. 1la | SK12 | Heavy chain | DNA | SEQ ID NO: 133 | SEQ ID NO: 134 | SEQ ID NO: 135 |
| FIG. 1lb | | Light chain | DNA | SEQ ID NO: 136 | SEQ ID NO: 137 | SEQ ID NO: 138 |
| FIG. 1lc | | Heavy chain | Amino acid | SEQ ID NO: 139 | SEQ ID NO: 140 | SEQ ID NO: 141 |
| FIG. 1ld | | Light chain | Amino acid | SEQ ID NO: 142 | SEQ ID NO: 143 | SEQ ID NO: 144 |
| FIG. 1ma | SK13 | Heavy chain | DNA | SEQ ID NO: 145 | SEQ ID NO: 146 | SEQ ID NO: 147 |
| FIG. 1mb | | Light chain | DNA | SEQ ID NO: 148 | SEQ ID NO: 149 | SEQ ID NO: 150 |
| FIG. 1mc | | Heavy chain | Amino acid | SEQ ID NO: 151 | SEQ ID NO: 152 | SEQ ID NO: 153 |
| FIG. 1md | | Light chain | Amino acid | SEQ ID NO: 154 | SEQ ID NO: 155 | SEQ ID NO: 156 |
| FIG. 1na | SK14 | Heavy chain | DNA | SEQ ID NO: 157 | SEQ ID NO: 158 | SEQ ID NO: 159 |
| FIG. 1nb | | Light chain | DNA | SEQ ID NO: 160 | SEQ ID NO: 161 | SEQ ID NO: 162 |
| FIG. 1nc | | Heavy chain | Amino acid | SEQ ID NO: 163 | SEQ ID NO: 164 | SEQ ID NO: 165 |
| FIG. 1nd | | Light chain | Amino acid | SEQ ID NO: 166 | SEQ ID NO: 167 | SEQ ID NO: 168 |
| FIG. 1oa | SK15 | Heavy chain | DNA | SEQ ID NO: 169 | SEQ ID NO: 170 | SEQ ID NO: 171 |
| FIG. 1ob | | Light chain | DNA | SEQ ID NO: 172 | SEQ ID NO: 173 | SEQ ID NO: 174 |
| FIG. 1oc | | Heavy chain | Amino acid | SEQ ID NO: 175 | SEQ ID NO: 176 | SEQ ID NO: 177 |
| FIG. 1od | | Light chain | Amino acid | SEQ ID NO: 178 | SEQ ID NO: 179 | SEQ ID NO: 180 |
| FIG. 1pa | SK16 | Heavy chain | DNA | SEQ ID NO: 181 | SEQ ID NO: 182 | SEQ ID NO: 183 |
| FIG. 1pb | | Light chain | DNA | SEQ ID NO: 184 | SEQ ID NO: 185 | SEQ ID NO: 186 |
| FIG. 1pc | | Heavy chain | Amino acid | SEQ ID NO: 187 | SEQ ID NO: 188 | SEQ ID NO: 189 |
| FIG. 1pd | | Light chain | Amino acid | SEQ ID NO: 190 | SEQ ID NO: 191 | SEQ ID NO: 192 |
| FIG. 1qa | SK17 | Heavy chain | DNA | SEQ ID NO: 193 | SEQ ID NO: 194 | SEQ ID NO: 195 |
| FIG. 1qb | | Light chain | DNA | SEQ ID NO: 196 | SEQ ID NO: 197 | SEQ ID NO: 198 |
| FIG. 1qc | | Heavy chain | Amino acid | SEQ ID NO: 199 | SEQ ID NO: 200 | SEQ ID NO: 201 |
| FIG. 1qd | | Light chain | Amino acid | SEQ ID NO: 202 | SEQ ID NO: 203 | SEQ ID NO: 204 |
| FIG. 1ra | SL18 | Heavy chain | DNA | SEQ ID NO: 205 | SEQ ID NO: 206 | SEQ ID NO: 207 |
| FIG. 1rb | | Light chain | DNA | SEQ ID NO: 208 | SEQ ID NO: 209 | SEQ ID NO: 210 |
| FIG. 1rc | | Heavy chain | Amino acid | SEQ ID NO: 211 | SEQ ID NO: 212 | SEQ ID NO: 213 |
| FIG. 1rd | | Light chain | Amino acid | SEQ ID NO: 214 | SEQ ID NO: 215 | SEQ ID NO: 216 |
| FIG. 1sa | CB301_H 3L1_A10 | Heavy chain | DNA | SEQ ID NO: 217 | SEQ ID NO: 218 | SEQ ID NO: 219 |
| FIG. 1sb | | Light chain | DNA | SEQ ID NO: 220 | SEQ ID NO: 221 | SEQ ID NO: 222 |
| FIG. 1sc | | Heavy chain | Amino acid | SEQ ID NO: 223 | SEQ ID NO: 224 | SEQ ID NO: 225 |
| FIG. 1sd | | Light chain | Amino acid | SEQ ID NO: 226 | SEQ ID NO: 227 | SEQ ID NO: 228 |
| FIG. 1ta | CB301_H 3L1_A12 | Heavy chain | DNA | SEQ ID NO: 229 | SEQ ID NO: 230 | SEQ ID NO: 231 |
| FIG. 1tb | | Light chain | DNA | SEQ ID NO: 232 | SEQ ID NO: 233 | SEQ ID NO: 234 |
| FIG. 1tc | | Heavy chain | Amino acid | SEQ ID NO: 235 | SEQ ID NO: 236 | SEQ ID NO: 237 |
| FIG. 1td | | Light chain | Amino acid | SEQ ID NO: 238 | SEQ ID NO: 239 | SEQ ID NO: 240 |
| FIG. 1ua | CB301_H 3L1_E6 | Heavy chain | DNA | SEQ ID NO: 241 | SEQ ID NO: 242 | SEQ ID NO: 243 |
| FIG. 1ub | | Light chain | DNA | SEQ ID NO: 244 | SEQ ID NO: 245 | SEQ ID NO: 246 |
| FIG. 1uc | | Heavy chain | Amino acid | SEQ ID NO: 247 | SEQ ID NO: 248 | SEQ ID NO: 249 |
| FIG. 1ud | | Light chain | Amino acid | SEQ ID NO: 250 | SEQ ID NO: 251 | SEQ ID NO: 252 |
| FIG. 1va | CB301_H 3L1_F4 | Heavy chain | DNA | SEQ ID NO: 253 | SEQ ID NO: 254 | SEQ ID NO: 255 |
| FIG. 1vb | | Light chain | DNA | SEQ ID NO: 256 | SEQ ID NO: 257 | SEQ ID NO: 258 |
| FIG. 1vc | | Heavy chain | Amino acid | SEQ ID NO: 259 | SEQ ID NO: 260 | SEQ ID NO: 261 |
| FIG. 1vd | | Light chain | Amino acid | SEQ ID NO: 262 | SEQ ID NO: 263 | SEQ ID NO: 264 |
| FIG. 1wa | CB301_H 3L1_G1 1 | Heavy chain | DNA | SEQ ID NO: 265 | SEQ ID NO: 266 | SEQ ID NO: 267 |
| FIG. 1wb | | Light chain | DNA | SEQ ID NO: 268 | SEQ ID NO: 269 | SEQ ID NO: 270 |
| FIG. 1wc | | Heavy chain | Amino acid | SEQ ID NO: 271 | SEQ ID NO: 272 | SEQ ID NO: 273 |
| FIG. 1wd | | Light chain | Amino acid | SEQ ID NO: 274 | SEQ ID NO: 275 | SEQ ID NO: 276 |
| FIG. 1xa | CB301_O PALTL_B 5 | Heavy chain | DNA | SEQ ID NO: 277 | SEQ ID NO: 278 | SEQ ID NO: 279 |
| FIG. 1xb | | Light chain | DNA | SEQ ID NO: 280 | SEQ ID NO: 281 | SEQ ID NO: 282 |
| FIG. 1xc | | Heavy chain | Amino acid | SEQ ID NO: 283 | SEQ ID NO: 284 | SEQ ID NO: 285 |
| FIG. 1xd | | Light chain | Amino acid | SEQ ID NO: 286 | SEQ ID NO: 287 | SEQ ID NO: 288 |
| FIG. 1ya | CB301_O PALTL_E 6 | Heavy chain | DNA | SEQ ID NO: 289 | SEQ ID NO: 290 | SEQ ID NO: 291 |
| FIG. 1yb | | Light chain | DNA | SEQ ID NO: 292 | SEQ ID NO: 293 | SEQ ID NO: 294 |
| FIG. 1yc | | Heavy chain | Amino acid | SEQ ID NO: 295 | SEQ ID NO: 296 | SEQ ID NO: 297 |
| FIG. 1yd | | Light chain | Amino acid | SEQ ID NO: 298 | SEQ ID NO: 299 | SEQ ID NO: 300 |

As described above, 25 types of anti-CD300c monoclonal antibodies were identified that specifically bind, with high binding affinity, to the CD300c antigen and can be used for the prevention or treatment of cancer.

### Example 1.4. Production and Purification of Anti-CD300c Monoclonal Antibodies

By using the nucleotide sequences of the anti-CD300c monoclonal antibodies identified in Example 1.3, expression vectors having, separately, heavy and light chains capable of expressing antibodies were prepared. More specifically, the expression vectors were prepared by inserting genes into pCIW3.3 vectors using the analyzed CDR sequences so that the vectors can express the heavy and light chains, respectively. The prepared expression vectors for heavy and light chains were mixed with polyethylenimine (PEl) at a mass ratio of 1:1. The mixture was transfected into 293T cells to induce antibody expression, and on day 8, the culture was centrifuged to remove the cells and obtain the resultant culture. The obtained culture was filtered, and then resuspended using a mixed solution of 0.1 M NaH₂PO₄ and 0.1 M Na₂HPO₄ (pH 7.0). The resuspended solution was purified through affinity chromatography using protein A beads (GE Healthcare), followed by elution using an elution buffer (Thermofisher).

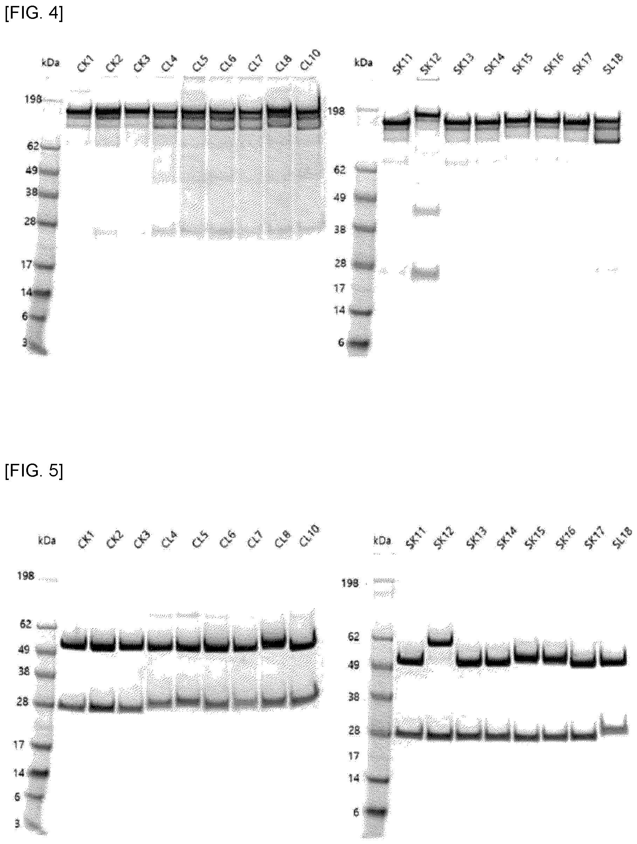

In order to identify the produced antibodies, each of reducing sample buffer and non-reducing sample buffer was added to 5 µg of purified antibody, and electrophoresis was performed using pre-made SDS-PAGE (Invitrogen). Then, the proteins were stained using Coomassie Blue. The results under non-reducing conditions are show in FIG. 4, and the results under reducing conditions are shown in FIG. 5.

As shown in FIGS. 4 and 5, it was identified that the anti-CD300c monoclonal antibodies having a high purity were produced and purified.

### II. Expression of CD300c in Cancer Cells and Binding of Anti-CD300c Monoclonal Antibodies to CD300c Antigen

### Experimental Example 1. Identification of Expression of CD300c in Cancer cell lines

In order to evaluate whether CD300c is expressed in various cancer cells, various cancer cell lines, such as MKN45 (human gastric cancer cell line), IM95 (human gastric cancer cell line), HT-29 (human colorectal cancer cell line), A549 (human lung cancer cell line), HCT116 (human colorectal cancer cell line), MDA-MB-231 (human breast cancer cell line), and HepG2 (human liver cancer cell line) were cultured and evaluated for the expression of CD300c at the mRNA and protein levels. In addition, THP-1 cells (human monocyte cell line), which are immune cells, were also evaluated. Specifically, HEK293T (normal cell line) was used as a control.

Meanwhile, the expression of proteins was identified by Western blot and flow cytometry (FACS) using fluorescently labeled cells. Specifically, each of the cultured cell lines was fixed with 4% formaldehyde, and then blocked using 5% normal bovine serum albumin. Then, staining was performed with 0.5 µg of eFluor660-labeled anti-CD300c antibody (Invitrogen). Subsequently, the fluorescently labeled cells were identified using flow cytometry (FACS).

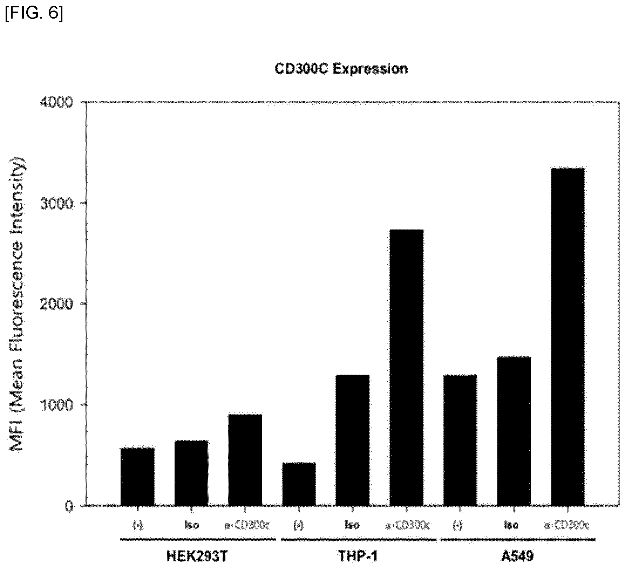

As a result, it was identified that the CD300c antigen was expressed at the mRNA and protein levels in various cancer cells, such as colorectal cancer, lung cancer, and breast cancer. In addition, as shown in FIG. 6, according to the results of analysis using flow cytometry (FACS), it was identified that significantly high expression of CD300c was observed in the human lung cancer cell line (A549) and the human monocyte cell line (THP-1) as compared with the normal cell line (HEK293T).

### Experimental Example 2. Identification of Antigen-Binding Affinity of Anti-CD300c Monoclonal Antibodies

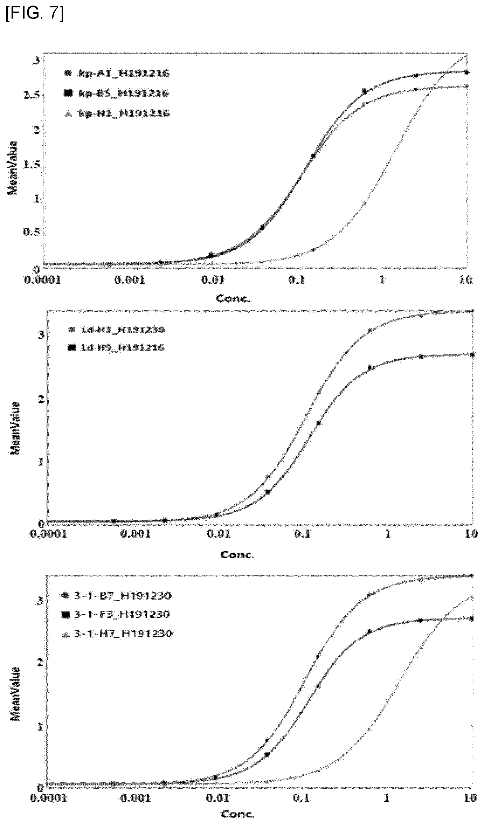

In order to identify the antigen-binding ability of the anti-CD300c monoclonal antibodies produced in Example 1, binding ELISA was performed. Specifically, each of the CD300c antigen (11832-H08H, Sino Biological) or CD300a antigen (12449-H08H, Sino Biological) in a coating buffer solution (0.1 M sodium carbonate, pH 9.0) was dispensed into an ELISA plate at a concentration of 8 µg/mL per well and then incubated at room temperature for 3 hours to allow the antigen to be bound to the plate. Thereafter, the plate was washed three times with PBST to remove unbound antigen, and then 300 µL of PBST supplemented with 5% bovine serum albumin (BSA) was added to each well, followed by incubation at room temperature for 1 hour, and the plate was again washed with PBST. Then, the anti-CD300c monoclonal antibody was diluted four times and added, and incubated at room temperature for 1 hour for antigen binding. After 1 hour, washing was performed three times using PBST to remove unbound anti-CD300c monoclonal antibody, and then 4 µg/mL of an antibody for detection (HRP conjugated anti-Fc IgG) was added, followed by again incubation at room temperature for 1 hour. Subsequently, the unbound antibody for detection was removed using PBST, and then TMB solution was added, followed by incubation for 10 minutes for development. Then, 2 N sulfuric acid solution was added to terminate the development reaction, and the absorbance was measured at 450 nm to identify the antibodies specifically binding to the CD300c antigen. The results are shown in Table 5 below and FIG. 7.

**[TABLE 5]**

| CB301 antibody | EC50 (µg/mL) |
|---|---|
| CK1 | 0.056 |
| CK2 | 0.033 |
| CK3 | 0.793 |
| CL4 | 0.031 |
| CL5 | 0.032 |
| CL6 | 0.148 |
| CL7 | 0.047 |
| CL8 | 49.7 |
| CL9 | 0.094 |
| CL10 | 0.039 |
| SK11 | 0.052 |
| SK12 | 0.067 |
| SK13 | 0.044 |
| SK14 | 0.065 |
| SK15 | 14.74 |
| SK16 | 2.42 |
| SK17 | 0.054 |
| SL18 | 0.17 |

As shown in Table 5, as a result of measuring the EC50 (effective concentration of drug that causes 50% of the maximum response) values of the anti-CD300c monoclonal antibodies, it was identified that the remaining all 14 clones except for 4 clones (CK3, CL8, SK15, and SK16) exhibited high binding affinity of 0.2 µg/mL or lower. Additionally, as shown in FIG. 7, it was identified that the anti-CD300c monoclonal antibodies of the present disclosure bound to the CD300c antigen with high binding affinity even in the sigmoid curves for the results of the binding ELISA.

### Experimental Example 3. Identification of Binding Specificity of Anti-CD300c Monoclonal Antibody to CD300c Antigen

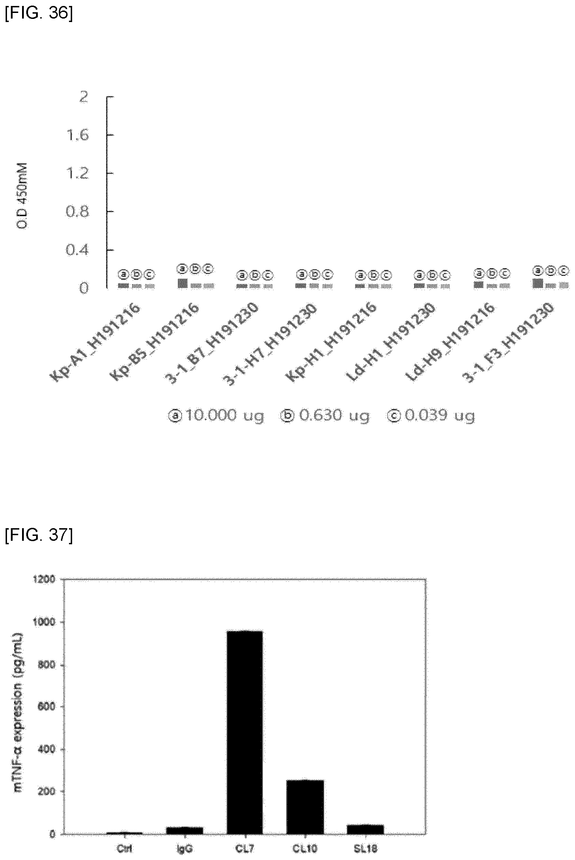

In order to identify the specificity of the anti-CD300c monoclonal antibody CL7 for the CD300c antigen, it was further examined whether CL7 exhibited cross-reactivity to the CD300a antigen, which was known to antagonize the CD300c antigen and had a similar protein sequence thereto. More specifically, the CD300a antigen (available from Sino Biological) was used at concentrations of 0.039, 0.63, and 10 µg/mL, and then binding ELISA was performed in the same manner as in Experimental Example 2. The results are shown in FIG. 36.

As shown in FIG. 36, it was found that the anti-CD300c monoclonal antibody did not bind to antigens other than CD300c and showed high binding specificity only to the CD300c antigen.

### III. Preparation of Cells Expressing Chimeric Antigen Receptor(s) Specifically Binding to CD300c Antigen or Receptor Thereof and Identification of Anticancer Effects Thereof

### Example 2. Construction of Expression Vectors for Chimeric Antigen Receptors Specifically Binding to CD300c Antigen or Receptor Thereof

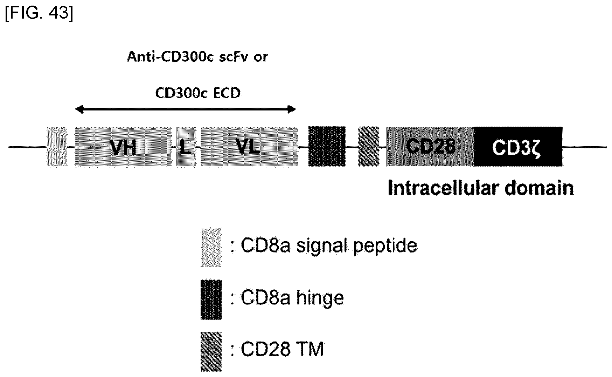

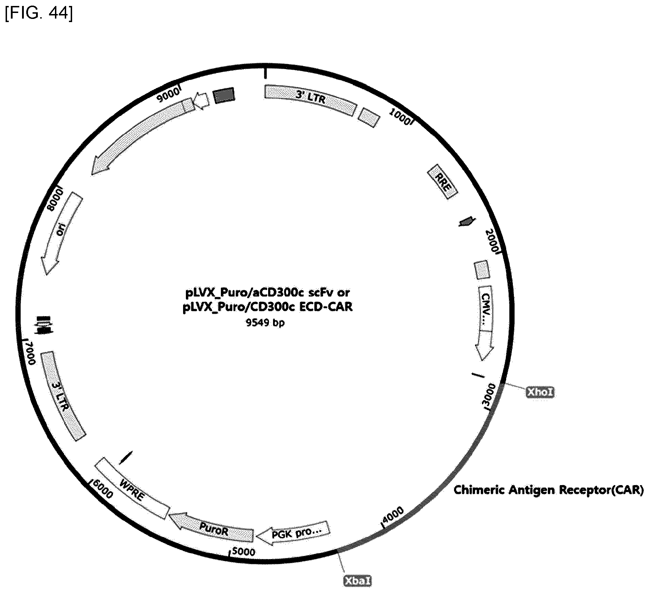

In order to produce chimeric antigen receptors including binding domains specifically binding to a CD300c antigen or a receptor thereof, the following sequences were sequentially inserted into the pLVX-Puro vector (Addgene) to construct an expression vector for a chimeric antigen receptor (pLVX-Puro/aCD300c scFv or pLVX-Puro/CD300c ECD-CAR) specifically binding to a CD300c antigen or a receptor thereof: a CD8α signal peptide (the DNA sequence being represented by SEQ ID NO: 301) including the amino acid sequence represented by SEQ ID NO: 302 which allows a synthesized protein to pass through the cell membrane and move to a correct position; an anti-CD300c single chain variable fragment of each of CK1, CL6, CL7, CL10, and SL18 including the amino acid sequences represented by SEQ ID NOs: 412, 414, 416, 418, and 420, respectively, and specifically binding to a CD300c antigen or a receptor thereof (aCD300c scFvs, the DNA sequences being represented by SEQ ID NO: 411, 413, 415, 417, and 419, respectively) or a CD300c extracellular domain (ECD) antigen including the amino acid sequence represented by SEQ ID NO: 402 (the DNA sequence being represented by SEQ ID NO: 401); a GS linker including the amino acid sequence represented by SEQ ID NO: 422 (the DNA sequence being represented by SEQ ID NO: 421); a CD8 hinge including the amino acid sequence represented by SEQ ID NO: 424 (hinge of cluster of differentiation 8, the DNA sequence being represented by SEQ ID NO: 423) and a CD28 transmembrane domain including the amino acid sequence represented by SEQ ID NO: 426 (the DNA sequence being represented by SEQ ID NO: 425) as transmembranes domains; and a CD28 intracellular domain including the amino acid sequence represented by SEQ ID NO: 428 (the DNA sequence being represented by SEQ ID NO: 427) and a CD3ζ intracellular domain including the amino acid sequence represented by SEQ ID NO: 430 (the DNA sequence being represented by SEQ ID NO: 429) as an intracytoplasmic domain for macrophage activation signaling. The gene and protein sequence combinations for the respective constructed respective vectors are shown in Table 6. A schematic diagram of the gene arrangement is shown in FIG. 43, and an example of the constructed vector map is shown in FIG. 44.

**[TABLE 6]**

| CAR name | DNA sequence combination | Protein sequence combination | SEQ ID NOs |
|---|---|---|---|
| CK1 CAR | Combination of SEQ ID NOs: 409, 411, 421, 423, 425, 427, and 429 | Combination of SEQ ID NOs: 410, 412, 422, 424, 426, 428, and 430 | 431 and 432 |
| CL6 CAR | Combination of SEQ ID NOs: 409, 413, 421, 423, 425, 427, and 429 | Combination of SEQ ID NOs: 410, 414, 422, 424, 426, 428, and 430 | 433 and 434 |
| CL7 CAR | Combination of SEQ ID NOs: 409, 307, 421, 423, 425, 427, and 429 | Combination of SEQ ID NOs: 410, 416, 422, 424, 426, 428, and 430 | 435 and 436 |
| CL10 CAR | Combination of SEQ ID NOs: 409, 309, 421, 423, 425, 427, and 429 | Combination of SEQ ID NOs: 410, 418, 422, 424, 426, 428, and 430 | 437 and 438 |
| SL18 CAR | Combination of SEQ ID NOs: 409, 311, 421, 423, 425, 427, and 429 | Combination of SEQ ID NOs: 410, 420, 422, 424, 426, 428, and 430 | 439 and 440 |
| CD300c ECD CAR | Combination of SEQ ID NOs: 409, 401, 421, 423, 425, 427, and 429 | Combination of SEQ ID NOs: 410, 402, 422, 424, 426, 428, and 430 | 441 and 442 |

### Example 3. Preparation of Recombinant Lentiviruses for Expressing Chimeric Antigen Receptors Specifically Binding to CD300c Antigen or Receptor Thereof

HEK293T cell line (ATCC) required for preparation of recombinant lentiviruses for expressing the chimeric antigen receptors specifically binding to a CD300c antigen or a receptor thereof was prepared as follows. A complete medium used for cell culture was prepared by adding heat-treated fetal bovine serum (FBS) to fresh DMEM (Gibco) to a concentration of 10%, adding 1× Penicillin-Streptomycin (Gibco) thereto, and then performing uniform mixing by inverting up and down. The resultant complete medium was preheated to 37°C before use. The HEK293T cell line was rapidly thawed for 2 to 3 minutes before use by quick transfer of its cryopreserved cell stock to a constant-temperature water bath at 37°C, inoculated into 30 mL of the complete medium, and cultured in a 5% CO₂ incubator at 37°C. When the cell confluency reached 80% or higher, the cells were maintained by subculture. For lentiviral transfection, the HEK293T cell line was inoculated into 10 mL of the complete medium at a concentration of 1 to 2×10⁶ cells, cultured for 16 hours, and then subjected to lentiviral transfection.

LENTI-X^{™} Expression System (Takara) kit was used for lentiviral transfection for expression of the chimeric antigen receptor specifically binding to a CD300c antigen or a receptor thereof. To 7 µg of the expression vector pLVX-Puro/aCD300C scFv or pLVX-Puro/CD300c ECD-CAR constructed in the same manner as in Example 2, sterile water (Invitrogen) was added to make 600 µL. Then, the resultant product was placed in a tube of Lenti-X Packaging Single Shots in LENTI-X^{™} Expression System and mixing was performed to prepare a nanoparticle complex solution. The nanoparticle complex solution obtained by reaction at room temperature for 10 minutes was added dropwise to the previously prepared HEK293T cells, and then mixing was performed by shaking side to side. The mixture was cultured for 4 hours in an incubator, followed by further addition of 6 mL of the fresh complete medium, and cultured for 48 hours. After culture, the supernatant was collected, centrifuged to remove cell debris, filtered through a 0.45 µm filter, and stored at -80°C until use.

After 20 µL of the obtained lentivirus supernatant was added to the GoStix cassette sample well(s) in the LENTI-X^{™} Expression System, three drops of Chase solution were added to the sample wells, followed by incubation at room temperature for 10 minutes. Subsequently, the presence or absence of a band confirmed whether recombinant lentiviruses at an effective dose of 5×10⁵ IFU/mL or higher were obtained.

As a result, it was identified that recombinant lentiviruses expressing the chimeric antigen receptors specifically binding to a CD300c antigen or a receptor thereof were prepared. It was identified that the prepared lentiviruses expressed chimeric antigen receptors, which include the amino acid sequences of single chain variable fragments of CK1, CL6, CL7, CL10, and SL18, respectively.

### Example 4. Production of Jurkat Cells Expressing Chimeric Antigen Receptors Specifically Binding to CD300c Antigen or Receptor Thereof

In order to produce Jurkat cells expressing the chimeric antigen receptors specifically binding to a CD300c antigen or a receptor thereof, the recombinant lentiviruses prepared in the same manner as in Example 2 were transfected into a Jurkat cell line. More specifically, a recombinant lentivirus at 0.1 to 10 MOI was inoculated in the complete medium supplemented with 8 µg/mL polybrene (Merk) and then uniformly mixed by inverting up and down. In addition, 1 mL of the mixture was added to the Jurkat cell line prepared in a 6-well plate for each well, centrifuged at 1,800 rpm for 45 to 90 minutes, and cultured in a 5% CO₂ incubator at 37°C for 24 hours. 24 hours later, subculture was performed at a concentration of 5×10⁵ cells/mL.

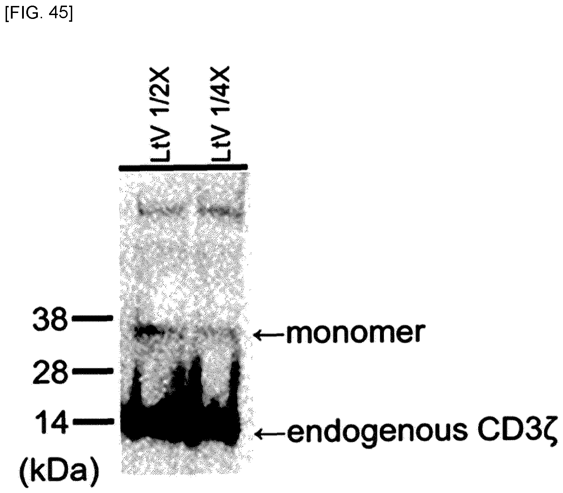

In order to identify whether the chimeric antigen receptor specifically binding to a CD300c antigen or a receptor thereof is normally expressed in the transfected Jurkat cell line, total proteins of the cultured Jurkat cells were obtained using PRO-PREP solution (iNtRON). The concentration of the obtained proteins was measured using a microBCA protein assay kit (Thermo Fisher), and then Western blotting was performed using an equal amount of protein. More specifically, the equal amount of protein was electrophoresed by SDS-PAGE (Invitrogen), and then the electrophoresed protein was transferred to a nitrocellulose membrane (Invitrogen). The protein-bound nitrocellulose membrane was blocked using a 5% skim milk (BD) solution to block non-specific antibody reactions. An anti-CD3 antibody and an anti-GAPDH antibody (Cell Signaling Technologies, USA) as primary antibodies were separately diluted to a concentration of 1:1,000 using 5% skim milk, and used to treat the nitrocellulose membranes. After the incubation, unbound antibodies were removed. A horseradish peroxidase (HRP)-conjugated secondary antibody (Cell Signaling Technologies) as a secondary antibody was diluted to a concentration of 1:2,000 and used to treat the nitrocellulose membrane. The membrane was treated with ECL solution (Thermo Fisher) to induce color development, and then the protein was quantified using the luminescent image analyzer iBright1500 (Invitrogen). The Western blotting results are shown in FIG. 45.

As shown in FIG. 45, the CD3ζ intracellular domain, to which the anti-CD3 antibody was bound, was observed, indicating that a Jurkat cell line expressing the chimeric antigen receptor specifically binding to a CD300c antigen or a receptor thereof was produced.

Additionally, the expression level of the chimeric antigen receptor specifically binding to a CD300c antigen or a receptor thereof was examined using a flow cytometer (FACS). More specifically, the Jurkat cell line was treated with a PE-fusion anti-IgG antibody (Jackson ImmunoResearch), cultured at 4°C for 30 minutes, and then examined using a flow cytometer.

As a result of flow cytometry, it was identified that a Jurkat cell line expressing the chimeric antigen receptor specifically binding to a CD300c antigen or a receptor thereof was produced.

### Experimental Example 4. Anticancer Effects of Jurkat Cells Expressing Chimeric Antigen Receptors Specifically Binding to CD300c Antigen or Receptor Thereof

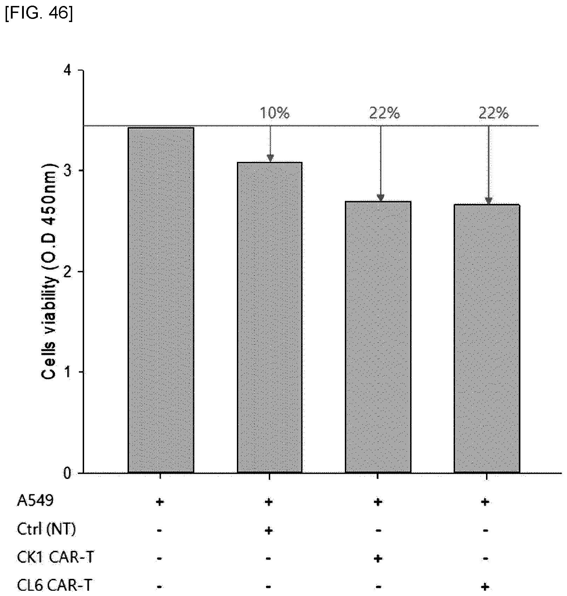

In order to identify anticancer effects of Jurkat cells expressing chimeric antigen receptors specifically binding to a CD300c antigen or a receptor thereof, cancer cell killing effects thereof were examined using A549 cell line. More specifically, the A549 cell line was inoculated into a 96-well plate at a concentration of 1×10⁵ cells/mL. The Jurkat cells expressing chimeric antigen receptors specifically binding to a CD300c antigen or a receptor thereof, as produced in the same manner as in Example 4, were applied to the cancer cells to a concentration of 20:1, and co-cultured. Jurkat cells receiving no lentivirus transfection were used as a control. After co-culture for 24 hours, the co-cultured Jurkat cell line was removed from each well, followed by incubation using CCK-8 (DOJINDO) for 1 hour, and then the absorbance was measured at OD_{450 nm} to examine the degree of cancer cell death. The results are shown FIG. 46.

As shown in FIG. 46, it was identified that the Jurkat cell lines expressing chimeric antigen receptors specifically binding to a CD300c antigen or a receptor thereof exhibited anticancer effects against the A549 cell line, and these anticancer effects were higher than those of the Jurkat cell line receiving no lentivirus transfection.

The above results could identify that the use of immune cell lines expressing chimeric antigen receptors specifically binding to a CD300c antigen or a receptor thereof increased cancer therapeutic effects using the immune cell lines and thus can effectively treat cancer. It was also found that the immune cell lines specifically respond only to cancer cells expressing a CD300c antigen on the surface and thus can maximize therapeutic effects with decreased adverse effects.

### IV. Anticancer Effects of Anti-CD300c Monoclonal Antibodies

### Experimental Example 5. Identification of Anticancer Effects by Administration of Anti-CD300c Monoclonal Antibodies

### Experimental Example 5.1. Identification of T Cell Activation Effect

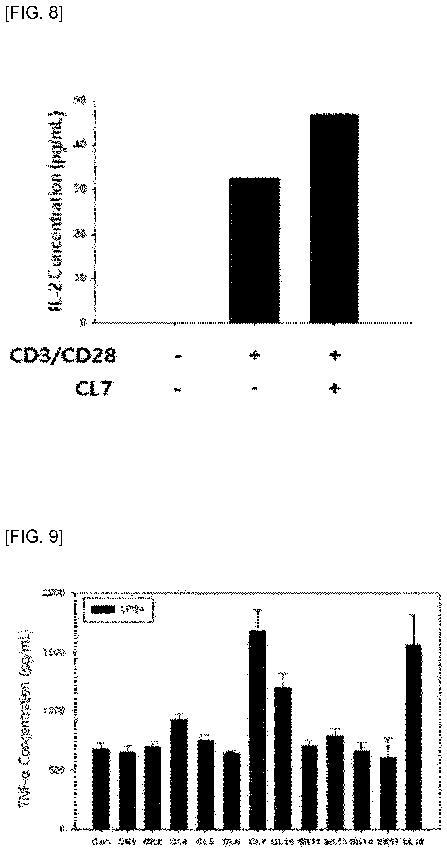

In order to identify whether the anti-CD300c monoclonal antibody produced in Example 1 can exhibit an anticancer effect by activating T cells, the production level of interleukin-2 (IL-2) according to the treatment with the anti-CD300c monoclonal antibody in human T cells was examined. IL-2 is an immune factor that helps growth, proliferation, and differentiation of T cells, and an increased production level of IL-2 means the activation of T cells due to an increase in stimulation that induces increased differentiation, proliferation, and growth of T cells. Specifically, each of an anti-CD3 monoclonal antibody and an anti-CD28 monoclonal antibody was added to a 96-well plate at a concentration of 2 µg/well and fixed for 24 hours. Then, the wells were co-treated with Jurkat T cells (human T lymphocyte cell line) at 1×10⁵ cells/well and the anti-CD300c monoclonal antibody at 10 µg/well. Subsequently, the production level of IL-2 was measured using an ELISA kit (IL-2 Quantikine kit, R&D Systems), and then compared with the control group not treated with the anti-CD300c monoclonal antibody. The results are shown FIG. 8.

As shown in FIG. 8, it was identified that the production level of IL-2 increased when the Jurkat T cells activated by treatment with the anti-CD3 monoclonal antibody and the anti-CD28 monoclonal antibody were treated with the anti-CD300c monoclonal antibody. The above results identified that the anti-CD300c monoclonal antibody can activate T cells and induce the anticancer immune action to inhibit the growth of cancer tissue.

### Experimental Example 5.2. Identification of Promotion of Differentiation into M1 Macrophages (I): Measurement of Production Level of Macrophage Differentiation Marker (TNF-α)

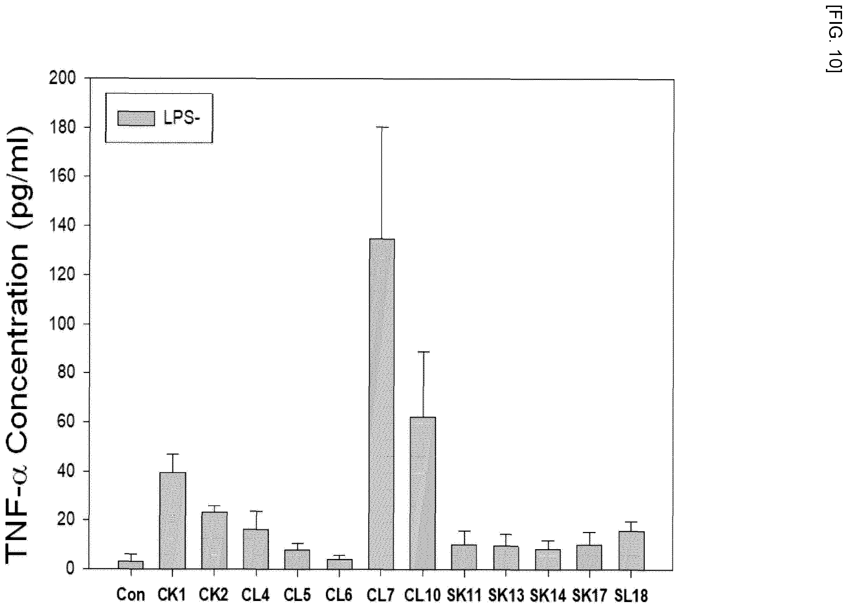

In order to identify whether the anti-CD300c monoclonal antibodies selected in Example 1 can promote the differentiation of monocytes into M1 macrophages, THP-1 (human monocyte cell line) was dispensed into a 96-well plate at 1.5×10⁴ cells/well, and treated with 10 µg/mL of the anti-CD300c monoclonal antibodies and/or 100 ng/mL of LPS. After the incubation for 48 hours, the production level of tumor necrosis factor-α (TNF-α), which is a differentiation marker of M1 macrophages, was measured using an ELISA kit (Human TNF-α Quantikine kit, R&D Systems). The results are shown in FIGS. 9 and 10.

As shown in FIG. 9, it was identified that the anti-CD300c monoclonal antibodies CL4, CL7, CL10, and SL18 exhibited an increase in the production level of TNF-α by about 2 times or higher compared with the control group (Con) treated with LPS alone.

In addition, as shown in FIG. 10, it was identified that all the experimental groups treated with the anti-CD300c monoclonal antibodies alone without LPS treatment exhibited an increase in the production level of TNF-α compared with the control group (Con) treated with LPS alone.

### Experimental Example 5.3. Identification of Antibody Concentration-Dependent Increase in Differentiation Ability into M1 Macrophages

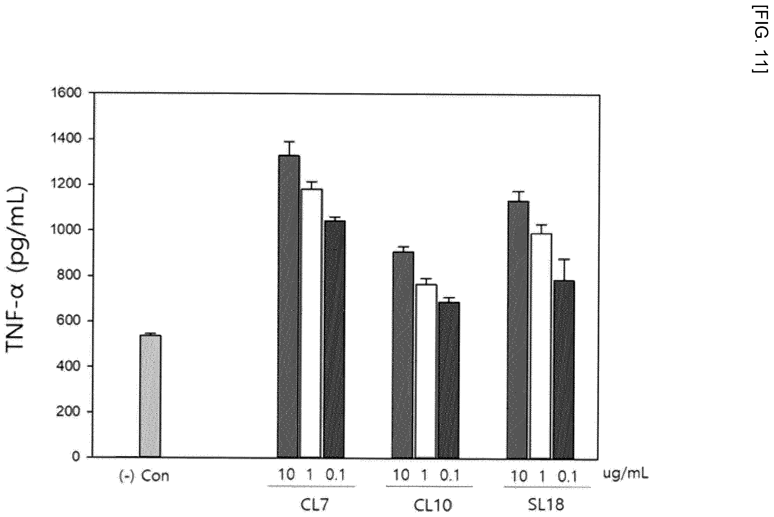

In order to identify that the induction of differentiation into M1 macrophages by the anti-CD300c monoclonal antibodies increases with concentrations of the anti-CD300c monoclonal antibodies, the production level of TNF-α was examined in the same manner as in Experimental Example 5.2. The anti-CD300c monoclonal antibodies were used at concentrations of 10, 1, and 0.1 µg/mL. The results are shown in FIG. 11.

As shown in FIG. 11, it was identified that the production level of TNF-α increased as the treatment concentration of the anti-CD300c monoclonal antibody (CL7, CL10, or SL18) increased.

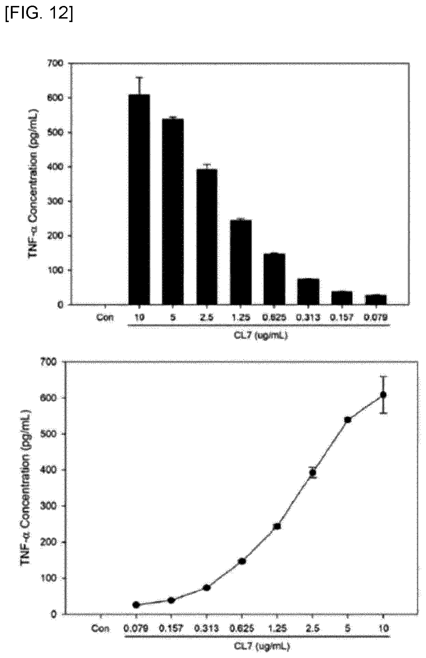

In order to identify under more specific concentrations, the anti-CD300c monoclonal antibody CL7 was used at concentrations of 10, 5, 2.5, 1.25, 0.625, 0.313, 0.157, and 0.079 µg/mL, and the production level of TNF-α was examined. The results are shown in FIG. 12.

As shown in FIG. 12, it was identified that the production level of TNF-α increased as the concentration of the anti-CD300c monoclonal antibody used for treatment increased.

### Experimental Example 5.4. Identification of Promotion of Differentiation Ability into M1 Macrophages (II): Cell Morphology Observation

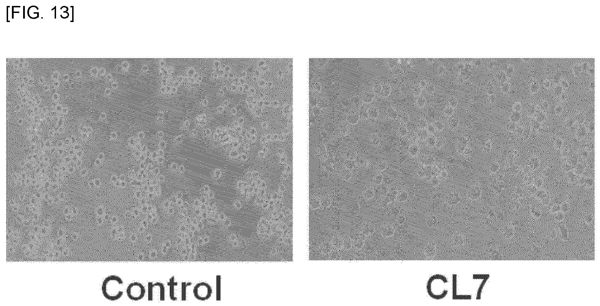

In order to identify the differentiation pattern into M1 macrophages through cell morphology when monocytes were treated with an anti-CD300c monoclonal antibody, THP-1 were treated with the anti-CD300c monoclonal antibody 10 µg/mL, cultured for 48 hours, and then observed for cell morphology under a microscope. The results are shown in FIG. 13.

As shown in FIG. 13, it was identified that as for the experimental group (CL7) treated with the anti-CD300c monoclonal antibody, the morphology of THP-1 cells was changed from suspension cells to circular adherent cells, which are in the form of M1 macrophages. The above results identified that the differentiation of monocytes into M1 macrophages was promoted by treatment with the anti-CD300c monoclonal antibody.

### Experimental Example 5.5. Re-identification of Promotion of Differentiation Ability into M1 Macrophages

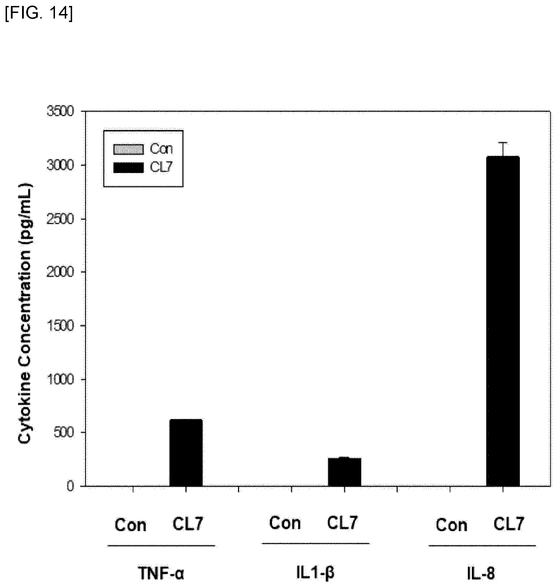

In order to re-identify whether the anti-CD300c monoclonal antibody CL7 promoted the differentiation of human monocytes into M1 macrophages, the secretion levels of TNF-α, interleukin-1β (IL-1β), and interleukin-8 (IL-8) were measured using an ELISA kit. More specifically, THP-1 was dispensed into a 96-well plate at 1.5×10⁴ cells/well, and treated with 10 µg/mL of the anti-CD300c monoclonal antibody. After the incubation for 48 hours, the production levels of TNF-α, IL-1β, and IL-8, which are markers for differentiation into M1 macrophages, were measured using an ELISA kit (Human TNF-α Quantikine kit, R&D Systems). The results are shown in FIG. 14.

As shown in FIG. 14, it was identified that all three types of markers for differentiation into M1 macrophages increased in the experimental group (CL7) treated with the anti-CD300c monoclonal antibody, compared with the control group (Con) not treated with the anti-CD300c monoclonal antibody.

### Experimental Example 5.6. Identification of Repolariation Ability of M2 Macrophages into M1 Macrophages

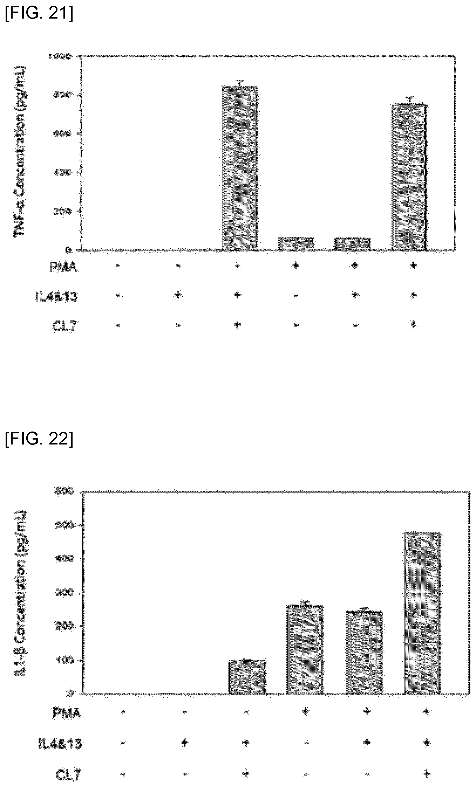

In order to identify whether the anti-CD300c monoclonal antibody can redifferentiate(repolarization) M2 macrophages into M1 macrophages, THP-1 was dispensed into a 96-well plate at 1.5×10⁴ cells/well, pre-treated for 6 hours by treatment with 320 nM of PMA, and then treated with 20 ng/mL of interleukin-4 (IL-4) and interleukin-13 (IL-13), and with 10 µg/mL of the anti-CD300c monoclonal antibody, followed by incubation for 18 hours. The production levels of TNF-α, IL-1β, and IL-8 were examined using an ELISA kit. The results are shown in FIGS. 21 to 23.

As shown in FIGS. 21 to 23, it was identified that the experimental groups not pre-treated with PMA and co-treated with IL-4, IL-13, and the anti-CD300c monoclonal antibody exhibited increased production levels of TNF-α, IL-1β, and IL-8, and the experimental groups pre-treated with PMA and co-treated with IL-4, IL-13, and the anti-CD300c monoclonal antibody also similarly exhibited increased production levels of TNF-α, IL-1β, and IL-8. The results identified that the anti-CD300c monoclonal antibody could effectively redifferentiate(repolarization) M2 macrophages into M1 macrophages.

### Experimental Example 5.7. Identification of Differentiation Ability and Repolarization Ability into M1 Macrophages

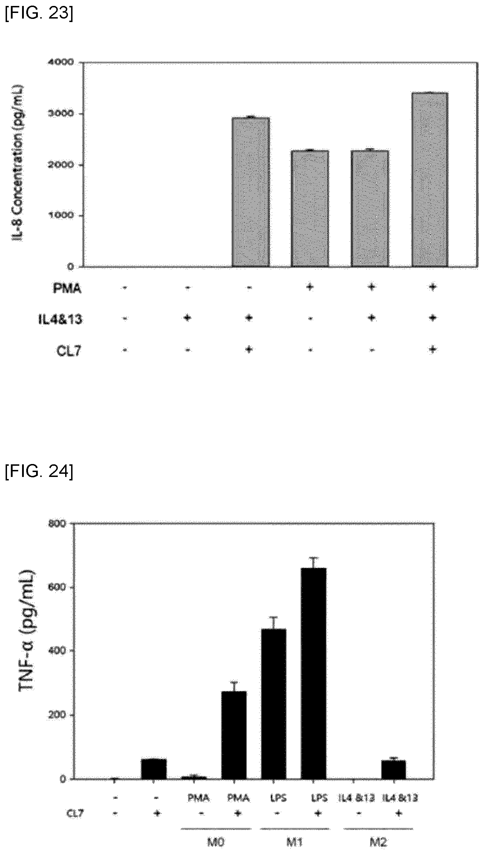

In order to identify the differentiation ability and redifferentiation(repolarization) ability of the anti-CD300c monoclonal antibody into M1 macrophages, THP-1 was dispensed into a 96-well plate at 1.5×10⁴ cells/well, pre-treated with 10 µg/mL of the anti-CD300c monoclonal antibody for 48 hours, and treated with 100 ng/mL of PMA, 100 ng/mL of LPS, and 20 ng/mL of IL-4 and IL-13, followed by incubation for 24 hours. The production level of TNF-α was examined using an ELISA kit. The results are shown in FIG. 24.

As shown in FIG. 24, it was identified that all the experimental groups pre-treated with the anti-CD300c monoclonal antibody exhibited a significant increase in the production level of TNF-α, compared with the M0 macrophage control group treated with PMA alone, the M1 macrophage control group treated with LPS alone, and the M2 macrophage control group treated with IL-4 and IL-13 alone. The results identified that the anti-CD300c monoclonal antibody had excellent abilities to differentiate M0 macrophages into M1 macrophages, differentiate THP-1 into M1 macrophages, and redifferentiate(repolarization) M2 macrophages into M1 macrophages.

### Experimental Example 6. Identification of Inter-Species Cross-Reactivity of Anti-CD300c Monoclonal Antibodies by Anticancer Effect Observation

### Experimental Example 6.1 Identification of Human Cancer Cell Growth inhibitory Effect

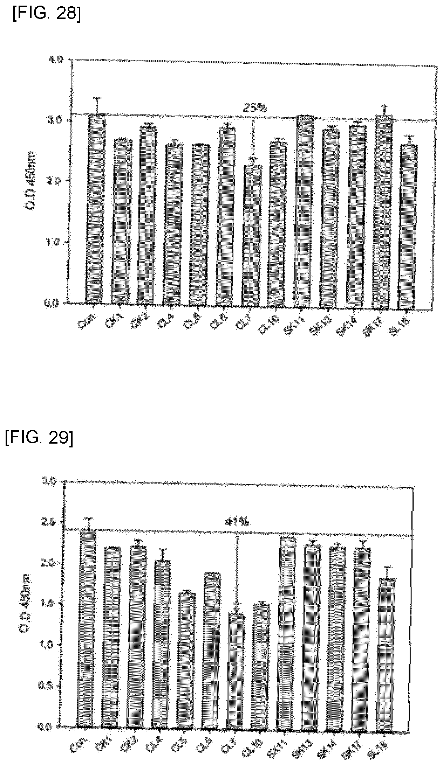

In order to identify the effect of CD300c-targeting monoclonal antibodies on the growth of cancer cells, cell proliferation assay was performed using A549 (human lung cancer cell line). More specifically, 2×10⁴ cells were dispensed into a 96-well plate under the conditions of 0% fetal bovine serum (FBS), and 6×10³ cells were dispensed under the conditions of 0.1% fetal bovine serum. Then, the cells were treated with 10 µg/mL of the anti-CD300c monoclonal antibodies, followed by incubation for 5 days. The cells were treated with CCK-8 (DOJINDO), and the absorbance was measured at OD₄₅₀ₙₘ to examine the cancer cell growth inhibitory effect of the anti-CD300c monoclonal antibodies. The results are shown in FIGS. 28 and 29.

As shown in FIG. 28, it was identified that all the antibodies except for SK11 and SK17 had a cancer cell growth inhibitory effect under the conditions of 0% FBS.

As illustrated in FIG. 29, it was identified that all the anti-CD300c monoclonal antibodies used in the experiment had a cancer cell growth inhibitory effect under 0.1% FBS conditions.

### Experimental Example 6.2. Identification of Cancer Cell Growth inhibitory Effect According to Concentration of Anti-CD300c Monoclonal Antibody

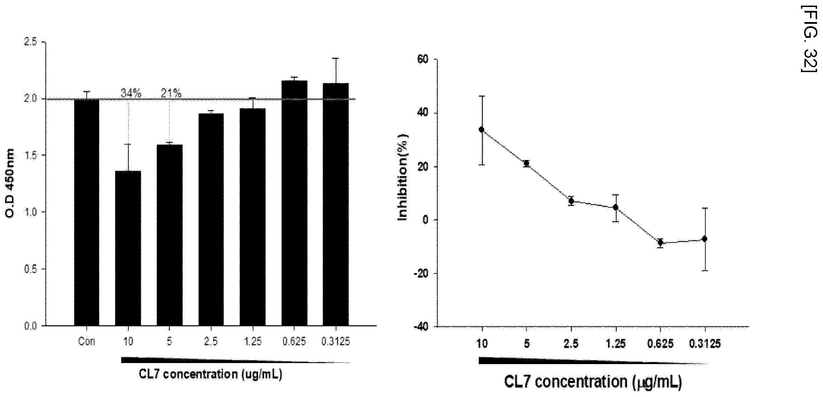

In order to identify the cancer cell growth inhibitory effect according to the concentration of the anti-CD300c monoclonal antibody, 2×10⁴ A549 cells were dispensed into a 96-well plate under the conditions of 0% fetal bovine serum (FBS), and treated with the anti-CD300c monoclonal antibody at 10 µg/mL, followed by incubation for 5 days. Subsequently, the cells were treated with CCK-8 (DOJINDO), followed by incubation for 3 hours, and then the absorbance was measured at OD₄₅₀ₙₘ to examine the cancer cell growth inhibitory effect of the anti-CD300c monoclonal antibody. The results are shown in FIG. 32.

As shown in FIG. 32, it was identified that the growth of cancer cells was inhibited as the concentration of the anti-CD300c monoclonal antibody increased.

### Experimental Example 6.3. Identification of Increased differentiation ability into M1 Macrophages in Mice

In order to identify whether the anti-CD300c monoclonal antibodies could promote the differentiation from mouse macrophages to M1 macrophages, mouse macrophages (Raw264.7) were dispensed into a 96-well plate at a concentration of 1×10⁴ cells/well, and then treated with the anti-CD300c monoclonal antibodies at 10 µg/mL, followed by incubation. The production level of TNF-α was examined using an ELISA kit. The results are shown in FIG. 37.

As shown in FIG. 37, it was identified that the production level of TNF-α increased in the experimental groups treated with the anti-CD300c monoclonal antibodies. The above results indicated that the anti-CD300c monoclonal antibodies acted equally in mice as well as humans, and thus had cross-reactivity of promoting the differentiation into M1 macrophages.

### Experimental Example 6.4. Identification of Cancer Cell Growth inhibitory Effect in Mice

In order to identify whether the anti-CD300c monoclonal antibodies CL7, CL10, and SL18 exhibit an anticancer effect, CT26 (mouse colorectal cancer cell line) was dispensed into a 96-well plate at a concentration of 1×10⁴ cells/well and treated with 10 ug/mL of the monoclonal antibodies, followed by incubation for 5 days. Then, cell proliferation assay was performed through CCK-8 detection.

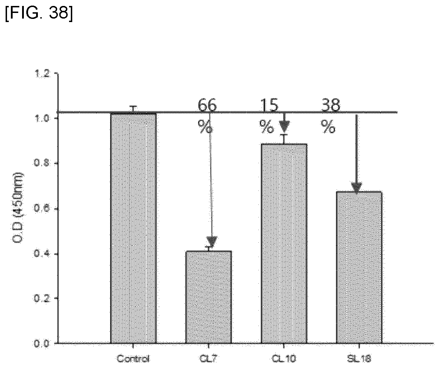

As shown in FIG. 38, it was identified that the anti-CD300c monoclonal antibodies exerted cancer cell proliferation inhibitory effects of 66% (CL7), 15% (CL10), and 38% (SL18), respectively, which were higher than that of the control group, indicating that the anti-CD300c monoclonal antibodies exhibited a cancer therapeutic effect in mice. Therefore, it can be seen that the anti-CD300c monoclonal antibodies acted equally in humans as well as mice, and thus has cross-reactivity showing an anticancer effect.

### Experimental Example 7. Comparison of In Vitro Anticancer Effect Between Anti-CD300c Monoclonal Antibodies and Conventional Immunotherapeutic Agent

Cancer immunotherapeutic agents used in the following experimental examples below are as follows: Imfinzi^{®} (AstraZeneca) and Keytruda^{®} (Merck Sharp & Dohme).

### Experimental Example 7.1. Comparison of Differentiation Ability into M1 Macrophages Between Anti-CD300c Monoclonal Antibodies and Conventional Immunotherapeutic Agent: Measurement of Production Levels of Three Differentiation Markers (TNF-α, IL-1β, and IL-8)

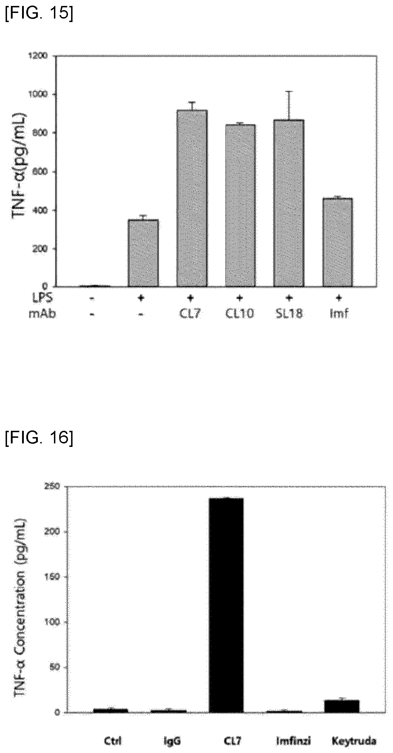

In order to compare the differentiation ability into M1 macrophages between anti-CD300c monoclonal antibodies and a conventional immunotherapeutic agent, the production level of TNF-α was examined using an ELISA kit in the same manner as in Experimental Example 5.2. As the conventional immunotherapeutic agent Imfinzi was used at a concentration of 10 µg/mL. The results are shown in FIG. 15.

As shown in FIG. 15, it was identified that the anti-CD300c monoclonal antibody significantly increased the production level of TNF-α compared with the control group treated with Imfinzi (Imf) alone. The above results identified that the anti-CD300c monoclonal antibodies significantly increased the differentiation ability into M1 macrophages compared with the conventionally known immunotherapeutic agent.

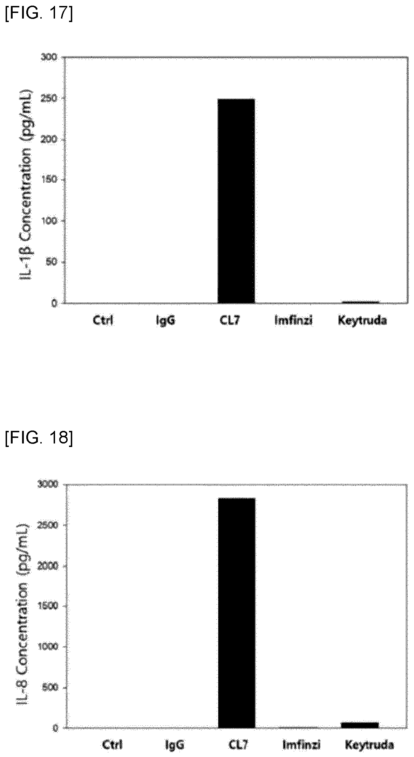

For the comparison with other immunotherapeutic agents, Imfinzi, which is an anti-PD-L1 immunotherapeutic agent, Keytruda, which is an anti-PD-1 immunotherapeutic agent, and an isotype control (immunoglobulin G) antibody were used at a concentration of 10 µg/mL for each, and the production levels of TNF-α, IL-1β, and IL-8 were examined using an ELISA kit. The results are shown in FIGS. 16 to 18.

As shown in FIGS. 16 to 18, it was identified that the anti-CD300c monoclonal antibody significantly increased the production levels of TNF-α, IL-1β, and IL-8 compared with Imfinzi, Keytruda, and the IgG antibody. The above results identified that the anti-CD300c monoclonal antibodies could significantly increase the promotion of differentiation into M1 macrophages compared with the conventional immunotherapeutic agents.

### Experimental Example 7.2. Comparison of Differentiation Ability from M0 Macrophages into M1 Macrophages Between Anti-CD300c Monoclonal Antibody and Conventional Immunotherapeutic agent

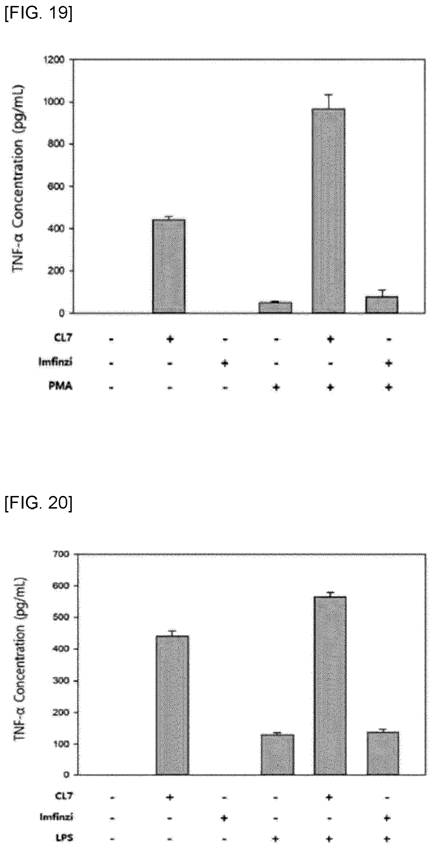

In order to compare the differentiation ability of M0 macrophages into M1 macrophages between the anti-CD300c monoclonal antibody and an immunotherapeutic agent, THP-1 was dispensed into a 96-well plate at 1.5×10⁴ cells/well and treated with the anti-CD300c monoclonal antibody 10 µg/mL, Imfinzi at 10 µg/mL, and/or 200 nM phorbol-12-myristate-13-acetate (PMA). After the incubation for 48 hours, the production level of TNF-α was measured using an ELISA kit. The results are shown in FIG. 19.

As shown in FIG. 19, it was identified that TNF-α was not produced in the comparison group treated with the immunotherapeutic agent Imfinzi alone, but the production level of TNF-α increased in the experimental group treated with the anti-CD300c monoclonal antibody alone. In addition, it was identified that even when THP-1 cells were differentiated into M0 macrophages by treatment with PMA, the experimental group treated with the anti-CD300c monoclonal antibody showed a significantly high production level of TNF-α compared with the experimental group treated with Imfinzi. The above results identified that the anti-CD300c monoclonal antibody promoted the differentiation of M0 macrophages into M1 macrophages compared with the conventional immunotherapeutic agent.

### Experimental Example 7.3. Comparison of Differentiation Ability into M1 Macrophages Between Anti-CD300c Monoclonal Antibody and Conventional Immunotherapeutic Agent

In order to compare the differentiation ability into M1 macrophages between an anti-CD300c monoclonal antibody and a conventional immunotherapeutic agent, the production level of TNF-α was examined in the same manner as in Experimental Example 5.2. The results are shown in FIG. 20.

As can be shown in FIG. 20, it was identified that when monocytes were differentiated into M1 macrophages by treatment with LPS, the experimental group co-treated with Imfinzi and LPS did not exhibit a significant difference in the production level of TNF-α, but the experimental group co-treated with the anti-CD300c monoclonal antibody and LPS exhibited a significant increase in the production level of TNF-α compared with the experimental group treated with the anti-CD300c monoclonal antibody alone.

### Experimental Example 7.4. Comparison of Cancer Cell Growth inhibitory Effect Between Anti-CD300c Monoclonal Antibodies and Conventional Immunotherapeutic Agent

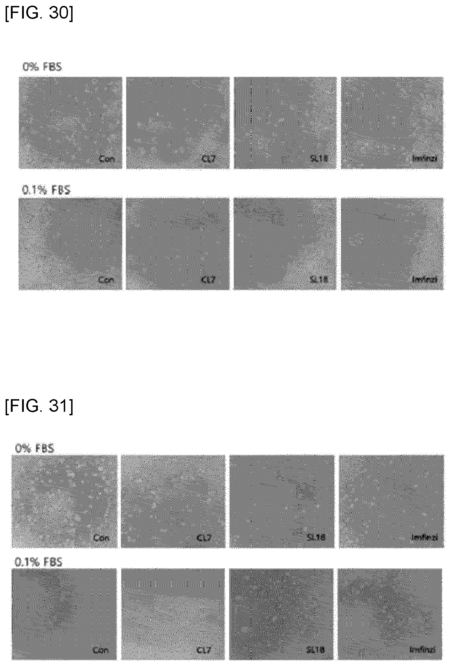

In order to compare the cancer cell growth inhibitory effect between an anti-CD300c monoclonal antibody and an immunotherapeutic agent, the cell growth inhibitory effect was examined using A549 (human lung cancer cell line) and MDA-MB-231 (human breast cancer cell line). More specifically, 2×10⁴ cells were dispensed into a 96-well plate under the conditions of 0% fetal bovine serum (FBS) and 6×10³ cells were dispensed under the conditions of 0.1% fetal bovine serum. Then, the cells were treated with an anti-CD300c monoclonal antibody at 10 µg/mL, followed by incubation for 5 days, and then observed under an optical microscope. The results are shown in FIGS. 30 and 31.

As shown in FIG. 30, it was identified that the anti-CD300c monoclonal antibodies more effectively inhibited the proliferation of cancer cells than the immunotherapeutic agent Imfinzi in the A549 cell line.

As shown in FIG. 31, it was identified that the anti-CD300c monoclonal antibodies more effectively inhibited the proliferation of cancer cells than the immunotherapeutic agent Imfinzi in the MDA-MB-231 cell line.

### V. Combination of Anti-CD300c Monoclonal Antibody and Immunotherapeutic Agent

### Example 5. Co-Administration of Anti-CD300c Monoclonal Antibody (CL7) and Immunotherapeutic Agent

The anti-CD300c monoclonal antibody (CL7) produced in Example 1 was used in combination with other immunotherapeutic agents, for example, the anti-PD-L1 antibodies Imfinzi^{®} and Opdivo^{®}, and the anti-PD-1 antibody Keytruda^{®}, an anti-CD47 antibody (aCD47), and an anti-CTLA-4 antibody.

The respective immunotherapeutic agents were accessible from the following: IMFINZI (AstraZeneca); Opdivo and the anti-CTLA-4 antibody (Bristol Myers Squibb Company); Keytruda (Merck Sharp & Dohme); and the anti-CD47 antibody (Abcam).

### Experimental Example 8. Identification of (Synergistic) Increase in Macrophage Activity by Combination

### Experimental Example 8.1. Identification of Increase in Differentiation Ability into M1 Macrophages

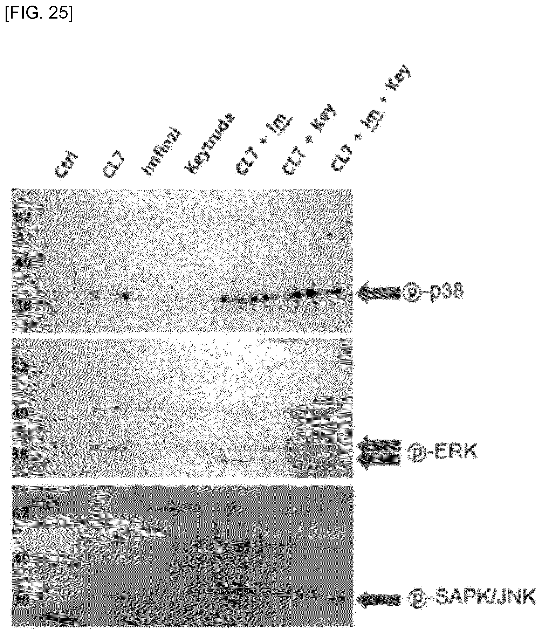

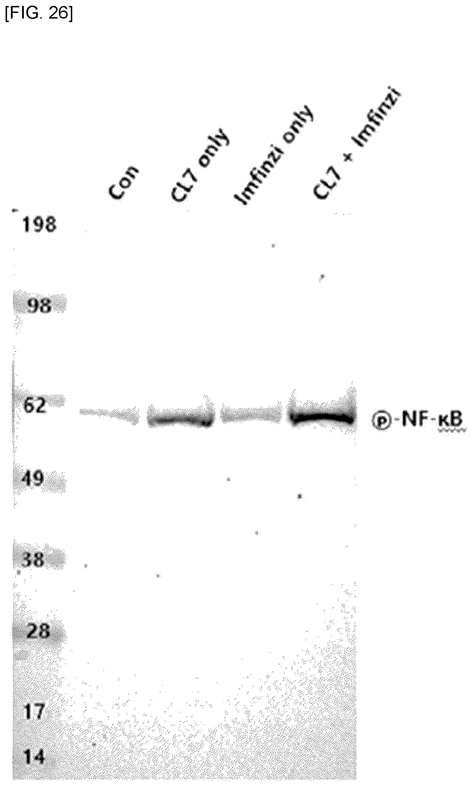

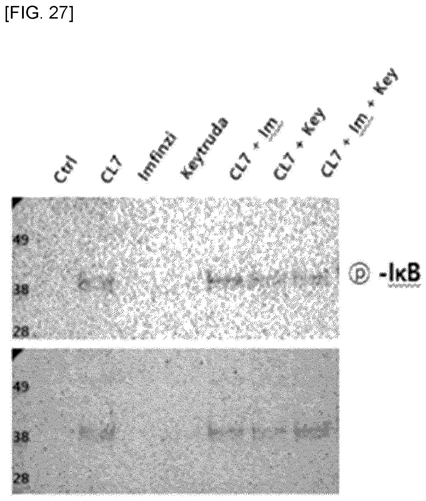

In order to identify that the differentiation ability of monocytes into M1 macrophages increased when the monocytes are treated with the anti-CD300c monoclonal antibody CL7 in combination with an immunotherapeutic agent, such as an anti-PD-1 antibody, an anti-PD-L1 antibody, an anti-CTLA-4 antibody, and an anti-CD47 antibody, the signaling of mitogen-activated protein kinase (MAPK), IκB, and NF-kB, which are representative signals of M1 macrophage differentiation was examined. Specifically, THP-1 was dispensed into a 6-well plate at 8.8×10⁵ cells/well, and treated with 10 µg/mL of the anti-CD300c monoclonal antibody, 10 µg/mL of Imfinzi, and/or 10 µg/mL of Keytruda. For a control group, an equal amount of phosphate buffer solution (PBS) was used for treatment. After incubation for 48 hours, phosphorylated SAPK/JNK, phosphorylated ERK, and phosphorylated p38 for MAPK signal, phosphorylated NF-kB for NF-kB signal, and phosphorylated IkB for IkB signal were examined through Western blotting. The results are shown in FIGS. 25 to 27.

FIGS. 25, 26, and 27 illustrate the results of signaling of MAPK, NF-Kb, and IkB, respectively. It was identified that the levels of phosphorylated MAPK, IkB, and NF-kB increased when the cells were treated with the anti-CD300c monoclonal antibody in combination with an immunotherapeutic agent, such as an anti-PD-1 antibody, an anti-PD-L1 antibody, an anti-CTLA-4 antibody, and an anti-CD47 antibody, compared with when the cells were treated with the anti-CD300c monoclonal antibody alone. The above results identified that the cell signaling representing the differentiation into M1 macrophages increased when the cells were treated with the anti-CD300c monoclonal antibody in combination with an immunotherapeutic agent, such as an anti-PD-1 antibody, an anti-PD-L1 antibody, an anti-CTLA-4 antibody, and an anti-CD47 antibody, compared with when the cells were treated with the anti-CD300c monoclonal antibody alone.

### Experimental Example 9. Identification of (Synergistic) Increase in Cancer Cell Growth Inhibitory Effect by Combination (In Vitro)

### Experimental Example 9.1. Identification of Apoptosis Signals

It was identified whether the apoptosis signal increased when the anti-CD300c monoclonal antibody CL7 was used in combination with an immunotherapeutic agent, such as an anti-PD-1 antibody, an anti-PD-L1 antibody, an anti-CTLA-4 antibody, and an anti-CD47 antibody. Specifically, A549 cells were dispensed into a 6-well plate at 8×10⁵ cells/well and treated with 10 µg/mL of the anti-CD300c monoclonal antibody, and 10 µg/mL of Imfinzi, Keytruda, Opdivo, and an anti-CD47 antibody, alone or in combination. After incubation for 48 hours, the apoptosis signal or cell cycle signal was examined through Western blotting. Cleaved caspase-9, caspase-3, caspase-2, and caspase-8 were checked as markers for the apoptosis signal, and cyclin D1, CDK2, p27kip1, CDK6, cyclin D3, P21 Waf1, Cip1, and the like were checked as markers for the cell cycle signal.

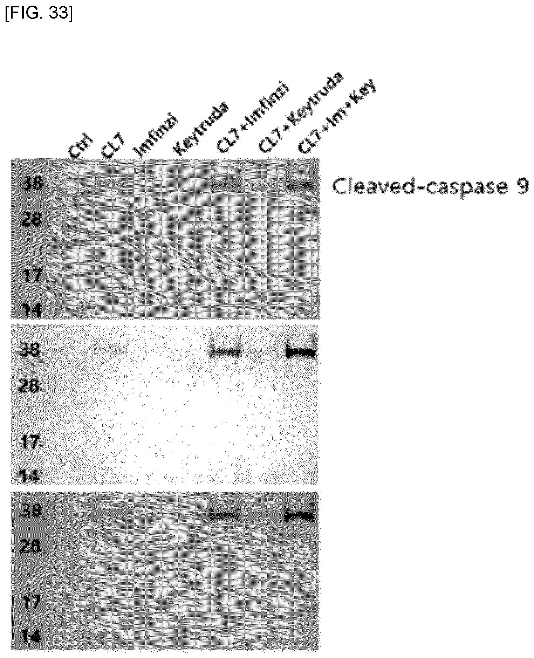

As shown in FIG. 33, the apoptosis signal increased when the cells were co-treated with the anti-CD300c monoclonal antibody and the anti-PD-1 antibody Imfinzi compared with when the cells were treated with the anti-CD300c monoclonal antibody alone, and the levels of cleaved-caspase9 and p21 increased and the level of cyclin D1 decreased when the cells were co-treated with the anti-CD300c monoclonal antibody in combination with an immunotherapeutic agent, such as an anti-PD-1 antibody, an anti-PD-L1 antibody, an anti-CTLA-4 antibody, or an anti-CD47 antibody. The above results identified that the apoptosis of cancer cells was better induced when the cells were treated with the anti-CD300c monoclonal antibody in combination with an immunotherapeutic agent, such as an anti-PD-1 antibody, an anti-PD-L1 antibody, an anti-CTLA-4 antibody, or an anti-CD47 antibody, compared with when the cells were treated with the anti-CD300c monoclonal antibody alone.

### Experimental Example 9.2. Identification of Growth inhibitory Effect on Cancer Cell Lines

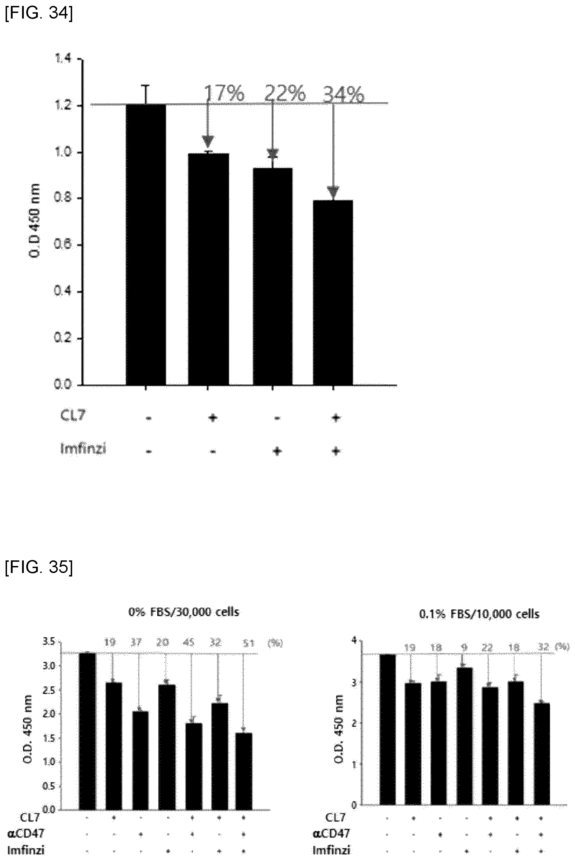

In order to identify the cancer cell growth inhibitory effect by co-administration of the anti-CD300c monoclonal antibody CL7 and an immunotherapeutic agent, the comparison of the cancer cell growth inhibitory effect was performed using A549 (human lung cancer cell line) and MDA-MB-231 (human breast cancer cell line). Specifically, 2×10⁴ cells (A549) or 3×10⁴ cells (MDA-MB-231) were dispensed into a 96-well plate in the absence of fetal bovine serum (FBS) conditions, and at 6×10³ cells (A549) or 1×10⁴ cells (MDA-MB-231) were dispensed under the conditions of 0.1% fetal bovine serum. Subsequently, the cells were treated with 10 µg/mL of the anti-CD300c monoclonal antibody and Imfinzi alone or in combination and incubated for 5 days. For a control group, an equal amount of phosphate buffer solution (PBS) was used for treatment. Then, the cells were treated with CCK-8 (DOJINDO) and the absorbance was measured at OD₄₅₀ₙₘ. The results are shown in FIG. 34 (A549) and FIG. 35 (MDA-MB-231).

As shown in FIG. 34, it was identified that as for the A549 cell line in the absence of FBS, compared with the control group, the cell growth inhibitory effect was 17% higher in the treatment with the anti-CD300c monoclonal antibody alone and 34% higher in the treatment with the anti-CD300c monoclonal antibody and Imfinzi in combination.

As shown in FIG. 35, as for the MDA-MB-231 cell line under the conditions of 0% FBS, compared with the control group, the cancer cell growth inhibitory effect was observed to be 19% higher in the treatment with the anti-CD300c monoclonal antibody alone, 45% higher in the treatment with the anti-CD300c monoclonal antibody and the anti-CD47 antibody in combination, and 51% higher in the treatment with the anti-CD300c monoclonal antibody, the anti-CD47 antibody, and Imfinzi in combination. Under 0.1% FBS conditions, the cancer cell growth inhibitory effect was observed to be 19% higher in the treatment with the anti-CD300c monoclonal antibody, 22% higher in the treatment with the anti-CD300c monoclonal antibody and the anti-CD47 antibody in combination, and 32% higher in the treatment with the anti-CD300c monoclonal antibody, the anti-CD47 antibody, and Imfinzi in combination.

The above results identified that the cancer cell growth was further inhibited in the treatment with the anti-CD300c monoclonal antibody in combination with an immunotherapeutic agent, such as an anti-PD-1 antibody, an anti-PD-L1 antibody, an anti-CTLA-4 antibody, or an anti-CD47 antibody, compared with the treatment with the anti-CD300c monoclonal antibody alone.

### Experimental Example 10. Identification of In Vivo (Synergistic) Increase by Combination

### Experimental Example 10.1. Identification of In Vivo Cancer Cell Growth inhibitory Effect

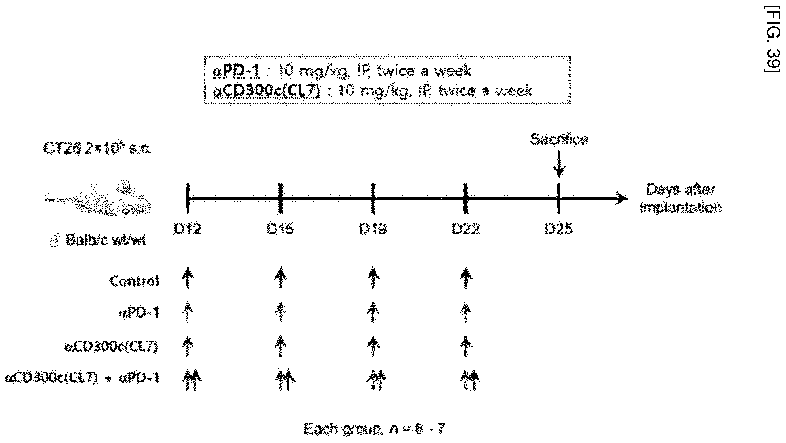

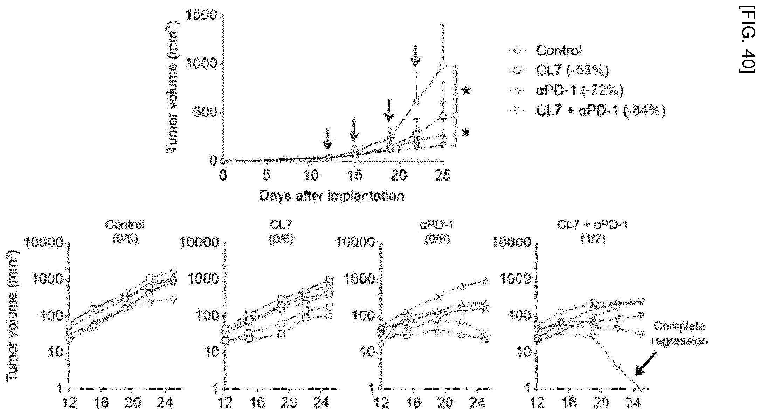

In order to identify the anticancer effect *in vivo* of the anti-CD300c monoclonal antibody CL7, allograft mouse tumor models were constructed by implanting a colorectal cancer cell line (CT26) at 2×10⁵ cells into 8-week-old BALB/c mice through subcutaneous injection. Animal breeding and experiments were all carried out in a specific pathogen free (SPF) facility. On day 12 (D12) after implantation of the colorectal cancer cell line, the mice with a tumor size of 50 to 100 mm³ were administered with the anti-CD300c monoclonal antibody and an anti-PD-1 antibody purchased from BioXcell alone or in combination, and administered with an equal amount of phosphate buffered saline (PBS) for a control group. A schematic experimental method is shown in FIG. 39. Specifically, the mice were intraperitoneally injected with the respective antibodies (CL7: 10 mg/kg; and anti-PD-1 antibody: 10 mg/kg) alone or in combination, twice a week for two weeks (a total of 4 times on D12, D15, D19, and D22). The tumor volume was measured for 25 days. The results are shown in FIG. 40.

As can be seen from FIG. 40, it was identified that the cancer growth was inhibited in the experimental group administered with the anti-CD300c monoclonal antibody alone compared with the control group, but the cancer growth was more effectively inhibited in the treatment with the anti-CD300c monoclonal antibody and an immunotherapeutic agent such as the anti-PD-1 antibody in combination compared with the treatment with the anti-CD300c monoclonal antibody alone.

### Experimental Example 10.2. Identification of M1 Macrophage Increase Effect In Vivo

In order to identify whether an anti-CD300c monoclonal antibody increases M1 macrophages in the cancer tissue of mouse models, the mice experimented in the same manner as in Experimental Example 10.1 were euthanized on day 25, and 1% para-formaldehyde (PFA) was intravascularly injected and perfused into the mice, and then cancer tissue was obtained. The obtained cancer tissue was fixed using 1% PFA, and sequentially dehydrated using 10%, 20%, and 30% sucrose solution. The dehydrated cancer tissue was frozen in the optimal cutting temperature (OCT) compound, and then the cancer tissue was sectioned to a thickness of 50 µm by using a cryotome. The tissue was incubated for 1 hour at 37°C in a mixed solution of 20 mg/ml collagenase D and 2 mg/ml DNase I, and then filtered through a 70 µm cell strainer. The red blood cells were lyzed, and the cells were again filtered through a nylon mesh to become single cells. To inhibit non-specific reactions in the single cell suspension, the cells were incubated with a CD16/32 antibody (Invitrogen) for 1 hour, and the cell viability was examined. The resultant product was stained with antibodies to the M1 macrophage marker iNOS and the M2 macrophage marker CD206 and analyzed by FACS.

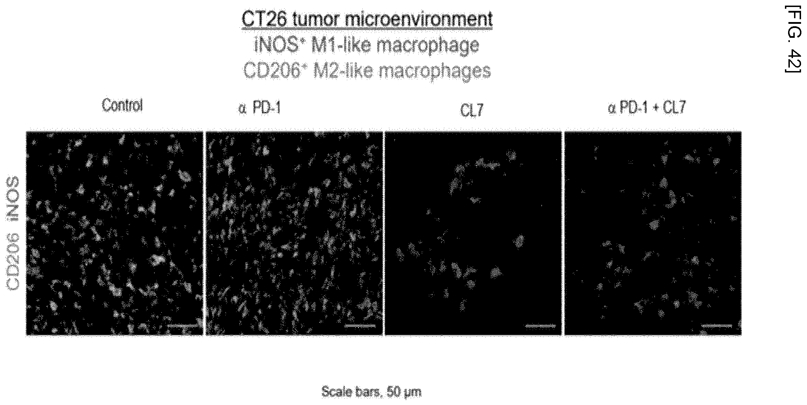

As a result, as shown in FIG. 42, it was identified that M1 macrophages partially increased in the experimental group treated with the anti-PD-1 antibody compared with the control group, but in the experimental group treated with the anti-CD300c monoclonal antibody, M1 macrophages significantly increased and M2 macrophages were hardly observed. In addition, it was identified that M1 macrophages further increased in the experimental group co-administered with the anti-CD300c monoclonal antibody and the anti-PD-1 antibody. The above results identified that the differentiation into M1 macrophages can be effectively promoted in the treatment with the anti-CD300c monoclonal antibody in combination with an immunotherapeutic agent, such as an anti-PD-1 antibody, an anti-PD-L1 antibody, an anti-CTLA-4 antibody, and an anti-CD47 antibody, compared with the treatment with the anti-CD300c monoclonal antibody alone.

### Experimental Example 10.3. Identification of CD8+ T Cell Immunity Stimulating Effect In Vivo

In order to identify whether the anti-CD300c monoclonal antibody CL7 stimulated CD8+ T cell immunity in mouse tumor models, the mice experimented in the same manner as in Experimental Example 10.1 were euthanized on day 25, and then 1% para-formaldehyde (PFA) was intravascularly injected and perfused into the mice, and then cancer tissue was obtained. The obtained cancer tissue was fixed using 1% PFA, and sequentially dehydrated using 10%, 20%, and 30% sucrose solution. The dehydrated cancer tissue was frozen in the optimal cutting temperature (OCT) compound, and then the cancer tissue was sectioned to a thickness of 50 µm by using a cryotome. Then, staining was performed using CD8+ and iNOS.

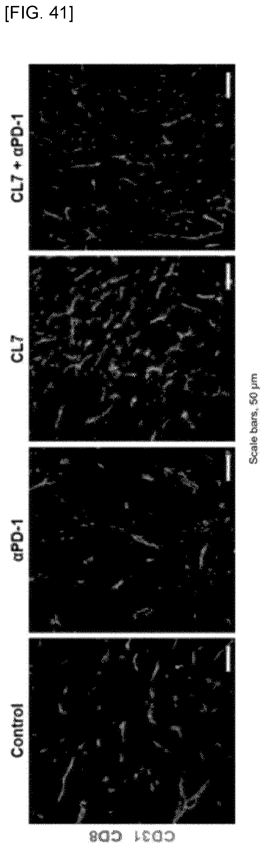

As shown in FIG. 41, it was identified that the CD8+ T cells partially increased in the experimental group treated with the anti-PD-1 antibody compared with the control group, but the CD8+ T cells significantly increased in the experimental group treated with the anti-CD300c monoclonal antibody. It was also identified that the CD8+ T cells further increased in the experimental group administered with the anti-CD300c monoclonal antibody and the anti-PD-1 antibody in combination compared with the anti-PD-1 alone treatment group. The above results identified that the anti-CD300c monoclonal antibody increased the number of CD8+ T cells more effectively when the anti-CD300c monoclonal antibody was used in combination with the conventional immunotherapeutic agent.

Through the results as above, it was identified that the anti-CD300c monoclonal antibodies of the present disclosure can bind with high specificity to the CD300c antigen and can be used for various individuals due to their inter-species cross-reactivity, for example, with mice. It was also identified both *in vitro* and *in vivo* that the anti-CD300c monoclonal antibodies can effectively inhibit the proliferation, metastasis, and the like of cancer cells by acting as immunotherapeutic agents through the activation of T cells and the promotion of differentiation into M1 macrophages, and it was identified that the anti-CD300c monoclonal antibodies further increase their therapeutic effects by co-administration with a conventional immunotherapeutic agent. Accordingly, it was found that the anti-CD300c monoclonal antibodies can be effectively used for immunotherapeutic agent against various cancers expressing CD300c antigen.

The description of the present disclosure as described above is provided for illustration, and those of ordinary skill in the art to which the present disclosure pertains would be able to understand that the embodiments disclosed herein can be easily modified into other specific forms without changing the technical spirit or essential features of the present disclosure. Therefore, the embodiments and examples described above should be understood as being illustrative but not limitative in all aspects.

## Claims

1. A chimeric antigen receptor comprising a binding domain specifically binding to a CD300c antigen or a receptor thereof.

2. The chimeric antigen receptor of claim 1, further comprising a transmembrane domain and an intracellular signaling domain.

3. The chimeric antigen receptor of claim 1, wherein the binding domain is any one selected from the group consisting of an antibody, a single domain antibody, and a single chain variable fragment.

4. The chimeric antigen receptor of claim 1,
wherein the binding domain comprises:
(i) a heavy chain variable region, comprising:
CDR1 comprising an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
CDR2 comprising an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
CDR3 comprising an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region, comprising:
CDR1 comprising an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
CDR2 comprising an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
CDR3 comprising an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300, or
wherein the binding domain comprises the amino acid sequence represented by SEQ ID NO: 402.

5. The chimeric antigen receptor of claim 1, wherein the binding domain comprises:
a heavy chain variable region comprising CDR1 to CDR3 comprising amino acid sequences represented by of Formulas (1) to (3), respectively, and
a light chain variable region comprising CDR1 to CDR3 comprising amino acid sequences represented by Formulas (4) to (6), respectively:
FTFSX1YX2MX3WVR (1)
wherein,
X1= R, S, or D
X2= A, G, or H
X3= T, H, or S
X1X2SX3X4GGX5TYYAX6 (2)
wherein,
X1= S, A, or T
X2= M or I
X3= G or S
X4= T or S
X5= T, S, or Y
X6= D or E
YCAX1X2X3X4X5X6X7X8X9X10X11W (3)
wherein,
X1= R, V, or S
X2= G or S
X3= A, G, S, Y, or I
X4= Y, A, Q, G, or R
X5= G or L
X6= F, R, I, M, or P
X7= D, G, F, or L
X8= H, F, D, or V
X9= F, I, Y or not present
X10= D or not present
X11= Y or not present
CX1X2X3X4X5X6X7X8X9X10X11X12X13W (4)
wherein,
X1= R, S, or T
X2= A, G, or R
X3= S or N
X4= Q, S, or N
X5= S, I, or G
X6= I, N, or G
X7= G, I, T, or S
X8= N, G, R, A, or K
X9= Y, S, R, or G
X10= N or not present
X11= Y or not present
X12= L or V
X13= N, Y, H, or Q
X1X2X3X4X5X6X7GX8X9 (5)
wherein,
X1= D, E, S, or R
X2= A, D, K, or N
X3= S or N
X4= N, K, or Q
X5= L or R
X6= E or P
X7= T or S
X8= I or V
X9= P or R
YCX1X2X3X4X5X6X7X8X9X10X11F (6)
wherein,
X1= Q, S, or A
X2= Q, S, or A
X3= S, Y, or W
X4= S, T, D, or A
X5= A, S, D, or G
X6= I, S, N, or T
X7= P, S, L, N, or K
X8= Y, T, S, N, or G
X9= V, G, L or not present
X10 = P or not present
X11= T, I, or V.

6. The chimeric antigen receptor of claim 4, wherein (i) the heavy chain variable region comprises:
CDR1 comprising or consisting of the amino acid sequence of SEQ ID NO: 7, SEQ ID NO: 67, SEQ ID NO: 79, SEQ ID NO: 115, or SEQ ID NO: 211;
CDR2 comprising or consisting of the amino acid sequence of SEQ ID NO: 8, SEQ ID NO: 68, SEQ ID NO: 80, SEQ ID NO: 116, or SEQ ID NO: 212; and
CDR3 comprising or consisting of the amino acid sequence of SEQ ID NO: 9, SEQ ID NO: 69, SEQ ID NO: 81, SEQ ID NO: 117, or SEQ ID NO: 213, and
(ii) the light chain variable region comprises:
CDR1 comprising or consisting of the amino acid sequence of SEQ ID NO: 10, SEQ ID NO: 70, SEQ ID NO: 82, SEQ ID NO: 118, or SEQ ID NO: 214;
CDR2 comprising or consisting of the amino acid sequence of SEQ ID NO: 11, SEQ ID NO: 71, SEQ ID NO: 83, SEQ ID NO: 119, or SEQ ID NO: 215; and
CDR3 comprising or consisting of the amino acid sequence of SEQ ID NO: 12, SEQ ID NO: 72, SEQ ID NO: 84, SEQ ID NO: 120, or SEQ ID NO: ID NO: 216.

7. The chimeric antigen receptor of claim 1, further comprising a signal peptide, a GS linker, a transmembrane domain, and an intracytoplasmic domain.

8. The chimeric antigen receptor of claim 7, wherein the signal peptide comprises a CD8α signal peptide.

9. The chimeric antigen receptor of claim 7, wherein the transmembrane domain comprises a CD8 hinge (hinge of cluster of differentiation 8) and a CD28 transmembrane domain.

10. The chimeric antigen receptor of claim 7, wherein the intracytoplasmic domain comprises a CD28 intracellular domain and a CD3ζ intracellular domain.

11. A polynucleotide comprising a nucleic acid sequence encoding the chimeric antigen receptor of any one of claims 1 to 10.

12. An expression vector comprising the polynucleotide of claim 11.

13. An immune cell comprising the chimeric antigen receptor of any one of claims 1 to 10.

14. The immune cell of claim 13, wherein the immune cell includes any one or more selected from the group consisting of a monocyte, a macrophage, a T cell, a natural killer cell (NK cell), and a dendritic cell.

15. A pharmaceutical composition for prevention or treatment of cancer expressing a CD300c antigen or a CD300c receptor, the pharmaceutical composition comprising the immune cell of claim 13 as an active ingredient.

16. The pharmaceutical composition of claim 15, wherein the cancer includes any one or more selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer.

17. The pharmaceutical composition of claim 15, further comprising another anticancer agent.

18. The pharmaceutical composition of claim 15, wherein the pharmaceutical composition inhibits the proliferation, survival, metastasis, recurrence, or anticancer agent resistance of cancer

19. A method for preventing or treating cancer, the method comprising administering to a subject a composition comprising the immune cell of claim 13 as an active ingredient.

20. Use of the immune cell of claim 13 for preventing or treating cancer.

21. Use of the immune cell of claim 13 for the manufacture of a medicament for preventing or treating cancer.
